# EUROPEAN PATENT APPLICATION

(11) **EP 3 753 560 A1**
(43) Date of publication of application: **23.12.2020**
(21) Application number: 19181253.6
(22) Date of filing: 19.06.2019
(51) Int. Cl.: A61K 31/343, A61K 31/352, A61K 31/353, A61K 31/401, A61K 31/405, A61K 31/422, A61K 31/451, A61P 21/00, A61P 21/04, C07D 491/00

(54) **COMPOUNDS FOR THE TREATMENT OF NEUROMUSCULAR DISORDERS**

(71) Applicant: NMD Pharma A/S, 8200 Århus N (DK)
(72) Inventor: J.S. Knutsen, Lars, Essex, CO13 9AY (GB); Holm Pedersen, Thomas, 8240 Risskov (DK); KELLY, Nicholas, 2880 Bagsværd (DK); E Cooper, Martin, Nottingham, NG4 1AQ (GB); W. Brown, Andrew, Derbyshire, DE56 2AG (GB)
(74) Representative: Høiberg P/S

(57) **Abstract**

The present disclosure relates to compounds suitable for treating, ameliorating and/or preventing neuromuscular disorders, including the reversal of drug-induced neuromuscular blockade. The compounds as defined herein can inhibit the CIC-1 ion channel.

## Description

### Technical field

The present disclosure relates to compounds and their use in treating, ameliorating and/or preventing neuromuscular disorders, including the reversal of drug-induced neuromuscular blockade. The compounds as defined herein can inhibit the CIC-1 ion channel. The disclosure further relates to methods of treating, preventing and/or ameliorating neuromuscular disorders, by administering said composition to a person in need thereof.

### Background

Walking, breathing, and eye movement are examples of essential everyday physiological activities that are powered by the contractile activity of skeletal muscle. Skeletal muscles are inherently in a resting state and contractile activity occurs exclusively in response to commands from the central nervous system (CNS). Such neuronal commands take the form of action potentials that travel from the brain to the muscle fibres in several steps. The neuromuscular junction (NMJ) is a highly specialized membrane area on muscle fibres where motor neurons come into close contact with the muscle fibres, and it is at the NMJ where neuronal action potentials are transmitted to muscular action potentials in a one-to-one fashion *via* synaptic transmission.

Neuromuscular transmission refers to the sequence of cellular events at the NMJ whereby an action potential in the lower motor neuron is transmitted to a corresponding action potential in a muscle fibre (Wood SJ, Slater CR. Safety factor at the neuromuscular junction. Prog. Neurobiol. 2001, 64, 393-429). When a neuronal action potential arrives at the pre-synaptic terminal it triggers influx of Ca²⁺ through voltage gated P/Q-type Ca²⁺ channels in the nerve terminal membrane. This influx causes a rise in cytosolic Ca²⁺ in the nerve terminal that triggers exocytosis of acetylcholine (ACh). Released ACh next diffuses across the synaptic cleft to activate nicotinic ACh receptors in the post-synaptic, muscle fibre membrane. Upon activation, ACh receptors convey an excitatory current flow of Na⁺ into the muscle fibre, which results in a local depolarization of the muscle fibre at the NMJ that is known as the endplate potential (EPP). If the EPP is sufficiently large, voltage gated Na⁺ channels in the muscle fibre will activate and an action potential in the muscle fibre will ensue. This action potential then propagates from the NMJ throughout the muscle fibre and triggers release of Ca²⁺ release from the sarcoplasmic reticulum. The released Ca²⁺ activates the contractile proteins within the muscle fibres, thus resulting in contraction of the fibre.

Failure of neuromuscular transmission can arise from both pre-synaptic dysfunction [Lambert Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107), amyotrophic lateral sclerosis (Killian JM, Wilfong AA, Burnett L, Appel SH, Boland D. Decremental motor responses to repetitive nerve stimulation in ALS. Muscle Nerve, 1994, 17, 747-754), spinal muscular atrophy (Wadman RI, Vrancken AF, van den Berg LH, van der Pol WL. Dysfunction of the neuromuscular junction in spinal muscular atrophy types 2 and 3. Neurology, 2012, 79, 2050-2055) and as a result of post-synaptic dysfunction as occurs in myasthenia gravis (Le Panse R, Berrih-Aknin S. Autoimmune myasthenia gravis: autoantibody mechanisms and new developments on immune regulation. Curr Opin Neurol., 2013, 26, 569-576)]. Failure to excite and/or propagate action potentials in muscle can also arise from reduced muscle excitability such as in critical illness myopathy (CIM) (Latronico, N., Bolton, C.F. Critical illness polyneuropathy and myopathy: a major cause of muscle weakness and paralysis. Lancet Neurol. 2011, 10, 931-941). In Lambert Eaton syndrome, an autoimmune attack against the pre-synaptic P/Q-type Ca²⁺ channels results in markedly reduced Ca²⁺ influx into the nerve terminal during the pre-synaptic action potential and consequently a reduced release of ACh into the synaptic cleft. In myasthenia gravis, the most common finding is an autoimmune attack on the post-synaptic membrane either against the nicotinic ACh receptors or the musk-receptor in the muscle fibre membrane³. Congenital forms of myasthenia are also known⁵. Common to disorders with neuromuscular transmission failure (Lambert Eaton syndrome, amyotrophic lateral sclerosis, spinal muscular atrophy and myasthenia gravis) is that the current flow generated by ACh receptor activation is markedly reduced, and EPPs therefore become insufficient to trigger muscle fibre action potentials.

Neuromuscular blocking agents also reduce EPP by antagonizing ACh receptors. In CIM with reduced muscle excitability, the EPP may be of normal amplitude but they are still insufficient to trigger muscle fibre action potentials because the membrane potential threshold for action potential excitation has become more depolarized because of loss of function of voltage gated Na⁺ channels in the muscle fibres.

While ACh release (Lambert Eaton, amyotrophic lateral sclerosis, spinal muscular atrophy), ACh receptor function (myasthenia gravis, neuromuscular blockade) and function of voltage gated Na⁺ channels (CIM) are essential components in the synaptic transmission at NMJ, the magnitude of the EPP is also affected by inhibitory currents flowing in the NMJ region of muscle fibres. These currents tend to outbalance excitatory current through ACh receptors and, expectedly, they thereby tend to reduce EPP amplitude. The most important ion channel for carrying such inhibitory membrane currents in muscle fibres is the muscle-specific CIC-1 Cl⁻ ion channel (Kwiecinski H, Lehmann-Horn F, Rüdel R. Membrane currents in human intercostal muscle at varied extracellular potassium. Muscle Nerve. 1984, 7, 465-469; Kwieciński H, Lehmann-Horn F, Rüdel R. Drug-induced myotonia in human intercostal muscle. Muscle Nerve. 1988, 11, 576-581; Pedersen, T.H., F. de Paoli, and O.B. Nielsen. Increased excitability of acidified skeletal muscle: role of chloride conductance. J. Gen. Physiol., 2005, 125, 237-246).

ACh esterase (AChE) inhibitors are traditionally used in the treatment of myasthenia gravis. This treatment leads to improvement in most patients but it is associated with side effects, some of which are serious (Mehndiratta MM, Pandey S, Kuntzer T. Acetylcholinesterase inhibitor treatment for myasthenia gravis. Cochrane Database Syst Rev. 2014, Oct 13;10). Because ACh is an import neurotransmitter in the autonomic nervous system, delaying its breakdown can lead to gastric discomfort, diarrhea, salivation and muscle cramping. Overdosing is a serious concern as it can lead to muscle paralysis and respiratory failure, a situation commonly referred to as cholinergic crisis. Despite the serious side effects of AChE inhibitors, these drugs are today the treatment of choice for a number of disorders involving neuromuscular impairment. In patients where pyridostigmine (a parasympathomimetic and a reversible ACHE inhibitor) is insufficient, corticosteroid treatment (prednisone) and immunosuppressive treatment (azathioprine) is used. Plasma exchange can be used to obtain a fast but transient improvement.

Unfortunately, all of the currently-employed drug regimens for treatment of myasthenia gravis are associated with deleterious long-term consequences (Howard, J.F. Jr. Adverse drug effects on neuromuscular transmission. Semin Neurol. 1990, 10, 89 - 102) despite research to identify new treatments (Gilhus, N.E. New England Journal of Medicine, 2016, 375, 2570-2581).

The CIC-1 ion channel (Pedersen, T.H., Riisager, A., Vincenzo de Paoli, F., Chen, T-Y, Nielsen, O.B. Role of physiological CIC-1 Cl- ion channel regulation for the excitability and function of working skeletal muscle. J. Gen. Physiol. 2016, 147, 291 - 308) is emerging as a target for potential drugs, although its potential has been largely unrealized.

There have been publications of various ligands at the CIC-1 ion channels, see for example: Liantonio, A., Accardi, A., Carbonara, G., Fracchiolla, G., Loiodice, F., Tortorella P, Traverso S, Guida P, Pierno S, De Luca A, Camerino DC, Pusch M. Molecular requisites for drug binding to muscle CIC-1 and renal CIC-K channel revealed by the use of phenoxy-alkyl derivatives of 2-(p-chlorophenoxy)propionic acid. Mol. Pharmacol., 2002, 62, 265 - 271 and Liantonio, A. et al., Structural requisites of 2-(p-chlorophenoxy)propionic acid analogues for activity on native rat skeletal muscle chloride conductance and on heterologously expressed CLC-1. Br. J. Phamacol., 2003, 129, 1255 - 1264.

In the article Liantonio, A., Pusch, M., Picollo, A., Guida, P., De Luca, A., Pierno, S., Fracchiolla, G., Loiodice, F., Tortorella, P., Conte-Camerino, D. Investigations of pharmacologic properties of the renal CIC-K1 chloride channel co-expressed with barttin by the use of 2-(p-chlorophenoxy)propionic acid derivatives and other structurally unrelated chloride hannels blockers. Journal of the American Society of Nephrology, 2004, 15, 13 - 20, ligands for CIC-K1 chloride channels were disclosed.

In WO 2016/202341, Pedersen *et al.* reported a series of of phenoxypropionic acids and related compounds that appear to block the CIC-1 ion channel for use in treating, ameliorating and/or preventing neuromuscular disorders. However, they possess alternative structural features to those in the current disclosure.

### Summary

The present disclosure comprises a new series of compounds that alleviate disorders of the neuromuscular junction through inhibition of CIC-1 channels.

It has been found that a set of compounds that inhibit CIC-1 ion channels are capable of restoring neuromuscular transmission, as evidenced by the data generated by investigation of the compound set in biological models described herein. Compounds of the disclosure thus constitute a new group of potential drugs that can be used to treat or ameliorate muscle weakness and muscle fatigue in neuromuscular junction disorders caused by disease or by neuromuscular blocking agents.

The present disclosure thus concerns the discovery of CIC-1 ion channel inhibitors with application in the treatment of a range of conditions, such as reversal of block, ALS and myasthenic conditions, in which muscle activation by the nervous system is compromised and symptoms of weakness and fatigue are prominent.

In one aspect, the present disclosure concerns a compound of Formula A: 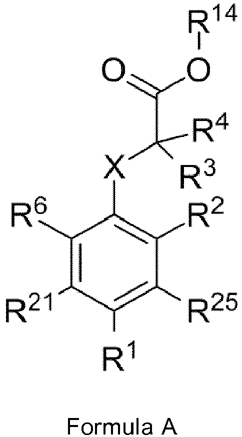 wherein
- X: is O, NR⁵, or CHR⁵;
- R¹: is Cl, Br F, I or H;
- R²: is H, a bond, -O-, C₁₋₃ alkyl or C₂₋₃ alkenyl, wherein said alkyl or alkenyl is optionally substituted with one or more F, =O, -OH, or phenyl optionally substituted with halogen;
- R³: is a bond, H, C₁₋₃ alkyl or C₂₋₃ alkenyl, wherein said alkyl or alkenyl is optionally substituted with one or more F, =O, -OH, or phenyl optionally substituted with halogen;
- R⁴: is a bond, H, C₁₋₃ alkyl or C₂₋₃ alkenyl;
- R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
- R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F;
- R²¹: is H, F or Br; and
- R²⁵: is H, F or Br;
and wherein at least one ring is formed by linking together:
a. R² and R³ to form a ring;
b. R² and R⁵ to form a ring;
c. R² and R³/R⁴ to form a ring, wherein R³ and R⁴ together form a double bond linked to R²; or
d. R³ and R⁵ to form a ring;
or a pharmaceutically acceptable salt, hydrate, polymorph, tautomer, or solvate thereof,
for use in treating, ameliorating and/or preventing a neuromuscular disorder, and/or for use in reversing and/or ameliorating a neuromuscular blockade.

In another aspect, the present disclosure concerns a compound of Formula A: 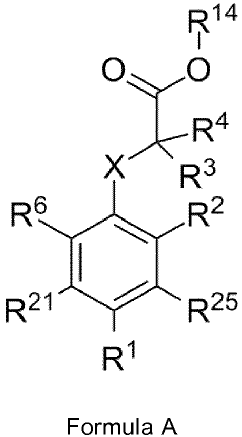 wherein
- X: is O, NR⁵, or CHR⁵;
- R¹: is Cl, Br F, I or H;
- R²: is H, a bond, -O-, C₁₋₃ alkyl or C₂₋₃ alkenyl, wherein said alkyl or alkenyl is optionally substituted with one or more F, =O, -OH, or phenyl optionally substituted with halogen;
- R³: is a bond, H, C₁₋₃ alkyl or C₂₋₃ alkenyl, wherein said alkyl or alkenyl is optionally substituted with one or more F, =O, -OH, or phenyl optionally substituted with halogen;
- R⁴: is a bond, H, C₁₋₃ alkyl or C₂₋₃ alkenyl;
- R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
- R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F;
- R²¹: is H, F or Br; and
- R²⁵: is H, F or Br;
and wherein at least one ring is formed by linking together:
a. R² and R³ to form a ring;
b. R² and R⁵ to form a ring;
c. R² and R³/R⁴ to form a ring, wherein R³ and R⁴ together form a double bond linked to R²; or
d. R³ and R⁵ to form a ring;
with the proviso that when X is CHR⁵ then R² is -O-; and
with the proviso that when R¹ is H, then X is NR⁵, R² is a bond, R⁵ is C₂ alkenyl optionally substituted with isoxazole, and R² and R⁵ are linked together to form a ring;
or a pharmaceutically acceptable salt, hydrate, polymorph, tautomer, or solvate thereof.

In yet another aspect, the disclosure concerns a composition comprising a compound as defined herein.

### Description of Drawings

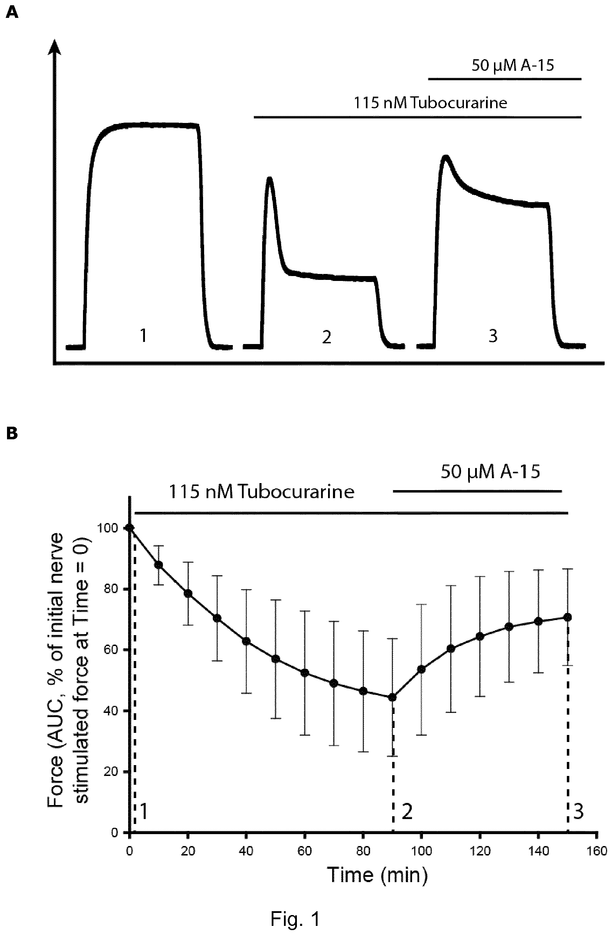

**Figure 1**. Panel A shows representative force traces before and after exposure to compound A-15. Force traces from a representative muscle stimulated to contract in 1) control condition before addition of neuromuscular blocking agent, 2) the force response to stimulation after 90 minutes incubation with Tubocurarine. Here the muscle displays severe neuromuscular transmission impediment, and 3) The muscle force response after addition of 50 µM compound A-15. Panel B shows average force (AUC) from 3 muscles relative to their initial force. The traces presented in panel A (1, 2, 3), correspond to the dotted lines in panel B, respectively. Thus, force is lost due to 90 min incubation in tubocurarine and is subsequently recovered when compound A-15 is added.

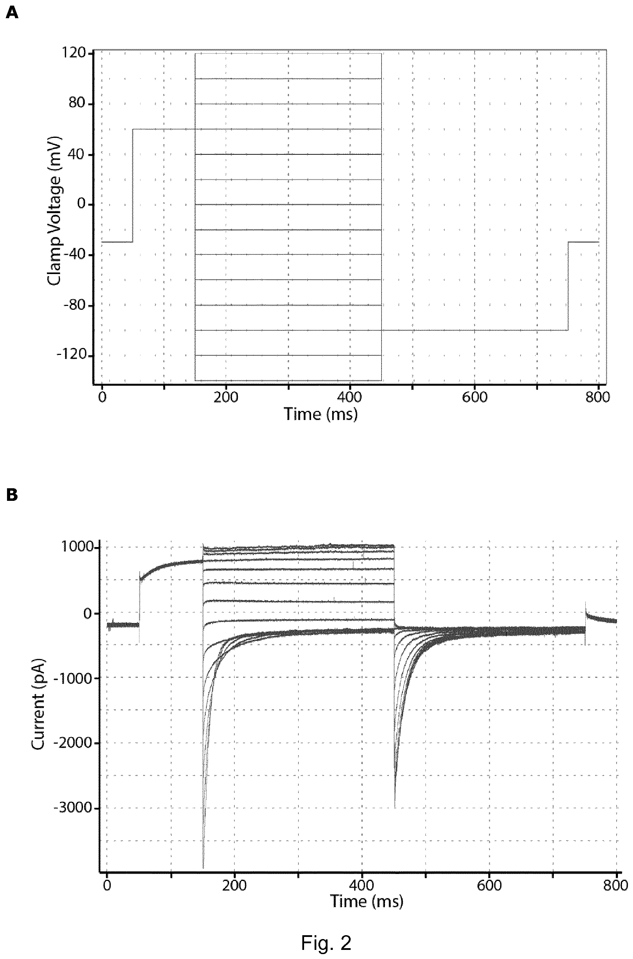

**Figure 2****:** Panel A illustrates the voltage protocol used to evoke currents in whole cell patches of CHO cells expressing human CIC-1 channels. Panel B shows representative whole cell current traces from a patched CHO cell expressing human CIC-1 channels. Currents were evoked by applying the voltage protocol shown in Panel A.

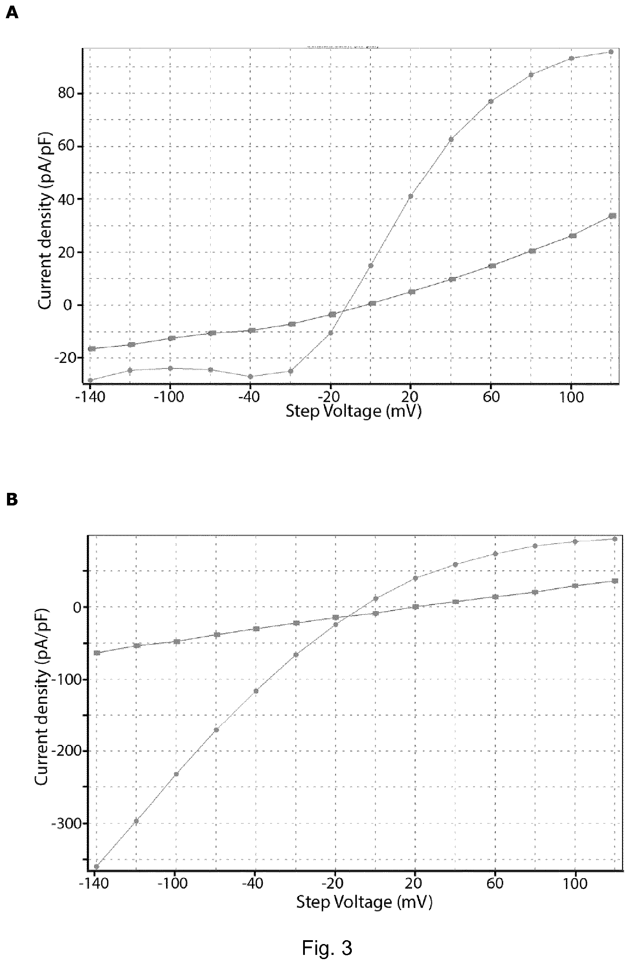

**Figure 3****:** Panel A shows a representative I/V plot of constant current density in a CIC-1 expressing CHO cell before (circles) and after (squares) application of 100 µM of the CIC-1 inhibitor, 9-anthracenecarboxylic acid (9-AC, Sigma A89405). Panel B shows a I/V plot of instant tail current density from the same CIC-1 expressing CHO cell as illustrated in Panel A, before (circles) and after (squares) application of 100 µM 9-AC.

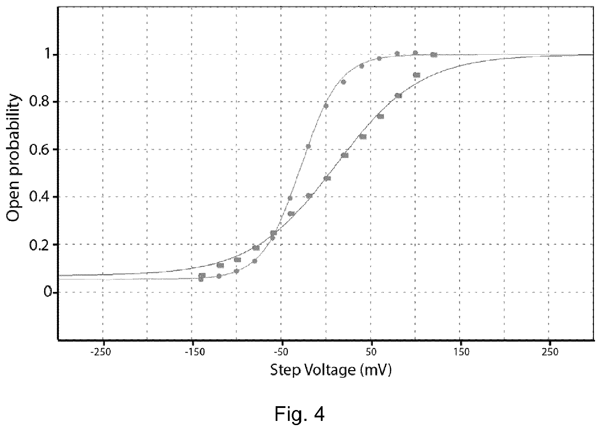

**Figure 4:** Figure 4 shows representative plots of normalized instant tail currents from a CIC-1 expressing CHO cell patch before (circles) and after (squares) application of 100 µM 9-AC. The instant tail currents at each voltage step were normalized to the maximal tail current obtained following the (+)120 mV voltage step and fitted to a Boltzmann function to determine the half activation potential, V_{1/2}.

### Definitions

The terms "C₁₋₃ alkyl" and "C₁₋₅ alkyl" refers to a branched or unbranched alkyl group having from one to three, one to five or two to five carbon atoms respectively, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl and pent-3-yl.
The term "C₃₋₅ cycloalkyl" and "C₃₋₆ cycloalkyl" refers to a group having three to five or three to six carbon atoms respectively including a monocyclic or bicyclic carbocycle, including but not limited to cyclopropyl, cyclobutyl, cyclopentyl and cyclohexyl.
The term "C₂₋₅ alkenyl" refers to a branched or unbranched alkenyl group having from two to five carbon atoms, two of which are connected by a double bond, including but not limited to ethenyl, propenyl, isopropenyl, butenyl, isobutenyl, pentenyl and isopentenyl.
The term "C₂₋₅ alkynyl" refers to a branched or unbranched alkynyl group having from one to five carbon atoms, two of which are connected by a triple bond, including but not limited to ethynyl, prop-1-ynyl, prop-2-ynyl, but-1-ynyl, but-2-ynyl, but-3-ynyl, buta-1,3-diynyl, pent-1-ynyl, pent-2-ynyl, pent-3-ynyl, pent-4-ynyl, penta-2,4-diynyl and penta-1,3-diynyl.
The term "5 membered aromatic heterocycle" refers to an aromatic group having five carbon atoms wherein between 1 and 3 carbon atoms in the ring have been replaced with a heteroatom selected from the group comprising nitrogen, sulphur and oxygen. Binding to the heterocycle may be at the position of the heteroatom or via a carbon atom of the heterocycle. 5-membered aromatic heterocycles include but are not limited to furan, thiophene, pyrrole, imidazole, pyrazole, oxazole, thiazole, isoxazole, isothiazole, 1,2,3-triazole, 1,2,4-triazole, 1,2,5-oxadiazole, 1,2,3-oxadiazole, 1,2,4-oxadiazole, 1,3,4-oxadiazole, 1,2,5-thiadiazole, 1,2,3-thiadiazole, 1,2,4-thiadiazole, and 1,3,4-thiadiazole.
The term "half-life" as used herein is the time it takes for the compound to lose one-half of its pharmacologic activity. The term "plasma half-life" is the time that it takes the compound to lose one-half of its pharmacologic activity in the blood plasma.
The term "treatment" refers to the combating of a disease or disorder. "Treatment" or "treating," as used herein, includes any desirable effect on the symptoms or pathology of a disease or condition as described herein, and may include even minimal changes or improvements in one or more measurable markers of the disease or condition being treated. "Treatment" or "treating" does not necessarily indicate complete eradication or cure of the disease or condition, or associated symptoms thereof. In some embodiments, the term "treatment" encompasses amelioration and prevention.
The term "amelioration" refers to moderation in the severity of the symptoms of a disease or condition. Improvement in a patient's condition, or the activity of making an effort to correct, or at least make more acceptable, conditions that are difficult to endure related to patient's conditions is considered "ameliorative" treatment.
The term "prevent" or "preventing" refers to precluding, averting, obviating, forestalling, stopping, or hindering something from happening, especially by advance action.
The term "reversal" or "reversing" refers to the ability of a compound to restore nerve-stimulated force in skeletal muscle exposed either *ex vivo* or *in vivo* to a non-depolarizing neuromuscular blocking agent or another pharmaceutical that is able to depress neuromuscular transmission
The term "non-depolarizing blockers" refers to pharmaceutical agents that antagonize the activation of acetylcholine receptors at the post-synaptic muscle fibre membrane by blocking the acetylcholine binding site on the receptor. These agents are used to block neuromuscular transmission and induce muscle paralysis in connection with surgery.
The term "ester hydrolysing reagent" refers to a chemical reagent which is capable of converting an ester functional group to a carboxylic acid with elimination of the alcohol moiety of the original ester, including but not limited to acid, base, a fluoride source, PBr₃, PCl₃ and lipase enzymes.
The term "recovery of force in muscle with neuromuscular dysfunction" refers to the ability of a compound to recover contractile force in nerve-stimulated healthy rat muscle after exposure to submaximal concentration of (115 nM) tubocurarine for 90 mins. Recovery of force is quantified as the percentage of the force prior to tubocurarine that is recovered by the compound.
The term "total membrane conductance (Gm)" is the electrophysiological measure of the ability of ions to cross the muscle fibre surface membrane. It reflects the function of ion channels that are active in resting muscle fibres of which CIC-1 is known to contribute around 80 % in most animal species.

### Detailed description

### Compounds

It is within the scope of the present disclosure to provide a compound for use in treating, ameliorating and/or preventing neuromuscular disorders characterized in that the neuromuscular function is reduced. As disclosed herein, inhibition of CIC-1 improves or restores neuromuscular function. The compounds of the present disclosure comprise compounds capable of inhibiting the CIC-1 channel thereby improving or restoring neuromuscular function. In one embodiment, the EC₅₀ of the compound is <50 µM, such as <40 µM, such as <30 µM, such as <20 µM, such as <15 µM, and such as <10 µM.

In one aspect, the present disclosure concerns a compound of Formula A: 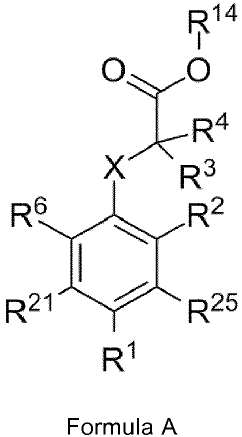 wherein
- X: is O, NR⁵, or CHR⁵;
- R¹: is Cl, Br F, I or H;
- R²: is H, a bond, -O-, C₁₋₃ alkyl or C₂₋₃ alkenyl, wherein said alkyl or alkenyl is optionally substituted with one or more F, =O, -OH, or phenyl optionally substituted with halogen;
- R³: is a bond, H, C₁₋₃ alkyl or C₂₋₃ alkenyl, wherein said alkyl or alkenyl is optionally substituted with one or more F, =O, -OH, or phenyl optionally substituted with halogen;
- R⁴: is a bond, H, C₁₋₃ alkyl or C₂₋₃ alkenyl;
- R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
- R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;

- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F;
- R²¹: is H, F or Br; and
- R²⁵: is H, F or Br;
and wherein at least one ring is formed by linking together:
a. R² and R³ to form a ring;
b. R² and R⁵ to form a ring;
c. R² and R³/R⁴ to form a ring, wherein R³ and R⁴ together form a double bond linked to R²; or
d. R³ and R⁵ to form a ring;
or a pharmaceutically acceptable salt, hydrate, polymorph, tautomer, or solvate thereof.

In one aspect, the compound is of Formula A: 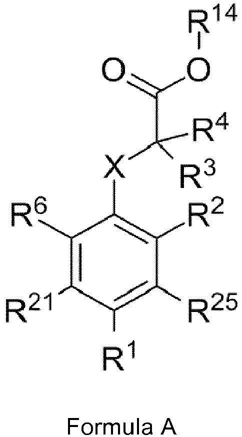 wherein
- X is: O, NR⁵, or CHR⁵;
- R¹: is Cl, Br F, I or H;
- R²: is H, a bond, -O-, C₁₋₃ alkyl or C₂₋₃ alkenyl, wherein said alkyl or alkenyl is optionally substituted with one or more F, =O, -OH, or phenyl optionally substituted with halogen;
- R³: is a bond, H, C₁₋₃ alkyl or C₂₋₃ alkenyl, wherein said alkyl or alkenyl is optionally substituted with one or more F, =O, -OH, or phenyl optionally substituted with halogen;
- R⁴: is a bond, H, C₁₋₃ alkyl or C₂₋₃ alkenyl;
- R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
- R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F;
- R²¹: is H, F or Br; and
- R²⁵: is H, F or Br;
and wherein at least one ring is formed by linking together:
a. R² and R³ to form a ring;
b. R² and R⁵ to form a ring;
c. R² and R³/R⁴ to form a ring, wherein R³ and R⁴ together form a double bond linked to R²; or
d. R³ and R⁵ to form a ring;
with the proviso that when X is CHR⁵ then R² is -O-; and
with the proviso that when R¹ is H, then X is NR⁵, R² is a bond, R⁵ is C₂ alkenyl optionally substituted with isoxazole, and R² and R⁵ are linked together to form a ring;
or a pharmaceutically acceptable salt, hydrate, polymorph, tautomer, or solvate thereof.

In one embodiment, X is O or NR⁵, and R² is a bond, C₁₋₃ alkyl or C₂₋₃ alkenyl, wherein said alkyl or alkenyl is optionally substituted with one or more F, =O, -OH, or phenyl optionally substituted with halogen.

In one aspect, the compound is of Formula B: 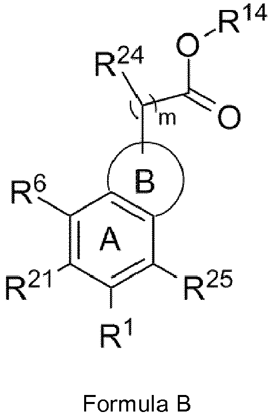 wherein
- B: is a 5-, 6-, or 7-membered heterocycle, optionally substituted with one or more substituents individually selected from the group consisting of C₁₋₃ alkyl, a 5-membered aromatic heterocycle optionally substituted with C₁₋₃ alkyl, =O, F, -OH and phenyl optionally substituted with halogen;
- m: is 0 or 1;
- R¹: is Cl, Br F or I;
- R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F; and
- R¹⁶: is independently selected from the group consisting of deuterium, methoxy, nitro, cyano, Cl, Br, I and F;
- R²⁴: is H or C₁₋₃ alkyl;
- R²¹: is H or F; and
- R²⁵: is H or F;
or a pharmaceutically acceptable salt, hydrate, polymorph, tautomer, or solvate thereof.

In one embodiment, rings A and B together constitutes a bicyclic structure selected from the group consisting of formulas AB1 to AB7:

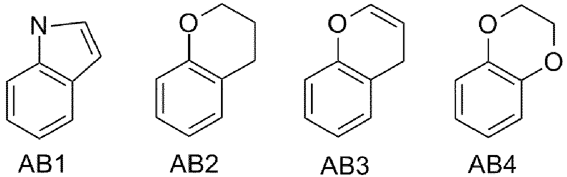

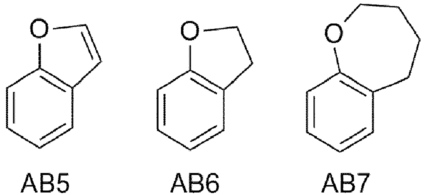

Said structures AB1 to AB7 may be substituted to fit into the Formulas defining the compound of the present disclosure.

In one embodiment, the compound is of Formula C: 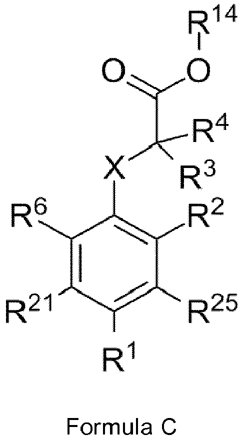 wherein
- X: is O or NR⁵;
- R¹: is Cl, Br, F or I;
- R²: is H; a bond, C₁₋₃ alkyl optionally substituted with one or more F, C₂₋₃ alkenyl optionally substituted with one or more F, =O, or phenyl optionally substituted with halogen;
- R³: is a bond, H, C₁₋₃ alkyl or C₂₋₃ alkenyl;
- R⁴: is a bond, H, C₁₋₃ alkyl or C₂₋₃ alkenyl;
- R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
- R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;

- R¹⁵: is independently selected from the group consisting of deuterium and F;
- R¹⁶: is independently selected from the group consisting of deuterium, methoxy, nitro, cyano, Cl, Br, I and F;
- R²¹: is H or F; and
- R²⁵: is H or F;
and wherein at least one ring is formed by linking together:
a. R² and R³ to form a ring;
b. R² and R⁵ to form a ring; or
c. R² and R³/R⁴ to form a ring, wherein R³ and R⁴ together form a double bond linked to R²;
or a pharmaceutically acceptable salt, hydrate, polymorph, tautomer, or solvate thereof.

In one embodiment, R⁶ is a 5-membered aromatic heterocycle, such as isoxazole, optionally substituted with C₁₋₃ alkyl. In one embodiment, R⁶ is isoxazol-3-yl or isoxazol-5-yl.

In one embodiment, R⁶ is C₂₋₄ alkyl, optionally substituted with oxime. In one embodiment, R⁶ is H. In one embodiment, R⁶ is Br, Cl or F, such as R⁶ is Br.

In one embodiment, R²¹ is H. In one embodiment, R²⁵ is H. In one embodiment, R²¹ and R²⁵ are H. In one embodiment, R²¹ is H and R²⁵ is F. In one embodiment, R²¹ is F and R²⁵ is H. In one embodiment, R²¹ is Br. In one embodiment, R²⁵ is Br.

In one embodiment, R² and R³ are linked together to form a ring. Said ring formed by R² and R³ may result in a bicyclic moiety selected from the group consisting AB1, AB2, AB3, AB4, AB5, AB6 and AB7.

In some embodiments, X is O.

In one embodiment, X is O and R² and R³ are linked together to form a ring. Hence, in one embodiment, the compound is of Formula D: 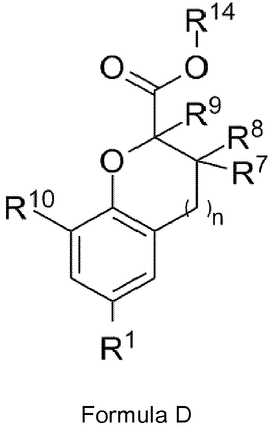 wherein
- R¹: is Cl, Br, F or I;
- R⁷: is H, F or C₁₋₅ alkyl optionally substituted with one or more, identical or different, substituents R¹⁵;
- R⁸: is H or F;
- R⁹: is H or methyl;
- R¹⁰: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F; and
- n: is 0, 1 or 2;
or a pharmaceutically acceptable salt, hydrate, polymorph, tautomer, or solvate thereof.

In one embodiment, the compound is of Formula D1. In one embodiment, the compound is of Formula D2.

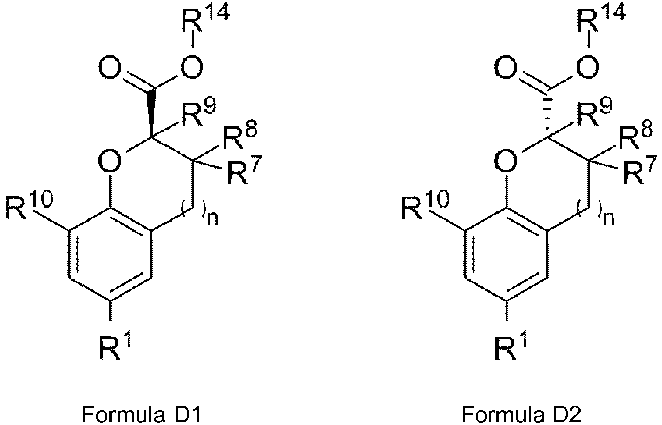

In one embodiment, R¹⁰ is a 5-membered aromatic heterocycle, optionally substituted with C₁₋₃ alkyl. In one embodiment, R¹⁰ is isoxazol-3-yl, isoxazol-4-yl, or isoxazol-5-yl, optionally substituted with C₁₋₃ alkyl. In one embodiment, R¹⁰ is isoxazol-3-yl.

In one embodiment, R¹⁰ is Br. In one embodiment, R¹⁰ is -C(=NOH)CH₃.

In one embodiment, n is 1. When n is 1, the compound of any one of Formulas B, B1 and B2 may comprise a bicyclic structure of Formula AB2. In another embodiment, n is 0. When n is 0, the compound of any one of Formulas B, B1 and B2 may comprise a bicyclic structure of Formula AB6. In another embodiment, n is 2. When n is 2, the compound of any one of Formulas B, B1 and B2 may comprise a bicyclic structure of Formula AB7.

In one embodiment, R⁷ is H. In one embodiment, R⁸ is H. In one embodiment, R⁹ is H. In one embodiment, R¹⁴ is H. In one embodiment, R⁷, R⁸, R⁹, and R¹⁴ are H. In one embodiment, R⁷, R⁸, and R⁹ are H and R¹⁰ is isoxazol-3-yl.

In one embodiment, R¹ is Br or Cl. In one embodiment, R¹ is Br. In one embodiment, R¹ is CI.

In one embodiment, X is O and R² and R³ of Formula H or Formula A are linked together to form a 5-membered ring. Hence, in one embodiment, and the compound is of Formula E: 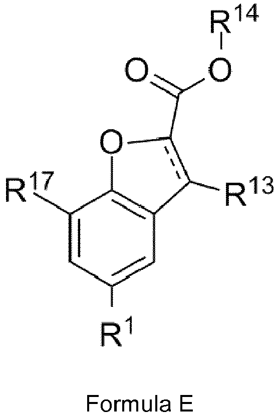 wherein
-̅ -̅ -̅ represents a single or a double bond;
- R¹: is Cl, Br, F or I;
- R¹³: is H or phenyl optionally substituted with halogen;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F; and
- R¹⁷: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
or a pharmaceutically acceptable salt, hydrate, polymorph, tautomer, or solvate thereof.

In one embodiment, -̅ -̅ -̅ of Formula E represents a single bond. When -̅ -̅ -̅ represents a single bond, the compound of Formula E may comprise a bicyclic structure of Formula AB6. In one embodiment, -̅ -̅ -̅ of Formula E represents a double bond. When -̅ -̅ -̅ represents a double bond, the compound of Formula E may comprise a bicyclic structure of Formula AB5.

In one embodiment, R¹⁷ is H. In one embodiment, R¹⁷ is a 5-membered aromatic heterocycle, optionally substituted with C₁₋₃ alkyl. In one embodiment, R¹⁷ is isoxazole, such as isoxazol-3-yl, isoxazol-4-yl or isoxazol-5-yl, optionally substituted with C₁₋₃ alkyl. In one embodiment, R¹⁷ is isoxazol-3-yl. In one embodiment, R¹⁷ is isoxazol-5-yl.

In one embodiment, R¹⁴ is H. In one embodiment, R¹³, R¹⁴, and R¹⁷ are H. In one embodiment, R¹³ and R¹⁴ are H and R¹⁷ is isoxazol-3-yl, isoxazol-4-yl or isoxazol-5-yl.

In one embodiment, R¹ is Br or Cl. In one embodiment, R¹ is Br. In one embodiment, R¹ is CI.

In one embodiment, the compound is of Formula F: 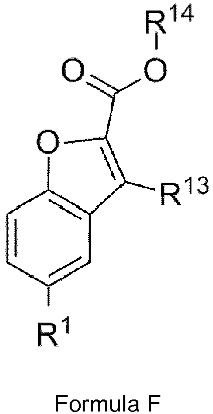 wherein
- R¹: is Cl, Br, F or I;
- R¹³: is phenyl optionally substituted with halogen;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F; and
- R¹⁶: is independently selected from the group consisting of deuterium, methoxy, nitro, cyano, Cl, Br, I and F;
or a pharmaceutically acceptable salt, hydrate, polymorph, tautomer, or solvate thereof.

In one embodiment, R¹³ is H. In one embodiment, R¹³ is phenyl substituted with halogen, such as phenyl substituted with one Cl.

In one embodiment, X is O and R² and R³ are linked together to form a ring. In one embodiment, R³ and R⁴ together constitutes a double bond, which is linked to R² to form a ring. Hence, in one embodiment, the compound is is of Formula G: 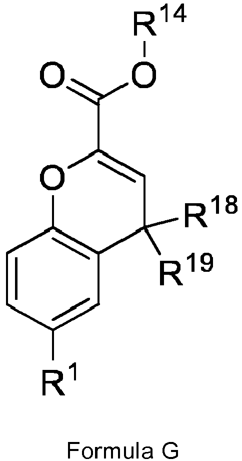 wherein
- R¹: is Cl, Br, F or I;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F; and
- R¹⁸ and R¹⁹: are F, or R¹⁸ is =O and R¹⁹ is absent;
or a pharmaceutically acceptable salt, hydrate, polymorph, tautomer, or solvate thereof.

In one embodiment, X is NR⁵.

In one embodiment, X is NR⁵ and R² and R⁵ are linked together to form a ring. In one embodiment, R² is C₂ alkenyl, optionally substituted with a 5-membered aromatic heterocycle, such as isoxazole, optionally substituted with C₁₋₃ alkyl. In one embodiment, the compound comprises a structure of Formula AB1. Hence, in one embodiment, the compound is of Formula H: 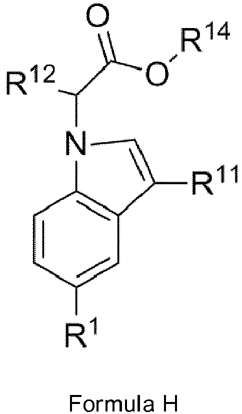 wherein
- R¹: is Cl, Br, F or I;
- R¹¹: is H or a 5-membered aromatic heterocycle, such as isoxazole, optionally substituted with C₁₋₃ alkyl;
- R¹²: is H, C₁₋₅ alkyl, or C₃₋₆ cycloalkyl;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F; and
- R¹⁶: is independently selected from the group consisting of deuterium, methoxy, nitro, cyano, Cl, Br, I and F;
or a pharmaceutically acceptable salt, hydrate, polymorph, tautomer, or solvate thereof.

In one embodiment, the compound is of Formula H1 or Formula H2:

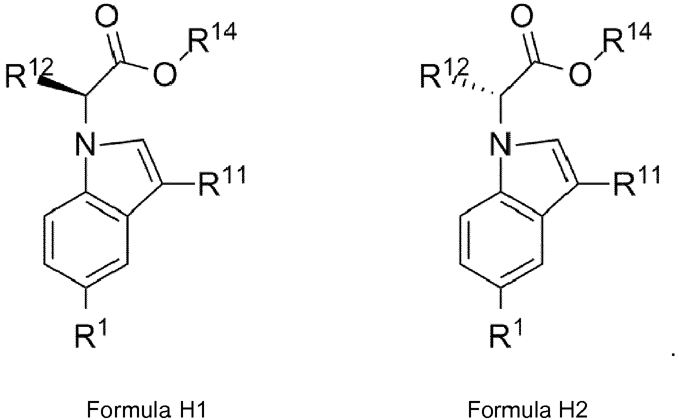

In one embodiment, R¹¹ is a 5-membered aromatic heterocycle. In one embodiment, R¹¹ is not a pyrazole. In one embodiment, R¹¹ is H. In one embodiment, R¹¹ is isoxazole, optionally substituted with C₁₋₃ alkyl. In one embodiment, R¹¹ is isoxazol-3-yl, isoxazol-4-yl, or isoxazol-5-yl.

In one embodiment, R¹² is H. In one embodiment, R¹² is C₁₋₅ alkyl. In one embodiment, R¹² is methyl.

In one embodiment, when R¹² is H or methyl then R¹¹ is not H.

In one embodiment, R¹⁴ is H. In one embodiment, R¹¹ is isoxazol-3-yl, isoxazol-4-yl, or isoxazol-5-yl, R¹² is H and R¹⁴ is H.

In one embodiment, R¹ is Br or Cl. In one embodiment, R¹ is Br. In one embodiment, R¹ is Cl.

In one embodiment, X is NR⁵, R²⁵ is H, and R³ and R⁵ are linked together to form a 5-membered ring. Thus, in one embodiment, the compound is of Formula I: 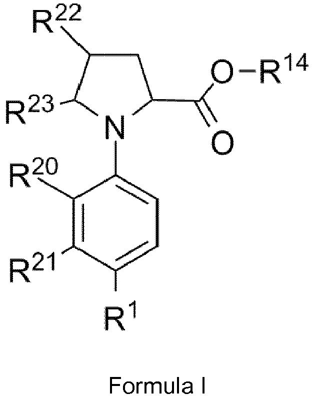 wherein
- R¹: is Br, Cl, F or I;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F;
- R²⁰: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
- R²¹: is H or F;
- R²²: is H or-OH;
- R²³: is H or =O;
or a pharmaceutically acceptable salt, hydrate, polymorph, tautomer, or solvate thereof.

In one embodiment, X is NR⁵, R²¹ and R²⁵ are H, and R³ and R⁵ are linked together to form a 5-membered ring. Thus, in one embodiment, the compound is of Formula J: 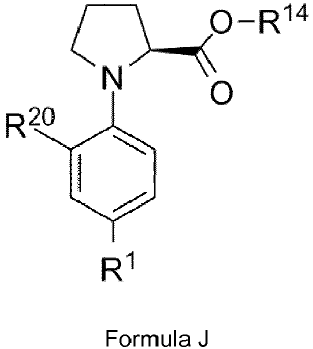 wherein
- R¹: is Br, Cl, F or I;
- R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
- R¹⁵: is independently selected from the group consisting of deuterium and F;
- R¹⁶: is independently selected from the group consisting of deuterium, methoxy, nitro, cyano, Cl, Br, I and F; and
- R²⁰: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
or a pharmaceutically acceptable salt, hydrate, polymorph, tautomer, or solvate thereof.

In one embodiment, R²⁰ is H. In one embodiment, R²⁰ is Br, Cl, or F. In one embodiment, R²⁰ is a 5-membered aromatic heterocycle, optionally substituted with a C₁₋₃ alkyl. In one embodiment, R²⁰ is isoxazole such as isoxazol-3-yl, isoxazol-4-yl or isoxazol-5-yl, optionally substituted with C₁₋₃ alkyl.

In one embodiment, R¹ is Br or Cl. In one embodiment, R¹ is Br. In one embodiment, R¹ is CI.

In one embodiment, R¹⁴ is H.

In one embodiment, said 5-membered aromatic heterocycle is individually selected from the group consisting of isoxazole, oxazole, furan, thiophene, pyrrole, imidazole, pyrazole, thiazole, isothiazole, 1,2,3-triazole, 1,2,4-triazole, 1,2,5-oxadiazole, 1,2,3-oxadiazole, 1,2,4-oxadiazole, 1,3,4-oxadiazole, 1,2,5-thiadiazole, 1,2,3-thiadiazole, 1,2,4-thiadiazole, and 1,3,4-thiadiazole.

In one embodiment, said isoxazole is isoxazol-3-yl, isoxazol-4-yl or isoxazol-5-yl.

In one embodiment, said phenyl optionally substituted with halogen is a phenyl substituted with Cl.

In one embodiment, R⁶ is R¹⁰, R¹⁷, or R²⁰. In one embodiment, R¹⁰ is R⁶, R¹⁷, or R²⁰. In one embodiment, R¹⁷ is R⁶, R¹⁰, or R²⁰. In one embodiment, R²⁰ is R⁶, R¹⁰, or R¹⁷.

In one embodiment, the compound is selected from the group consisting of Formula A-1 to A-33.

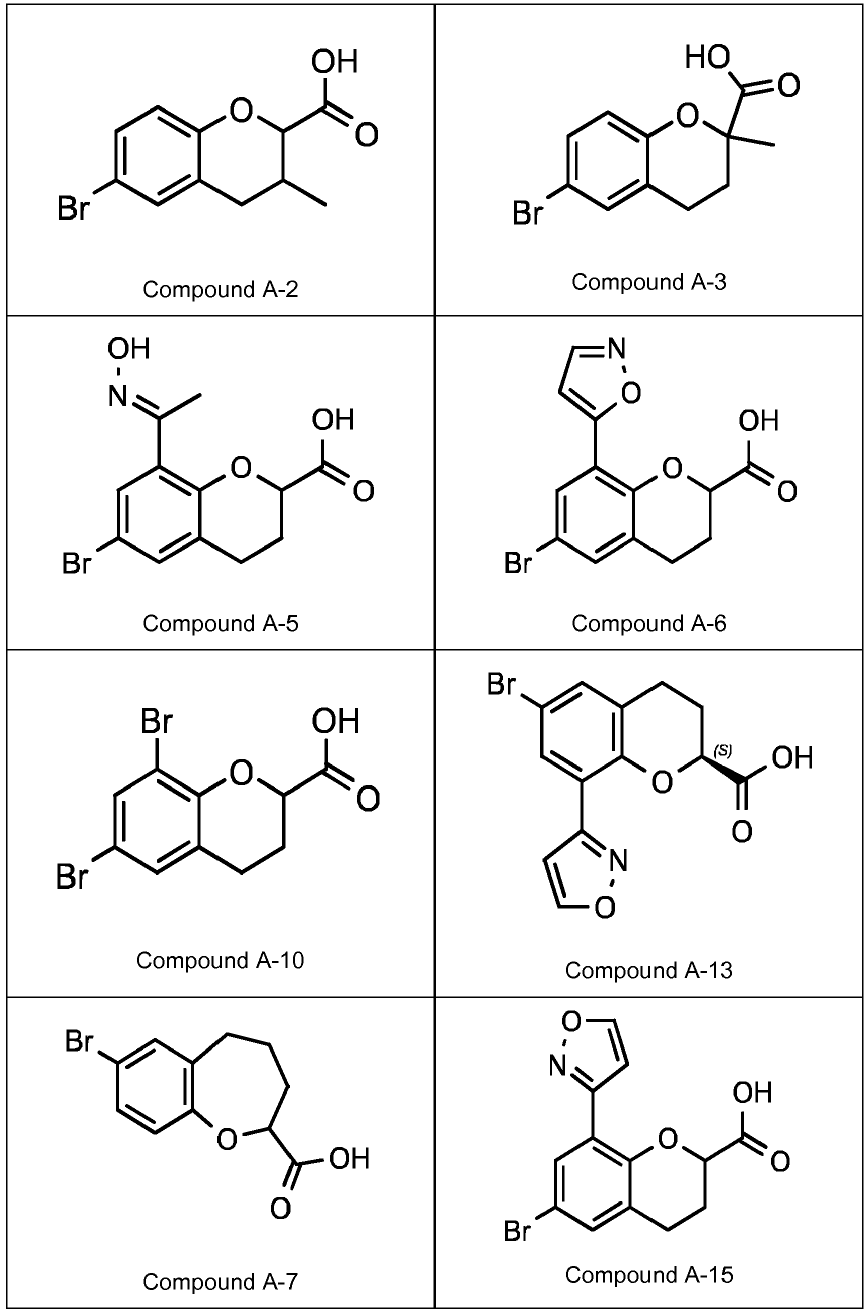

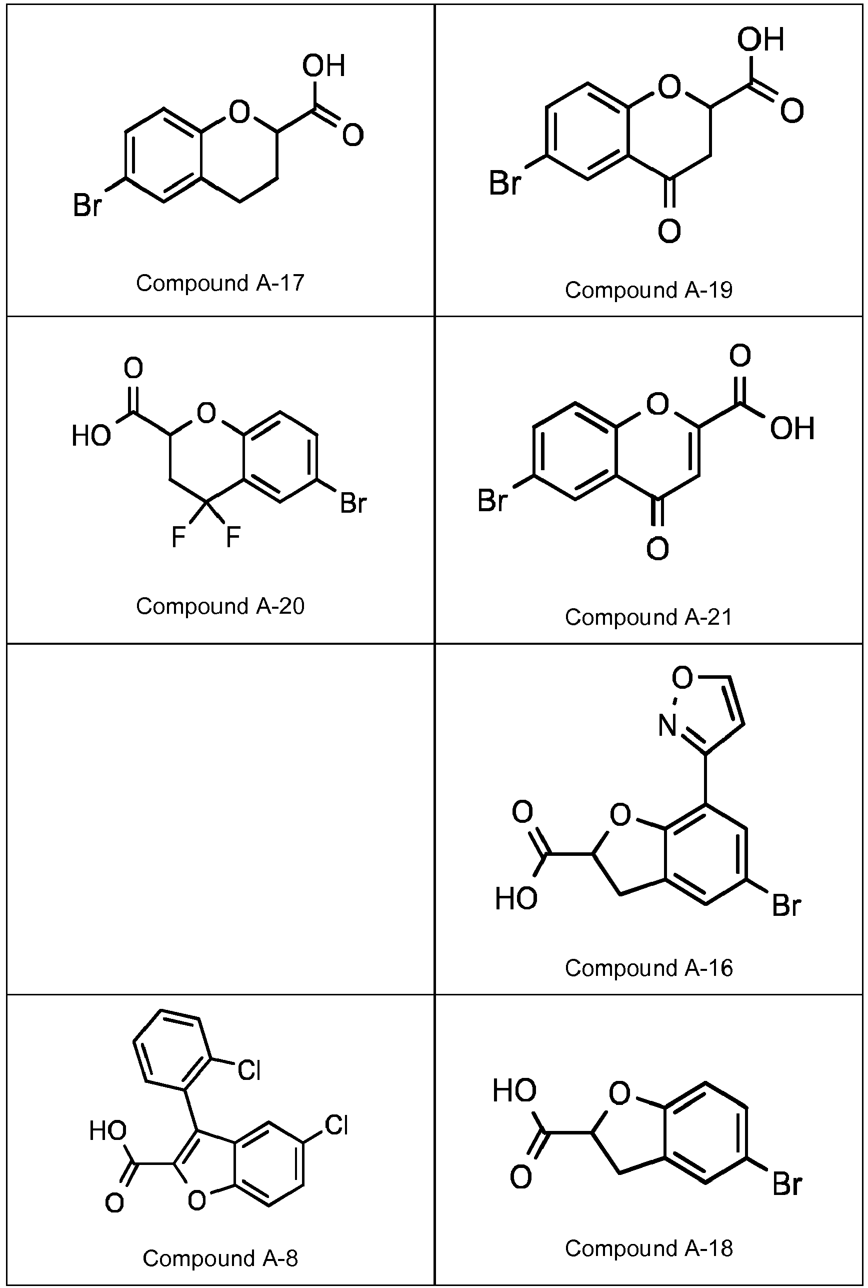

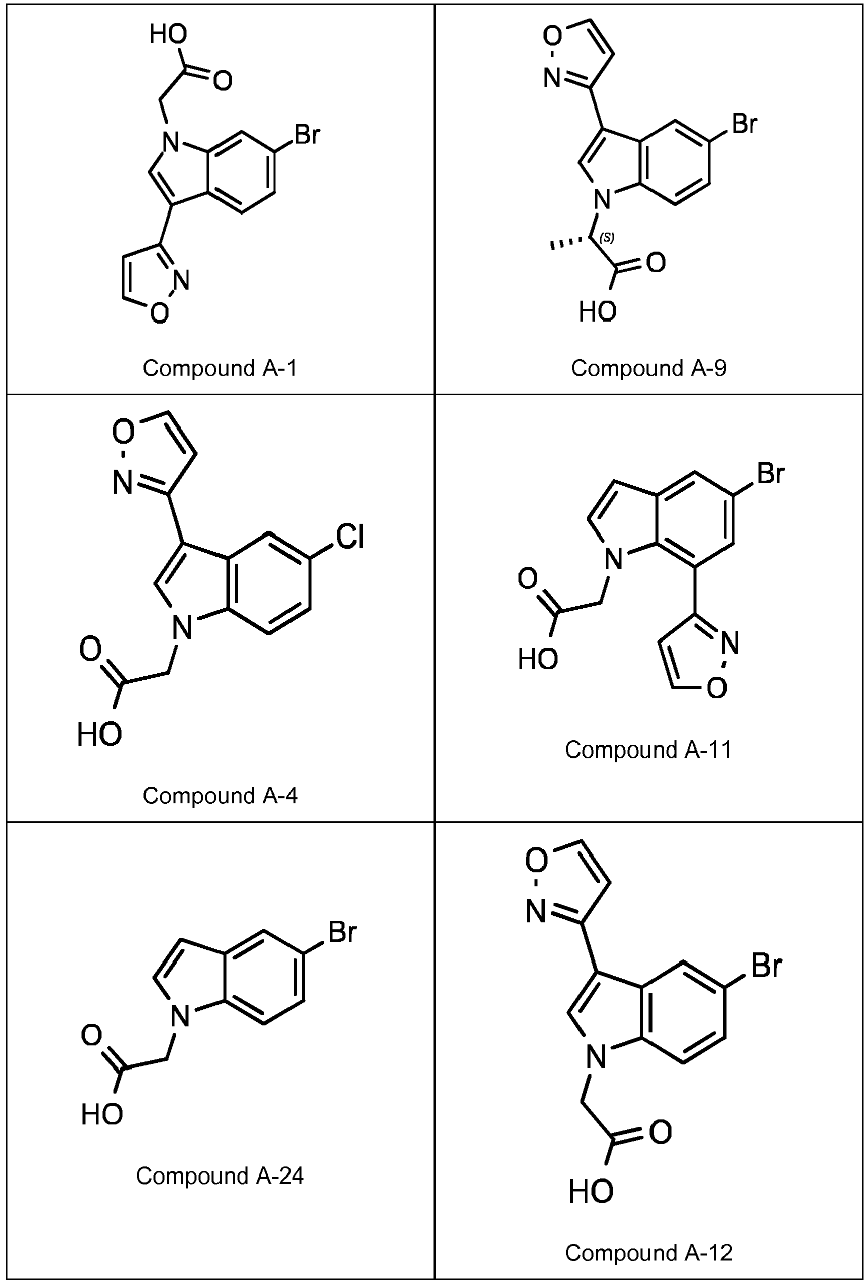

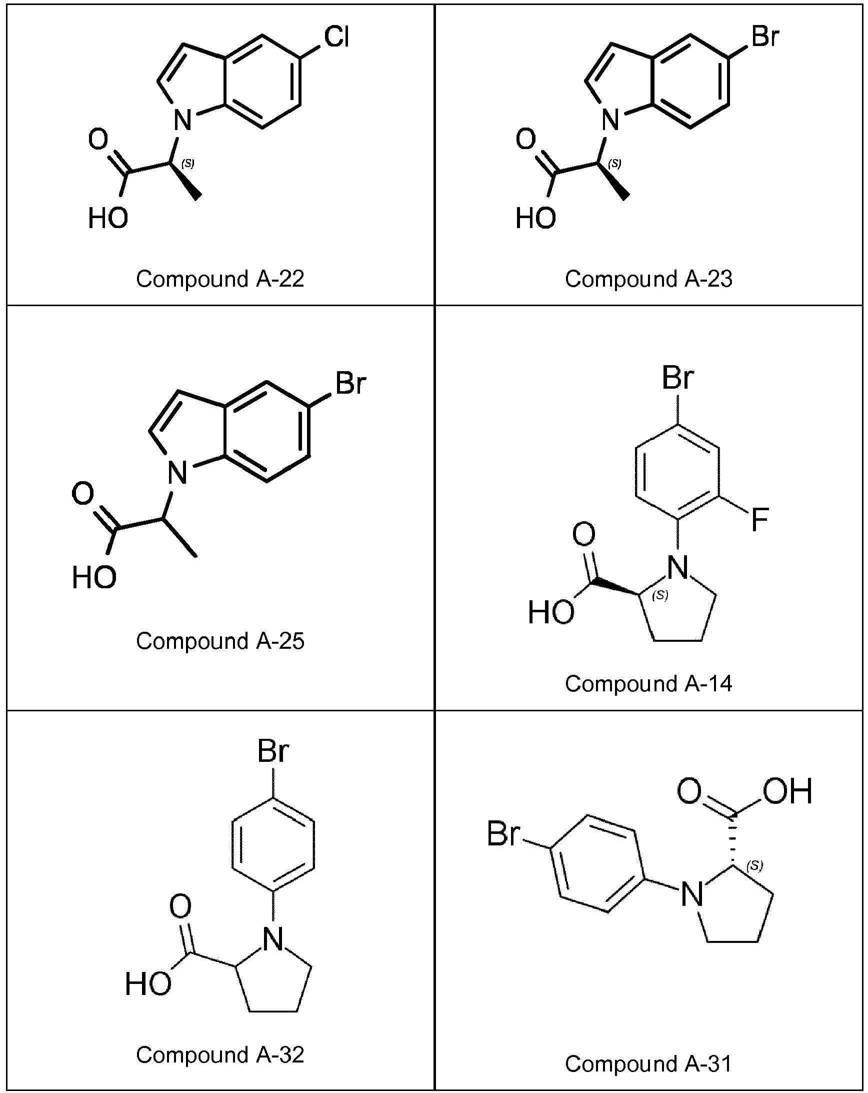

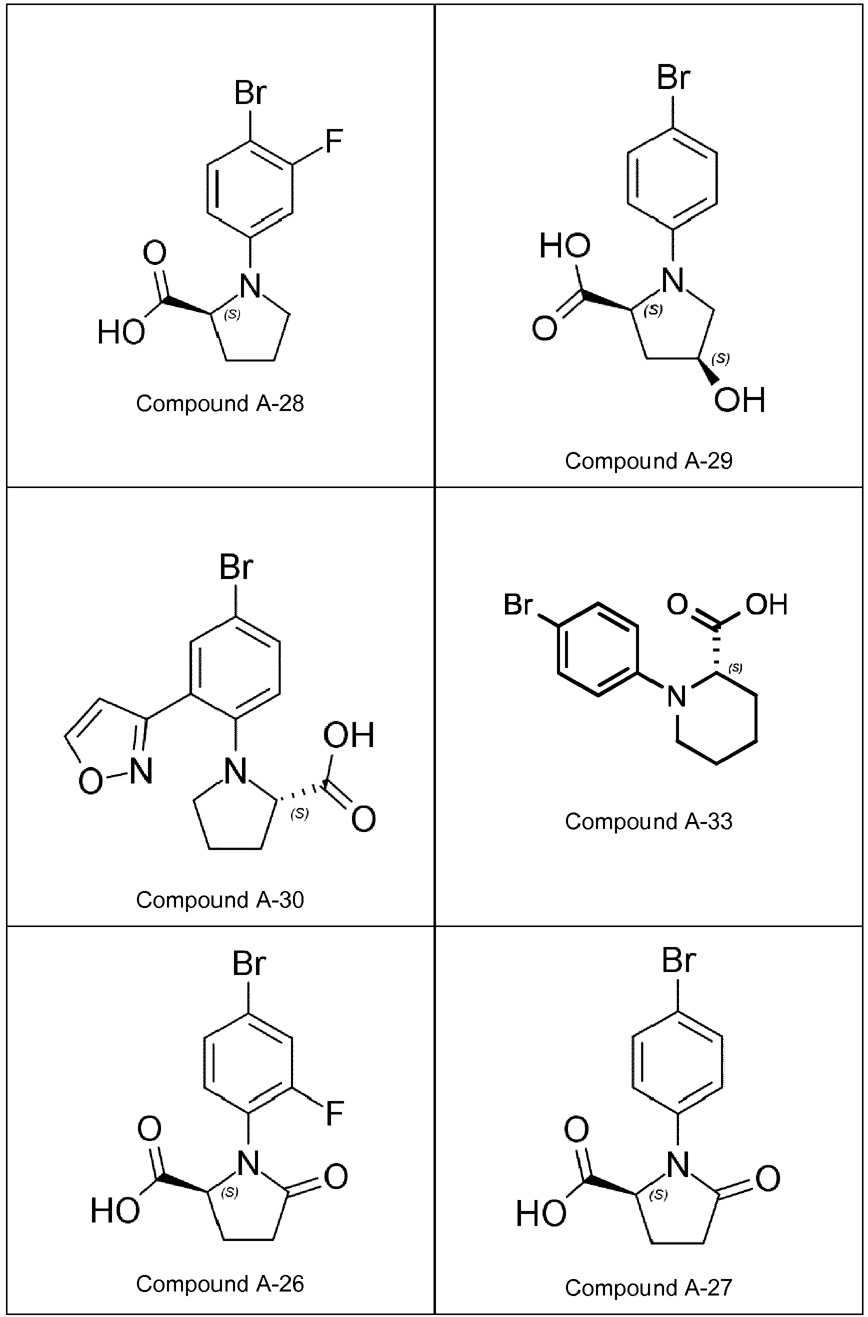

In one embodiment, the compound is selected from the group consisting of:
6-bromo-3-methyl-3,4-dihydro-2H-1-benzopyran-2-carboxylic acid;
6-bromo-2-methyl-3,4-dihydro-2H-1-benzopyran-2-carboxylic acid;
6-bromo-8-(1,2-oxazol-5-yl)-3,4-dihydro-2H-1-benzopyran-2-carboxylic acid;
6-bromo-8-[(1E)-1-(hydroxyimino)ethyl]-3,4-dihydro-2H-1-benzopyran-2-carboxylic acid;
6,8-dibromo-3,4-dihydro-2H-1-benzopyran-2-carboxylic acid;
(2S)-6-bromo-8-(1,2-oxazol-3-yl)-3,4-dihydro-2H-1-benzopyran-2-carboxylic acid;
6-bromo-8-(1,2-oxazol-3-yl)-3,4-dihydro-2H-1-benzopyran-2-carboxylic acid;
(2R)-6-bromo-8-(1,2-oxazol-3-yl)-3,4-dihydro-2H-1-benzopyran-2-carboxylic acid;
6-bromo-3,4-dihydro-2H-1-benzopyran-2-carboxylic acid;
6-bromo-4-oxo-3,4-dihydro-2H-1-benzopyran-2-carboxylic acid;
6-bromo-4-oxo-4H-chromene-2-carboxylic acid;
6-bromo-4,4-difluoro-3,4-dihydro-2H-1-benzopyran-2-carboxylic acid;
7-bromo-2,3,4,5-tetrahydro-1-benzoxepine-2-carboxylic acid;
5-bromo-7-(1,2-oxazol-3-yl)-2,3-dihydro-1-benzofuran-2-carboxylic acid;
5-bromo-2,3-dihydro-1-benzofuran-2-carboxylic acid;
5-chloro-3-(2-chlorophenyl)-1-benzofuran-2-carboxylic acid;
2-[6-bromo-3-(1,2-oxazol-3-yl)-1H-indol-1-yl]acetic acid;
(2S)-2-[5-bromo-3-(1,2-oxazol-3-yl)-1H-indol-1-yl]propanoic acid;
2-[5-bromo-7-(1,2-oxazol-3-yl)-1H-indol-1-yl]acetic acid;
2-[5-chloro-3-(1,2-oxazol-3-yl)-1H-indol-1-yl]acetic acid;
(2R)-2-(5-chloro-1H-indol-1-yl)propanoic acid;
2-[5-bromo-3-(1,2-oxazol-3-yl)-1H-indol-1-yl]acetic acid;
(2S)-2-(5-chloro-1H-indol-1-yl)propanoic acid;
(2S)-2-(5-bromo-1H-indol-1-yl)propanoic acid;
2-(5-bromo-1H-indol-1-yl)acetic acid;
(2R)-2-(5-bromo-1H-indol-1-yl)propanoic acid;
2-(5-bromo-1H-indol-1-yl)propanoic acid;
(2S)-1-(4-bromo-2-fluorophenyl)pyrrolidine-2-carboxylic acid;
1-(4-bromophenyl)pyrrolidine-2-carboxylic acid;
(2S)-1-(4-bromophenyl)pyrrolidine-2-carboxylic acid;
(2S)-1-(4-bromo-3-fluorophenyl)pyrrolidine-2-carboxylic acid;
(2S,4S)-1-(4-bromophenyl)-4-hydroxypyrrolidine-2-carboxylic acid;
(2R)-1-(4-bromophenyl)pyrrolidine-2-carboxylic acid;
(2S)-1-[4-bromo-2-(1,2-oxazol-3-yl)phenyl]pyrrolidine-2-carboxylic acid;
(2S)-1-(4-bromophenyl)-5-oxopyrrolidine-2-carboxylic acid; and
(2S)-1-(4-bromo-2-fluorophenyl)-5-oxopyrrolidine-2-carboxylic acid.

In one embodiment, the compound is selected from the group consisting of:
6-bromo-3-methyl-3,4-dihydro-2H-1-benzopyran-2-carboxylic acid;
6-bromo-2-methyl-3,4-dihydro-2H-1-benzopyran-2-carboxylic acid;
6-bromo-8-(1,2-oxazol-5-yl)-3,4-dihydro-2H-1-benzopyran-2-carboxylic acid;
6-bromo-8-[(1E)-1-(hydroxyimino)ethyl]-3,4-dihydro-2H-1-benzopyran-2-carboxylic acid;
6,8-dibromo-3,4-dihydro-2H-1-benzopyran-2-carboxylic acid;
(2S)-6-bromo-8-(1,2-oxazol-3-yl)-3,4-dihydro-2H-1-benzopyran-2-carboxylic acid;
6-bromo-8-(1,2-oxazol-3-yl)-3,4-dihydro-2H-1-benzopyran-2-carboxylic acid;
(2R)-6-bromo-8-(1,2-oxazol-3-yl)-3,4-dihydro-2H-1-benzopyran-2-carboxylic acid;
7-bromo-2,3,4,5-tetrahydro-1-benzoxepine-2-carboxylic acid;
5-bromo-7-(1,2-oxazol-3-yl)-2,3-dihydro-1-benzofuran-2-carboxylic acid;
2-[6-bromo-3-(1,2-oxazol-3-yl)-1H-indol-1-yl]acetic acid;
(2S)-2-[5-bromo-3-(1,2-oxazol-3-yl)-1H-indol-1-yl]propanoic acid;
2-[5-bromo-7-(1,2-oxazol-3-yl)-1H-indol-1-yl]acetic acid;
2-[5-chloro-3-(1,2-oxazol-3-yl)-1H-indol-1-yl]acetic acid;
(2R)-2-(5-chloro-1H-indol-1-yl)propanoic acid;
2-[5-bromo-3-(1,2-oxazol-3-yl)-1H-indol-1-yl]acetic acid;
(2S)-2-(5-chloro-1H-indol-1-yl)propanoic acid;
(2R)-2-(5-bromo-1H-indol-1-yl)propanoic acid;
(2S)-1-(4-bromo-2-fluorophenyl)pyrrolidine-2-carboxylic acid;
(2S)-1-(4-bromo-3-fluorophenyl)pyrrolidine-2-carboxylic acid;
(2S,4S)-1-(4-bromophenyl)-4-hydroxypyrrolidine-2-carboxylic acid; and
(2S)-1-[4-bromo-2-(1,2-oxazol-3-yl)phenyl]pyrrolidine-2-carboxylic acid.

In one embodiment, the compound is selected from the group consisting of:
6-bromo-3-methyl-3,4-dihydro-2H-1-benzopyran-2-carboxylic acid;
6-bromo-2-methyl-3,4-dihydro-2H-1-benzopyran-2-carboxylic acid;
6-bromo-8-(1,2-oxazol-5-yl)-3,4-dihydro-2H-1-benzopyran-2-carboxylic acid;
6-bromo-8-[(1E)-1-(hydroxyimino)ethyl]-3,4-dihydro-2H-1-benzopyran-2-carboxylic acid;
6,8-dibromo-3,4-dihydro-2H-1-benzopyran-2-carboxylic acid;
(2S)-6-bromo-8-(1,2-oxazol-3-yl)-3,4-dihydro-2H-1-benzopyran-2-carboxylic acid;
6-bromo-8-(1,2-oxazol-3-yl)-3,4-dihydro-2H-1-benzopyran-2-carboxylic acid;
(2R)-6-bromo-8-(1,2-oxazol-3-yl)-3,4-dihydro-2H-1-benzopyran-2-carboxylic acid;
6-bromo-3,4-dihydro-2H-1-benzopyran-2-carboxylic acid;
6-bromo-4-oxo-3,4-dihydro-2H-1-benzopyran-2-carboxylic acid; and
6-bromo-4-oxo-4H-chromene-2-carboxylic acid.

In one embodiment, the compound is selected from the group consisting of:
2-[6-bromo-3-(1,2-oxazol-3-yl)-1H-indol-1-yl]acetic acid;
(2S)-2-[5-bromo-3-(1,2-oxazol-3-yl)-1H-indol-1-yl]propanoic acid;
2-[5-bromo-7-(1,2-oxazol-3-yl)-1H-indol-1-yl]acetic acid;
2-[5-chloro-3-(1,2-oxazol-3-yl)-1H-indol-1-yl]acetic acid;
(2R)-2-(5-chloro-1H-indol-1-yl)propanoic acid;
2-[5-bromo-3-(1,2-oxazol-3-yl)-1H-indol-1-yl]acetic acid;
(2S)-2-(5-chloro-1H-indol-1-yl)propanoic acid;
(2S)-2-(5-bromo-1H-indol-1-yl)propanoic acid;
2-(5-bromo-1H-indol-1-yl)acetic acid;
(2R)-2-(5-bromo-1H-indol-1-yl)propanoic acid; and
2-(5-bromo-1H-indol-1-yl)propanoic acid.

In one embodiment, the compound is selected from the group consisting of:
(2S)-1-(4-bromo-2-fluorophenyl)pyrrolidine-2-carboxylic acid;
1-(4-bromophenyl)pyrrolidine-2-carboxylic acid;
(2S)-1-(4-bromophenyl)pyrrolidine-2-carboxylic acid;
(2S)-1-(4-bromo-3-fluorophenyl)pyrrolidine-2-carboxylic acid;
(2S,4S)-1-(4-bromophenyl)-4-hydroxypyrrolidine-2-carboxylic acid;
(2R)-1-(4-bromophenyl)pyrrolidine-2-carboxylic acid;
(2S)-1-[4-bromo-2-(1,2-oxazol-3-yl)phenyl]pyrrolidine-2-carboxylic acid;
(2S)-1-(4-bromophenyl)-5-oxopyrrolidine-2-carboxylic acid; and
(2S)-1-(4-bromo-2-fluorophenyl)-5-oxopyrrolidine-2-carboxylic acid.

In one embodiment, the compound is selected from the group consisting of:
6-bromo-8-(1,2-oxazol-5-yl)-3,4-dihydro-2H-1-benzopyran-2-carboxylic acid;
(2S)-6-bromo-8-(1,2-oxazol-3-yl)-3,4-dihydro-2H-1-benzopyran-2-carboxylic acid;
6-bromo-8-(1,2-oxazol-3-yl)-3,4-dihydro-2H-1-benzopyran-2-carboxylic acid;
(2R)-6-bromo-8-(1,2-oxazol-3-yl)-3,4-dihydro-2H-1-benzopyran-2-carboxylic acid;
5-bromo-7-(1,2-oxazol-3-yl)-2,3-dihydro-1-benzofuran-2-carboxylic acid;
2-[5-bromo-7-(1,2-oxazol-3-yl)-1H-indol-1-yl]acetic acid; and
(2S)-1-[4-bromo-2-(1,2-oxazol-3-yl)phenyl]pyrrolidine-2-carboxylic acid.

### Methods of treatment

In one aspect, the disclosure relates to the use of compounds of Formula (A), Formula (B), Formula (C), Formula (D), Formula (E), Formula (F), Formula (G), Formula (H), Formula (I), and/or Formula (J) as defined herein in the treatment of a neuromuscular disorder. In some embodiments, said treatment of a neuromuscular disorder includes treating, ameliorating and/or preventing the neuromuscular disorder. In one aspect, the disclosure relates to the use of compounds of Formula (A), Formula (B), Formula (C), Formula (D), Formula (E), Formula (F), Formula (G), Formula (H), Formula (I), and/or Formula (J) as defined herein in reversing and/or ameliorating a neuromuscular blockade.

In one embodiment, the compound or the compound for use according to the present disclosure has been modified in order to increase its half-life when administered to a patient, in particular its plasma half-life.

In one embodiment, the compound or the compound for use according to the present disclosure further comprises a moiety conjugated to said compound, thus generating a moiety-conjugated compound. In one embodiment, said moiety-conjugated compound has a plasma and/or serum half-life being longer than the plasma and/or serum half-life of the non-moiety conjugated compound.

In one embodiment, the moiety conjugated to the compound or compound for use according to the present disclosure, is one or more type(s) of moieties selected from the group consisting of albumin, fatty acids, polyethylene glycol (PEG), acylation groups, antibodies and antibody fragments.

### Neuromuscular disorders

The compound or compound for use of the present disclosure is used for treating, ameliorating and/or preventing a neuromuscular disorder, or reversing neuromuscular blockade caused by non-depolarizing neuromuscular blocker or antibiotic agent.

The inventors of the present disclosure have shown that inhibition of CIC-1 channels strengthens neuromuscular transmission. CIC-1 function may therefore contribute to muscle weakness in conditions of compromised neuromuscular transmission.

Thus, in one embodiment of the present disclosure, the compound or the compound for use as described herein inhibits CIC-1 channels. Thus, it is appreciated that compounds and/or compounds for use of Formula (I) inhibit CIC-1 channels.

The neuromuscular disorder may also include neuromuscular dysfunctions.

Neuromuscular disorders include for example disorders with symptoms of muscle weakness and fatigue. Such disorders may include conditions with reduced neuromuscular transmission safety factor. In one embodiment the neuromuscular disorders are motor neuron disorders. Motor neuron disorders are disorders with reduced safety in the neuromuscular transmission. In one embodiment motor neuron disorders are selected from the group consisting of amyotrophic lateral sclerosis (ALS) (Killian JM, Wilfong AA, Burnett L, Appel SH, Boland D. Decremental motor responses to repetitive nerve stimulation in ALS. Muscle Nerve, 1994, 17, 747-754), spinal muscular atrophy (SMA) (Wadman RI, Vrancken AF, van den Berg LH, van der Pol WL. Dysfunction of the neuromuscular junction in spinal muscular atrophy types 2 and 3. Neurology, 2012, 79, 2050-2055), Charcot-Marie Tooth disease (Bansagi B, Griffin H, Whittaker RG, Antoniadi T, Evangelista T, Miller J, Greenslade M, Forester N, Duff J, Bradshaw A, Kleinle S, Boczonadi V, Steele H, Ramesh V, Franko E, Pyle A, Lochmüller H, Chinnery PF, Horvath R. Genetic heterogeneity of motor neuropathies. Neurology, 2017, 28;88(13):1226-1234), X-linked spinal and bulbar muscular atrophy (Yamada, M., Inaba, A., Shiojiri, T. X-linked spinal and bulbar muscular atrophy with myasthenic symptoms. Journal of the Neurological Sciences, 1997, 146, 183-185), Kennedy's disorder (Stevic, Z., Peric, S., Pavlovic, S., Basta, I., Lavrnic, D., Myasthenic symptoms in a patient with Kennedy's disorder. Acta Neurologica Belgica, 2014, 114, 71-73), multifocal motor neuropathy (Roberts, M., Willison, H.J., Vincent, A., Newsom-Davis, J. Multifocal motor neuropathy human sera block distal motor nerve conduction in mice. Ann Neurol. 1995, 38, 111-118), Guillain-Barre syndrome (Ansar, V., Valadi, N. Guillain-Barre Syndrome Prim. Care, 2015, 42, 189-193; poliomyelitis (Trojan, D.A., Gendron, D., Cashman, N.R. Electrophysiology and electrodiagnosis of the post-polio motor unit. Orthopedics, 1991, 14, 1353-1361, and Birk T.J. Poliomyelitis and the post-polio syndrome: exercise capacities and adaptation - current research, future directions, and widespread applicability. Med. Sci. Sports Exerc., 1993, 25, 466-472), post-polio syndrome (Garcia, C.C., Potian, J.G., Hognason, K., Thyagarajan, B., Sultatos, L.G., Souayah, N., Routh, V.H., McArdle, J.J. Acetylcholinesterase deficiency contributes to neuromuscular junction dysfunction in type 1 diabetic neuropathy. Am. J. Physiol. Endocrinol. Metab., 2012, 15, E551 - 561) and sarcopenia (Gilmore K.J., Morat T., Doherty T.J., Rice C.L., Motor unit number estimation and neuromuscular fidelity in 3 stages of sarcopenia. 2017 55(5):676-684).

Thus, in one embodiment of the present disclosure the neuromuscular disorder is amyotrophic lateral sclerosis (ALS). In another embodiment the neuromuscular disorder is spinal muscular atrophy (SMA). In another embodiment the neuromuscular disorder is Charcot-Marie tooth disease (CMT). In another embodiment the neuromuscular disorder is sarcopenia. In yet another embodiment, the neuromuscular disorder is critical illness myopathy (CIM).

As stated above the neuromuscular disorders include for example disorders with symptoms of muscle weakness and fatigue. Such disorder may for example include diabetes (Burton, A. Take your pyridostigmine: that's an (ethical?) order! Lancet Neurol., 2003, 2, 268).

In one embodiment, the compound or the compound for use of the present disclosure is used to prevent neuromuscular disorder. The compound or the compound for use may for example be used prophylactically against nerve gas that is known to cause symptoms of muscle weakness and fatigue (Kawamura, Y., Kihara, M., Nishimoto, K., Taki, M. Efficacy of a half dose of oral pyridostigmine in the treatment of chronic fatigue syndrome: three case reports. Pathophysiology, 2003, 9, 189-194.

In another embodiment the neuromuscular disorders is chronic fatigue syndrome. Chronic fatigue syndrome (CFS) (Fletcher, S.N., Kennedy, D.D., Ghosh, I.R., Misra, V.P., Kiff, K., Coakley, J.H., Hinds, C.J. Persistent neuromuscular and neurophysiologic abnormalities in long-term survivors of prolonged critical illness. Crit. Care Med. 2003, 31, 1012 - 1016) is the common name for a medical condition characterized by debilitating symptoms, including fatigue that lasts for a minimum of six months in adults. CFS may also be referred to as systemic exertion intolerance disorder (SEID), myalgic encephalomyelitis (ME), post-viral fatigue syndrome (PVFS), chronic fatigue immune dysfunction syndrome (CFIDS), or by several other terms. Symptoms of CFS include malaise after exertion; unrefreshing sleep, widespread muscle and joint pain, physical exhaustion, and muscle weakness.

In a further embodiment, the neuromuscular disorder is a critical illness polyneuropathy (Angelini C. Spectrum of metabolic myopathies. Biochim. Biophys. Acta., 2015, 1852, 615 - 621) or CIM (Latronico, N., Bolton, C.F. Critical illness polyneuropathy and myopathy: a major cause of muscle weakness and paralysis. Lancet Neurol. 2011, 10, 931-941). Critical illness polyneuropathy and CIM are overlapping syndromes of widespread muscle weakness and neurological dysfunction developing in critically ill patients.

The neuromuscular disorder may also include metabolic myopathy (Milone, M., Wong, L.J. Diagnosis of mitochondrial myopathies. Mol. Genet. Metab., 2013, 110, 35 - 41) and mitochondrial myopathy (Srivastava, A., Hunter, J.M. Reversal of neuromuscular block. Br. J. Anaesth. 2009, 103, 115 - 129). Metabolic myopathies result from defects in biochemical metabolism that primarily affects muscle. These may include glycogen storage disorders, lipid storage disorder and 3-phosphocreatine stores disorder. Mitochondrial myopathy is a type of myopathy associated with mitochondrial disorder. Symptoms of mitochondrial myopathies include muscular and neurological problems such as muscle weakness, exercise intolerance, hearing loss and trouble with balance and coordination.

In another embodiment the neuromuscular disorder is periodic paralysis, in particular hypokalemic periodic paralysis which is a disorder of skeletal muscle excitability that presents with recurrent episodes of weakness, often triggered by exercise, stress, or carbohydrate-rich meals (Wu, F., Mi, W., Cannon, S.C., Neurology, 2013, 80, 1110-1116 and Suetterlin, K. et at, Current Opinion Neurology, 2014, 27, 583-590) or hyperkalemic periodic paralysis which is an inherited autosomal dominant disorder that affects sodium channels in muscle cells and the ability to regulate potassium levels in the blood (Ammat, T. et at, Journal of General Physiology, 2015, 146, 509-525).

In a embodiment the neuromuscular disorder is a myasthenic condition. Myasthenic conditions are characterized by muscle weakness and neuromuscular transmission failure. Congenital myasthenia gravis (Finlayson, S., Beeson, D., Palace, J. Congenital myasthenic syndromes: an update. Pract. Neurol., 2013, 13, 80 - 91) is an inherited neuromuscular disorder caused by defects of several types at the neuromuscular junction.

Myasthenia gravis and Lambert-Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107) are examples of myasthenic conditions. Myasthenia gravis is either an autoimmune or congenital neuromuscular disorder that leads to fluctuating muscle weakness and fatigue. In the most common cases, muscle weakness is caused by circulating antibodies that block ACh receptors at the postsynaptic neuromuscular junction, inhibiting the excitatory effects of the neurotransmitter ACh on nicotinic ACh-receptors at neuromuscular junctions (Gilhus, N.E., Owe, J.F., Hoff, J.M., Romi, F., Skeie, G.O., Aarli, J.A. Myasthenia Gravis: A Review of Available Treatment Approaches, Autoimmune Diseases, 2011, Article ID 84739). Lambert-Eaton myasthenic syndrome (also known as LEMS, Lambert-Eaton syndrome, or Eaton-Lambert syndrome) is a rare autoimmune disorder that is characterized by muscle weakness of the limbs. It is the result of an autoimmune reaction in which antibodies are formed against presynaptic voltage-gated calcium channels, and likely other nerve terminal proteins, in the neuromuscular junction.

Thus, in one embodiment of the present disclosure the neuromuscular disorder is myasthenia gravis. In another embodiment the neuromuscular disorder is Lambert-Eaton syndrome.

Neuromuscular blockade is used in connection with surgery under general anaesthesia. Reversing agents are used for more rapid and safer recovery of muscle function after such blockade. Complications with excessive muscle weakness after blockade during surgery can result in delayed weaning from mechanical ventilation and respiratory complications after the surgery. Since such complications have pronounced effects on outcome of the surgery and future quality of life of patients, there is a need for improved reversing agents (Murphy GS, Brull SJ. Residual neuromuscular block: lessons unlearned. Part I: definitions, incidence, and adverse physiologic effects of residual neuromuscular block. Anesth Analg. 2010 111(1):120-8). Thus, in one embodiment, the neuromuscular disorder has been induced by a neuromuscular blocking agent. In one particular embodiment the neuromuscular disorder is muscle weakness caused by neuromuscular blockade after surgery. In another embodiment of the present disclosure the compound or the compound for use is used for reversing and/or ameliorating neuromuscular blockade after surgery. In one embodiment, the neuromuscular blockade is drug induced. In one embodiment the neuromuscular blockade is induced by an antibiotic. In one embodiment the neuromuscular blockade is induced by a non-depolarizing neuromuscular blocker.

### Pharmaceutical formulations

In one embodiment, a composition comprising the compound or the compound for use, according to the present disclosure, is provided. The composition according to the present disclosure may be used for treating, ameliorating and/or preventing a neuromuscular disorder, and/or for use in reversing and/or ameliorating a neuromuscular blockade. Thus, the compositions and compounds described herein can be pharmaceutically acceptable. In one embodiment the composition as described herein is in the form of a pharmaceutical formulation. In one embodiment, the composition as described herein further comprises a pharmaceutically acceptable carrier.

### Combination therapy

The composition of the present disclosure may comprise further active ingredients/agents or other components to increase the efficiency of the composition. Thus, in one embodiment the composition further comprises at least one further active agent. It is appreciated that the active agent is suitable for treating, preventing or ameliorating said neuromuscular disorder.

The active agent in an embodiment can be an acetylcholine esterase inhibitor. Said acetylcholine esterase inhibitor may for example be selected from the group consisting of delta-9-tetrahydrocannabinol, carbamates, physostigmine, neostigmine, pyridostigmine, ambenonium, demecarium, rivastigmine, phenanthrene derivatives, galantamine, piperidines, donepezil, tacrine, edrophonium, huperzine, ladostigil, ungeremine and lactucopicrin.

In one embodiment, the acetylcholine esterase inhibitor is selected from the group consisting of neostigmine, physostigmine and pyridostigmine. In one embodiment the acetylcholine esterase inhibitor is neostigmine or pyridostigmine.

The active agent may also be an immunosuppressive drug. Immunosuppressive drugs are drugs that suppress or reduce the strength of the body's immune system. They are also known as anti-rejection drugs. Immunosuppressive drugs include but are not limited to glucocorticoids, corticosteroids, cytostatics, antibodies and drugs acting on immunophilins. In one embodiment the active agent is prednisone.

The active agent may also be an agent that is used in anti-myotonic treatment. Such agents include for example blockers of voltage gated Na⁺ channels, and aminoglycosides.

The active agent may also be an agent for reversing a neuromuscular blockade after surgery. Such agents include for example neostigmine or sugammadex (Org 25969, tradename Bridion). The active agent may also be an agent for increasing the Ca²⁺ sensitivity of the contractile filaments in muscle. Such agents include tirasemtiv and CK-2127107 (Hwee, D.T., Kennedy, A.R., Hartman, J.J., Ryans, J., Durham, N., Malik, F.I., Jasper, J.R. The small-molecule fast skeletal troponin activator, CK-2127107, improves exercise tolerance in a rat model of heart failure. Journal of Pharmacology and Experimental Therapeutics, 2015, 353, 159 - 168).

The active agent may also be an agent for increasing ACh release by blocking voltage-gated K⁺ channels in the pre-synaptic terminal. Such agent includes 3,4-aminopyridine.

### Methods

In one aspect, the present disclosure relates to a method of treating, preventing and/or ameliorating a neuromuscular disorder, said method comprising administering a therapeutically effective amount of the compound or the compound for use as defined herein to a person in need thereof.

In one aspect, the present disclosure relates to a method of reversing and/or ameliorating a neuromuscular blockade, said method comprising administering a therapeutically effective amount of the compound or the compound for use as defined herein to a person in need thereof.

In one aspect, the present disclosure relates to a method for recovery of neuromuscular transmission, said method comprising administering a therapeutically effective amount of the compound or the compound for use as defined herein to a person in need thereof.

The person in need thereof may be a person having a neuromuscular disorder or a person at risk of developing a neuromuscular disorder or a person having symptoms of muscle weakness and/or fatigue. In another embodiment the person in need thereof is a person with reduced neuromuscular transmission safety with prolonged recovery after neuromuscular blockade. Types of neuromuscular disorders are defined herein above. In an embodiment the person has, amyotrophic lateral sclerosis, spinal muscular atrophy, myasthenia gravis or Lambert-Eaton syndrome.

A therapeutically effective amount is an amount that produces a therapeutic response or desired effect in the person taking it. Administration routes, formulations and dosages can be optimized by persons of skill in the art.

The method of treatment may be combined with other methods that are known to treat, prevent and/or ameliorate neuromuscular disorders. The treatment method may for example be combined with administration of any of the agents mentioned herein above. In one embodiment the treatment is combined with administration of acetylcholine esterase inhibitor such as for example neostigmine or pyridostigmine.

In one embodiment, disclosure relates to a method for recovery of force in muscles with neuromuscular dysfunction, said method comprising administering a compound or a composition as defined herein to a subject in need thereof. In one embodiment, said recovery of force is >5%, such as > 10%, such as > 15%, such as >20%, such as >25%, such as >30%, such as >35%.

Another aspect of the disclosure relates to use of a compound as defined herein, for the manufacture of a medicament for the treatment, prevention and/or amelioration of a neuromuscular disorder.

Another aspect relates to use of a compound as defined herein, for the manufacture of a medicament or a reversal agent for reversing and/or ameliorating a neuromuscular blockade after surgery.

### Method of manufacturing

In one aspect, the present disclosure relates to methods of manufacturing compounds or compounds for use according to formula (A).

One method for manufacturing the compounds or compounds for use according to the present disclosure comprises the steps of
a. reacting a compound having formula (GM.II) 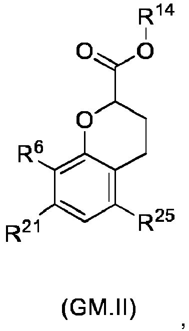 wherein R⁶, R²¹ and R²⁵ are defined herein and R¹⁴ is H with a halogenating agent, such as N-bromosuccinamide, to generate a compound having formula (GM.III) 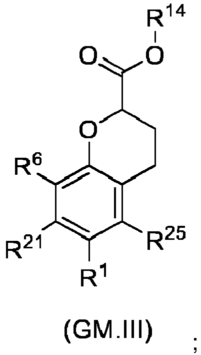 and
b. isolating the product compound of a) thus generating a compound of Formula (D) as defined herein.

An alternative method for manufacturing the compounds or compounds for use according to the present disclosure comprises the steps of
a. reacting a compound having formula (GM.V) 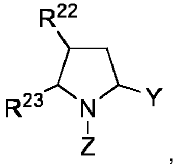 wherein R²¹ and R²² are defined herein, Y represents a carboxylic acid, or a feasible carboxylic acid precursor, which can include an ester, an alcohol or a nitrile function, and Z = H or suitable secondary amine protecting group, with a halobenzene derivative to generate a compound having formula (GM.VI) 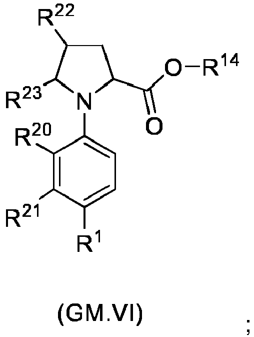 and
b. hydrolysing the product compound of a) thus generating a compound of Formula (I) as defined herein.

### Items

1. A compound of Formula A: 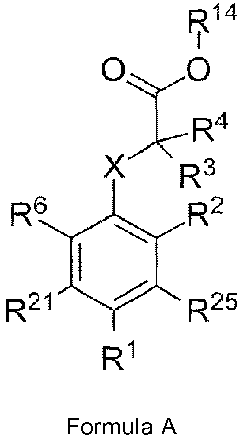 wherein
   - X: is O, NR⁵, or CHR⁵;
   - R¹: is Cl, Br, F, I or H;
   - R²: is H, a bond, -O-, C₁₋₃ alkyl or C₂₋₃ alkenyl, wherein said alkyl or alkenyl is optionally substituted with one or more F, =O, -OH, or phenyl optionally substituted with halogen;
   - R³: is a bond, H, C₁₋₃ alkyl or C₂₋₃ alkenyl, wherein said alkyl or alkenyl is optionally substituted with one or more F, =O, -OH, or phenyl optionally substituted with halogen;
   - R⁴: is a bond, H, C₁₋₃ alkyl or C₂₋₃ alkenyl;
   - R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
   - R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F;
   - R²¹: is H, F or Br; and
   - R²⁵: is H, F or Br;
   and wherein at least one ring is formed by linking together:
   a. R² and R³ to form a ring;
   b. R² and R⁵ to form a ring;
   c. R² and R³/R⁴ to form a ring, wherein R³ and R⁴ together form a double bond linked to R²; or
   d. R³ and R⁵ to form a ring;
   or a pharmaceutically acceptable salt, hydrate, polymorph, tautomer, or solvate thereof.
2. A compound of Formula A: 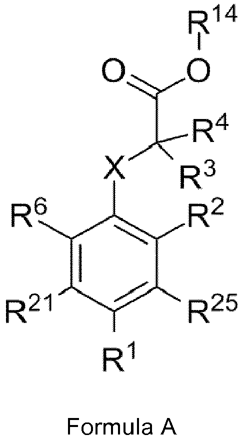 wherein
   - X: is O, NR⁵, or CHR⁵;
   - R¹: is Cl, Br F, I or H;
   - R²: is H, a bond, -O-, C₁₋₃ alkyl or C₂₋₃ alkenyl, wherein said alkyl or alkenyl is optionally substituted with one or more F, =O, -OH, or phenyl optionally substituted with halogen;
   - R³: is a bond, H, C₁₋₃ alkyl or C₂₋₃ alkenyl, wherein said alkyl or alkenyl is optionally substituted with one or more F, =O, -OH, or phenyl optionally substituted with halogen;
   - R⁴: is a bond, H, C₁₋₃ alkyl or C₂₋₃ alkenyl;
   - R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
   - R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F;
   - R²¹: is H, F or Br; and
   - R²⁵: is H, F or Br;
   and wherein at least one ring is formed by linking together:
   a. R² and R³ to form a ring;
   b. R² and R⁵ to form a ring;
   c. R² and R³/R⁴ to form a ring, wherein R³ and R⁴ together form a double bond linked to R²; or
   d. R³ and R⁵ to form a ring;
   with the proviso that when X is CHR⁵ then R² is -O-; and
   with the proviso that when R¹ is H, then X is NR⁵, R² is a bond, R⁵ is C₂ alkenyl optionally substituted with isoxazole, and R² and R⁵ are linked together to form a ring;
   or a pharmaceutically acceptable salt, hydrate, polymorph, tautomer, or solvate thereof.
3. The compound according to any one of the preceding items, wherein X is O or NR⁵; and R² is a bond, C₁₋₃ alkyl or C₂₋₃ alkenyl, wherein said alkyl or alkenyl is optionally substituted with one or more F, =O, -OH, or phenyl optionally substituted with halogen.
4. The compound according to any one of the preceding items, wherein R⁶ is isoxazol-3-yl or isoxazol-5-yl.
5. The compound according to any one of the preceding items, wherein the compound is of Formula B: 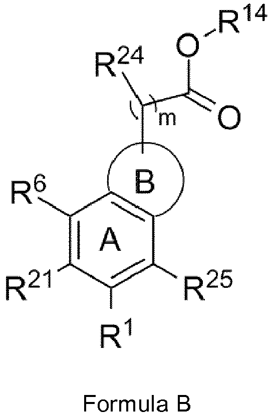 wherein
   - B: is a 5-, 6-, or 7-membered heterocycle, optionally substituted with one or more substituents individually selected from the group consisting of C₁₋₃ alkyl; a 5-membered aromatic heterocycle, such as isoxazole, optionally substituted with C₁₋₃ alkyl; =O; F; -OH; and phenyl optionally substituted with halogen;
   - m: is 0 or 1;
   - R¹: is Cl, Br F or I;
   - R⁶: is selected from the group consisting of H; Br; Cl; F; a 5-membered aromatic heterocycle optionally substituted with C₁₋₃ alkyl; C₂₋₅ alkyl optionally substituted with R¹⁵ or oxime; C₃₋₅ cycloalkyl optionally substituted with R¹⁵; and C₂₋₅ alkenyl optionally substituted with R¹⁵;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F; and
   - R¹⁶: is independently selected from the group consisting of deuterium, methoxy, nitro, cyano, Cl, Br, I and F;
   - R²⁴: is H or C₁₋₃ alkyl;
   - R²¹: is H or F; and
   - R²⁵: is H or F;
   or a pharmaceutically acceptable salt, hydrate, polymorph, tautomer, or solvate thereof.
6. The compound according to any one of the proceeding items, wherein rings A and B together constitutes a bicyclic structure selected from the group consisting of formulas AB1 to AB7: 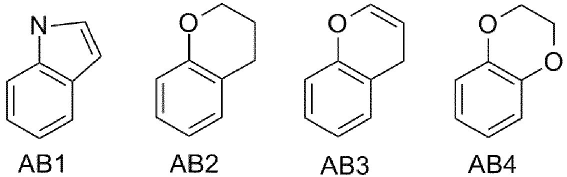 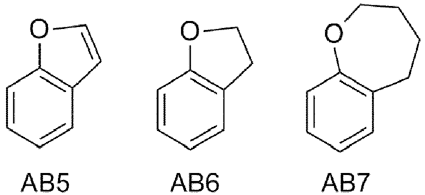
7. The compound according to any one of the preceding items, wherein the compound is of Formula C: 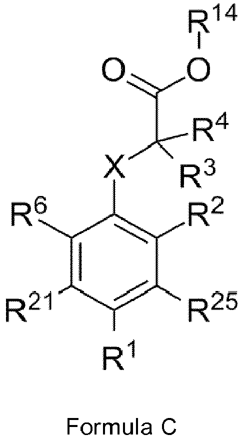 wherein
   - X: is O or N R⁵;
   - R¹: is Cl, Br, F or I;
   - R²: is H; a bond, C₁₋₃ alkyl optionally substituted with one or more F, C₂₋₃ alkenyl optionally substituted with one or more F, =O, or phenyl optionally substituted with halogen;
   - R³: is a bond, H, C₁₋₃ alkyl or C₂₋₃ alkenyl;
   - R⁴: is a bond, H, C₁₋₃ alkyl or C₂₋₃ alkenyl;
   - R⁵: is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
   - R⁶: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F;
   - R²¹: is H or F; and
   - R²⁵: is H or F;
   and wherein at least one ring is formed by linking together:
   a. R² and R³ to form a ring;
   b. R² and R⁵ to form a ring; or
   c. R² and R³/R⁴ to form a ring, wherein R³ and R⁴ together form a double bond linked to R²;
   or a pharmaceutically acceptable salt, hydrate, polymorph, tautomer, or solvate thereof.
8. The compound according to any one of the preceding items, wherein R⁶ is a 5-membered aromatic heterocycle, such as isoxazole, optionally substituted with C1-3 alkyl.
9. The compound according to any one of the preceding items, wherein X is O.
10. The compound according to any one of the preceding items, wherein the compound is of Formula D: 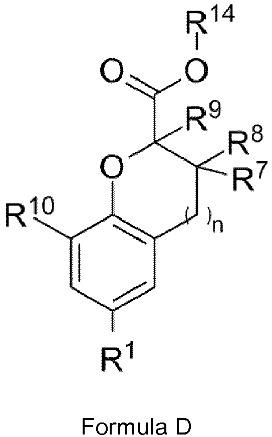 wherein
   - R¹: is Cl, Br, F or I;
   - R⁷: is H, F or C₁₋₅ alkyl optionally substituted with one or more, identical or different, substituents R¹⁵;
   - R⁸: is H or F;
   - R⁹: is H or methyl;
   - R¹⁰: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F; and
   - n: is 0, 1 or 2;
   or a pharmaceutically acceptable salt, hydrate, polymorph, tautomer, or solvate thereof.
11. The compound according to item 10, wherein the compound is of Formula D1 or Formula D2: 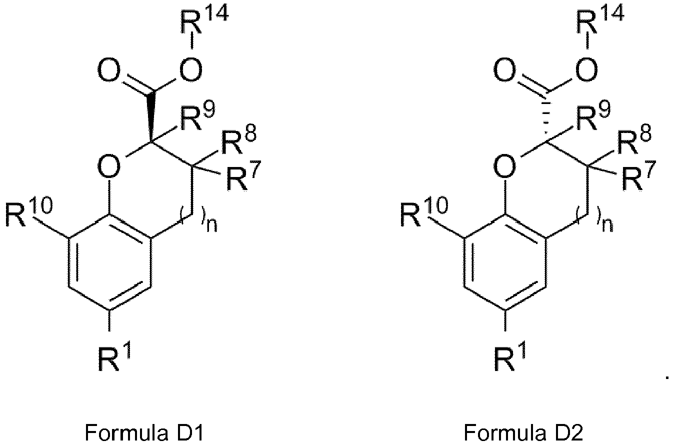
12. The compound according to any one of the proceeding items, wherein R¹⁰ is a 5-membered aromatic heterocycle, optionally substituted with C₁₋₃ alkyl.
13. The compound according to any one of the preceding items, wherein R¹⁰ is Br, isoxazole, such as isoxazol-3-yl, isoxazol-4-yl or isoxazol-5-yl, optionally substituted with C₁₋₃ alkyl; or -C(=NOH)CH₃.
14. The compound according to any one of the preceding items, wherein R¹⁰ is isoxazol-3-yl, optionally substituted with C₁₋₃ alkyl.
15. The compound according to any one of the preceding items, wherein R¹ is Cl or Br.
16. The compound according to any one of the preceding items, wherein n is 1.
17. The compound according to any one of the preceding items, wherein R⁷, R⁸, and R⁹ are each H.
18. The compound according to any one of the preceding items, wherein n is 0.
19. The compound according to any one of items 1 to 9, wherein the compound is of Formula E: 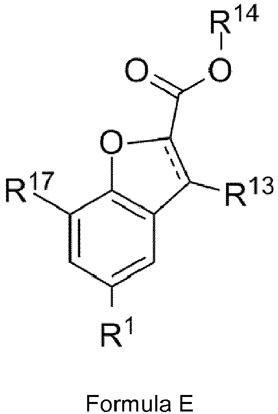 wherein
   -̅ -̅ -̅ represents a single or a double bond;
   - R¹: is Cl, Br, F or I;
   - R¹³: is H or phenyl optionally substituted with halogen;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F; and
   - R¹⁷: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
   or a pharmaceutically acceptable salt, hydrate, polymorph, tautomer, or solvate thereof.
20. The compound according to item 19, wherein R¹⁷ is H or a 5-membered aromatic heterocycle, optionally substituted with C₁₋₃ alkyl.
21. The compound according to any one of items 19 to 20, wherein R¹⁷ is isoxazole, such as isoxazol-3-yl, isoxazol-4-yl or isoxazol-5-yl, optionally substituted with C₁₋₃ alkyl.
22. The compound according to any one of items 1 to 9 or 19, wherein the compound is of Formula F: 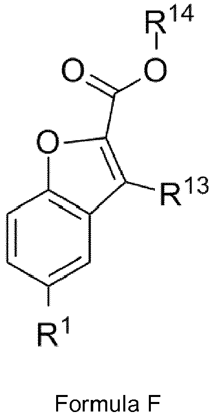 wherein
   - R¹: is Cl, Br, F or I;
   - R¹³: is phenyl optionally substituted with halogen;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F; and
   - R¹⁶: is independently selected from the group consisting of deuterium, methoxy, nitro, cyano, Cl, Br, I and F;
   or a pharmaceutically acceptable salt, hydrate, polymorph, tautomer, or solvate thereof.
23. The compound according to any one of items 19 to 22, wherein R¹³ is phenyl substituted with halogen, such as phenyl substituted with one Cl.
24. The compound according to any one of items 1 to 9, wherein the compound is of Formula G: 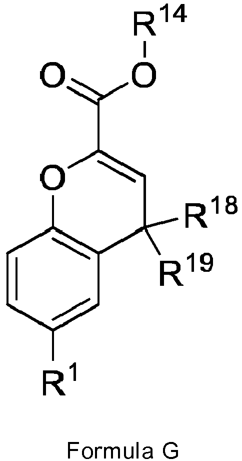 wherein
   - R¹: is Cl, Br, F or I;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F; and
   - R¹⁸ and R¹⁹: are F, or R¹⁸ is =O and R¹⁹ is absent;
   or a pharmaceutically acceptable salt, hydrate, polymorph, tautomer, or solvate thereof.
25. The compound according to any one of items 1 to 7, wherein X is NR⁵.
26. The compound according to any one of items 1 to 7 and 25, wherein the compound is of Formula H: 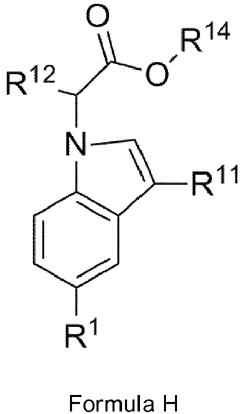 wherein
   - R¹: is Cl, Br, F or I;
   - R¹¹: is H or a 5-membered aromatic heterocycle, such as isoxazole, optionally substituted with C₁₋₃ alkyl;
   - R¹²: is H, C₁₋₅ alkyl, or C₃₋₆ cycloalkyl;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F; and
   - R¹⁶: is independently selected from the group consisting of deuterium, methoxy, nitro, cyano, Cl, Br, I and F;
   or a pharmaceutically acceptable salt, hydrate, polymorph, tautomer, or solvate thereof.
27. The compound according to item 26, wherein the compound is of Formula H1 or Formula H2: 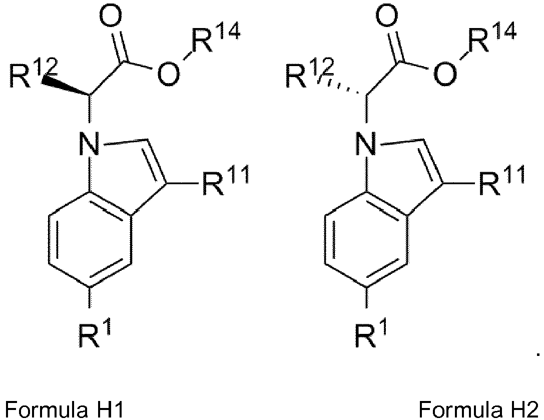
28. The compound according to any one of the preceding items, wherein R¹¹ is a 5-membered aromatic heterocycle.
29. The compound according to any one of items, with the proviso that R¹¹ is not a pyrazole.
30. The compound according to any one of the preceding items, wherein R¹¹ is H or isoxazole, optionally substituted with C₁₋₃ alkyl.
31. The compound according to any one of the preceding items, wherein R¹¹ is isoxazol-3-yl, isoxazol-4-yl, or isoxazol-5-yl.
32. The compound according to any one of the preceding items, wherein R¹² is H or methyl.
33. The compound according to any one the preceding items, wherein R¹¹ is not H when R¹² is H or methyl.
34. The compound according to any one of items 1 to 7 and 25 wherein the compound is of Formula I: 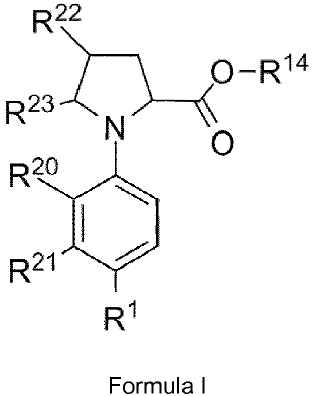 wherein
   - R¹: is Br, Cl, F or I;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F;
   - R²⁰: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
   - R²¹: is H or F;
   - R²²: is H or -OH;
   - R²³: is H or =O;
   or a pharmaceutically acceptable salt, hydrate, polymorph, tautomer, or solvate thereof.
35. The compound according to any one of items 1 to 7, 25 and 34, wherein the compound is of Formula J: 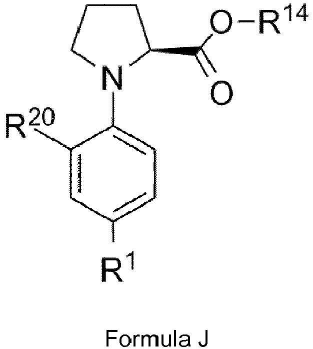 wherein
   - R¹: is Br, Cl, F or I;
   - R¹⁴: is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
   - R¹⁵: is independently selected from the group consisting of deuterium and F;
   - R¹⁶: is independently selected from the group consisting of deuterium, methoxy, nitro, cyano, Cl, Br, I and F; and
   - R²⁰: is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
   or a pharmaceutically acceptable salt, hydrate, polymorph, tautomer, or solvate thereof.
36. The compound according to any one of the preceding items, wherein R²⁰ is selected from the group consisting of H, Br, Cl, F, or a 5-membered aromatic heterocycle, optionally substituted with a C₁₋₃ alkyl.
37. The compound according to any one of the preceding items, wherein R²⁰ is isoxazole such as isoxazol-3-yl, isoxazol-4-yl or isoxazol-5-yl, optionally substituted with C₁₋₃ alkyl.
38. The compound according to any one of the preceding items, wherein R¹ is Br or Cl.
39. The compound according to any one of the preceding items, wherein R¹ is Br.
40. The compound according to any one of the preceding items, wherein R¹ is Cl.
41. The compound according to any one of the preceding items, wherein R¹⁴ is H.
42. The compound according to any one of the preceding items, wherein the 5-membered aromatic heterocycle is individually selected from the group consisting of isoxazole, oxazole, furan, thiophene, pyrrole, imidazole, pyrazole, thiazole, isothiazole, 1,2,3-triazole, 1,2,4-triazole, 1,2,5-oxadiazole, 1,2,3-oxadiazole, 1,2,4-oxadiazole, 1,3,4-oxadiazole, 1,2,5-thiadiazole, 1,2,3-thiadiazole, 1,2,4-thiadiazole, and 1,3,4-thiadiazole.
43. The compound according to any one of the preceding items, wherein the isoxazole is isoxazol-3-yl, isoxazol-4-yl or isoxazol-5-yl.
44. The compound according to any one of the preceding items, wherein said phenyl optionally substituted with halogen is a phenyl substituted with Cl.
45. The compound according to any one of the preceding items, wherein R² is H.
46. The compound according to any one of the preceding items, wherein R² is a bond.
47. The compound according to any one of the preceding items, wherein R² is -O-.
48. The compound according to any one of the preceding items, wherein R² is C₁₋₃ alkyl, wherein said alkyl is optionally substituted with one or more F, =O, -OH, or phenyl optionally substituted with halogen.
49. The compound according to any one of the preceding items, wherein R² is C₂₋₃ alkenyl, wherein said alkenyl is optionally substituted with one or more F, =O, - OH, or phenyl optionally substituted with halogen.
50. The compound according to any one of the preceding items, wherein R³ is H.
51. The compound according to any one of the preceding items, wherein R³ is a bond.
52. The compound according to any one of the preceding items, wherein R³ is -O-.
53. The compound according to any one of the preceding items, wherein R³ is C₁₋₃ alkyl, wherein said alkyl is optionally substituted with one or more F, =O, -OH, or phenyl optionally substituted with halogen.
54. The compound according to any one of the preceding items, wherein R³ is C₂₋₃ alkenyl, wherein said alkenyl is optionally substituted with one or more F, =O, - OH, or phenyl optionally substituted with halogen.
55. The compound according to any one of the preceding items, wherein R⁵ is a bond.
56. The compound according to any one of the preceding items, wherein R⁵ is C₁₋₃ alkyl optionally substituted with -OH.
57. The compound according to any one of the preceding items, wherein R⁵ is C₂₋₃ alkenyl optionally substituted with -OH or a 5-membered aromatic heterocycle, such as isoxazole, wherein said 5-membered aromatic heterocycle is optionally substituted with C₁₋₃ alkyl.
58. The compound according to any one of the preceding items, wherein R⁶ is H.
59. The compound according to any one of the preceding items, wherein R⁶ is Br, Cl, or F.
60. The compound according to any one of the preceding items, wherein R⁶ is a 5-membered aromatic heterocycle optionally substituted with a substituent selected from the group consisting of C₁₋₃ alkyl, C₂₋₅ alkyl optionally substituted with R¹⁵ or oxime, and C₃₋₅ cycloalkyl optionally substituted with R¹⁵.
61. The compound according to any one of the preceding items, wherein R⁶ is C₂₋₅ alkenyl optionally substituted with R¹⁵.
62. The compound according to any one of the preceding items, wherein R¹⁴ is H.
63. The compound according to any one of the preceding items, wherein R¹⁴ is C₁₋₅ alkyl optionally substituted with one or more, identical or different, substituents R¹⁵.
64. The compound according to any one of the preceding items, wherein R¹⁴ is C₂₋₅ alkenyl optionally substituted with one or more, identical or different, substituents R¹⁵.
65. The compound according to any one of the preceding items, wherein R¹⁴ is C₂₋₅ alkynyl optionally substituted with one or more, identical or different, substituents R¹⁵.
66. The compound according to any one of the preceding items, wherein R¹⁴ is C₃₋₆ cycloalkyl optionally substituted with one or more, identical or different, substituents R¹⁵.
67. The compound according to any one of the preceding items, wherein R¹⁴ is phenyl optionally substituted with one or more, identical or different, substituents R¹⁶.
68. The compound according to any one of the preceding items, wherein R¹⁴ is benzyl optionally substituted with one or more, identical or different, substituents R¹⁶.
69. The compound according to any one of the preceding items, wherein the compound is selected from the group consisting of: 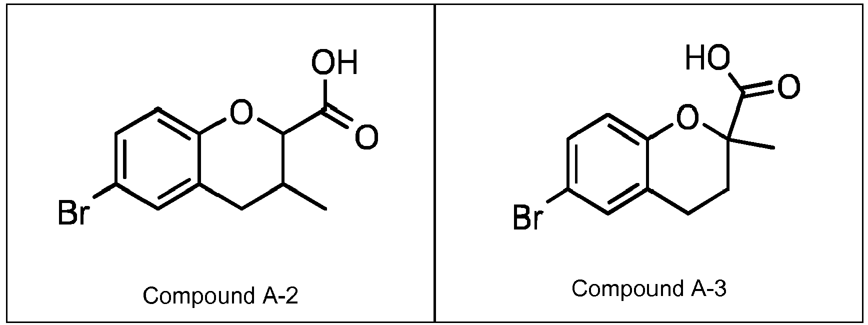 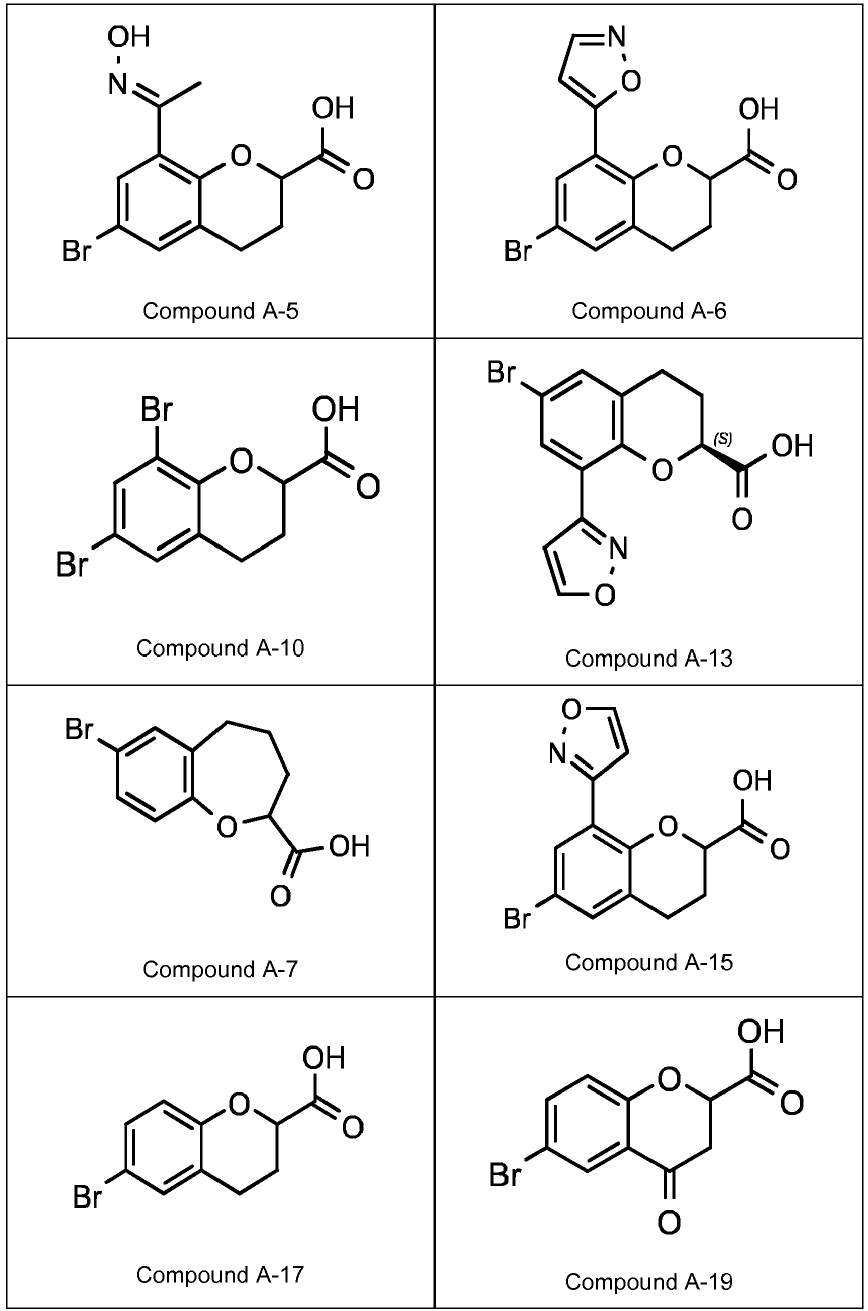 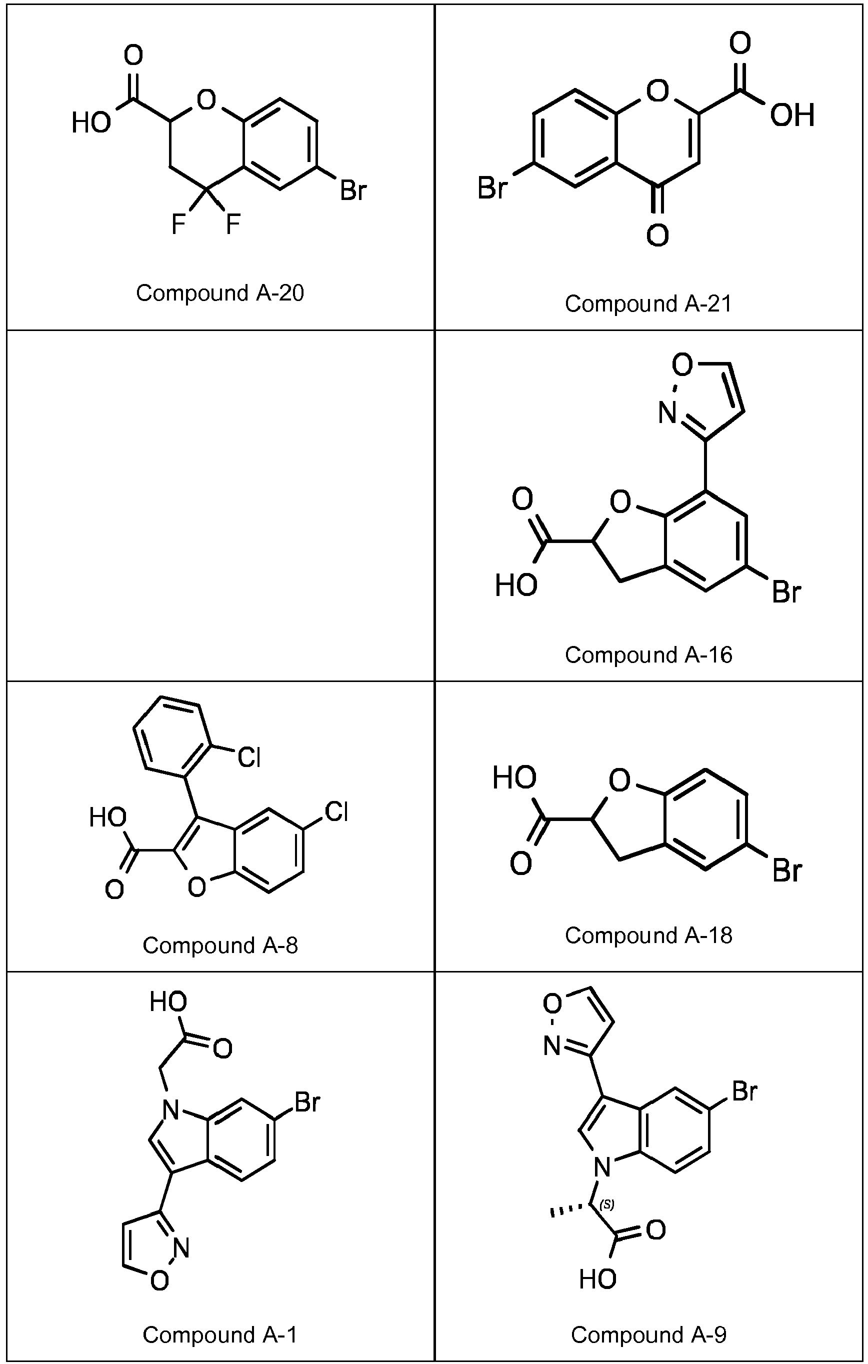 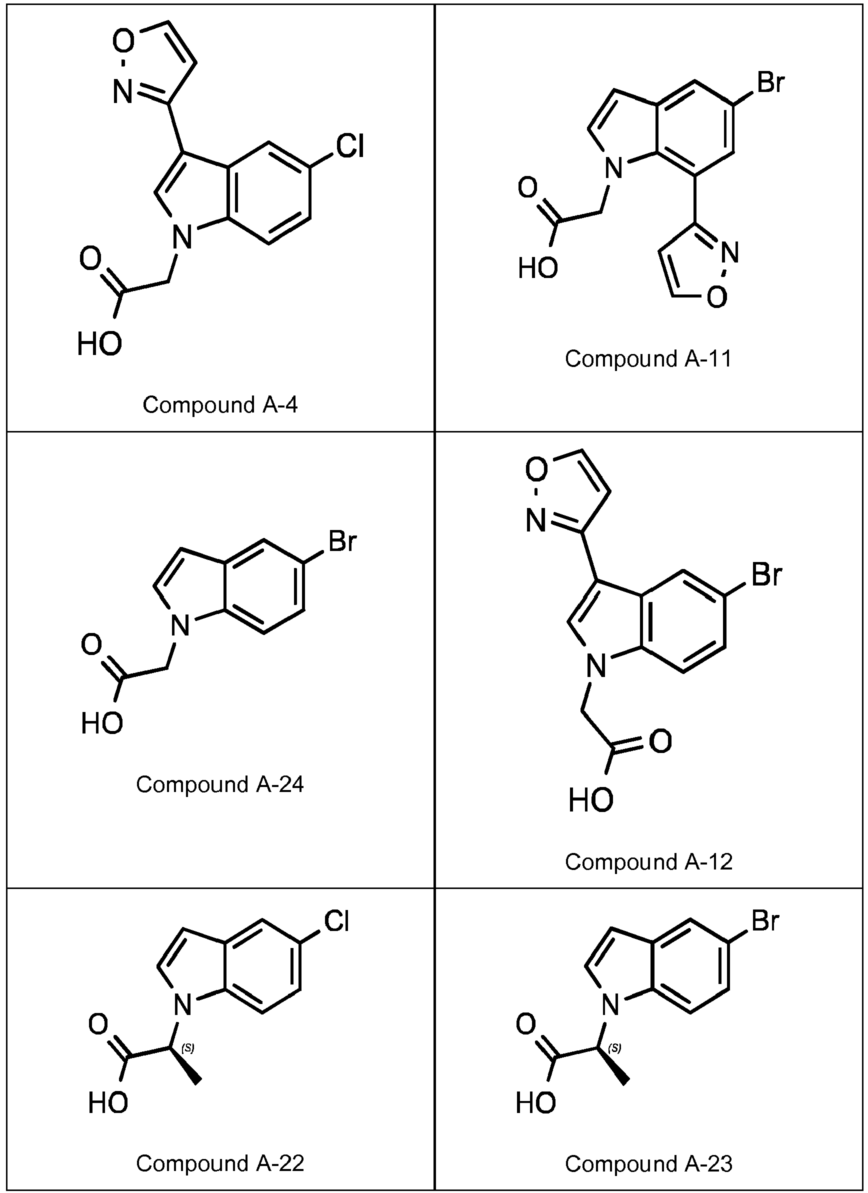 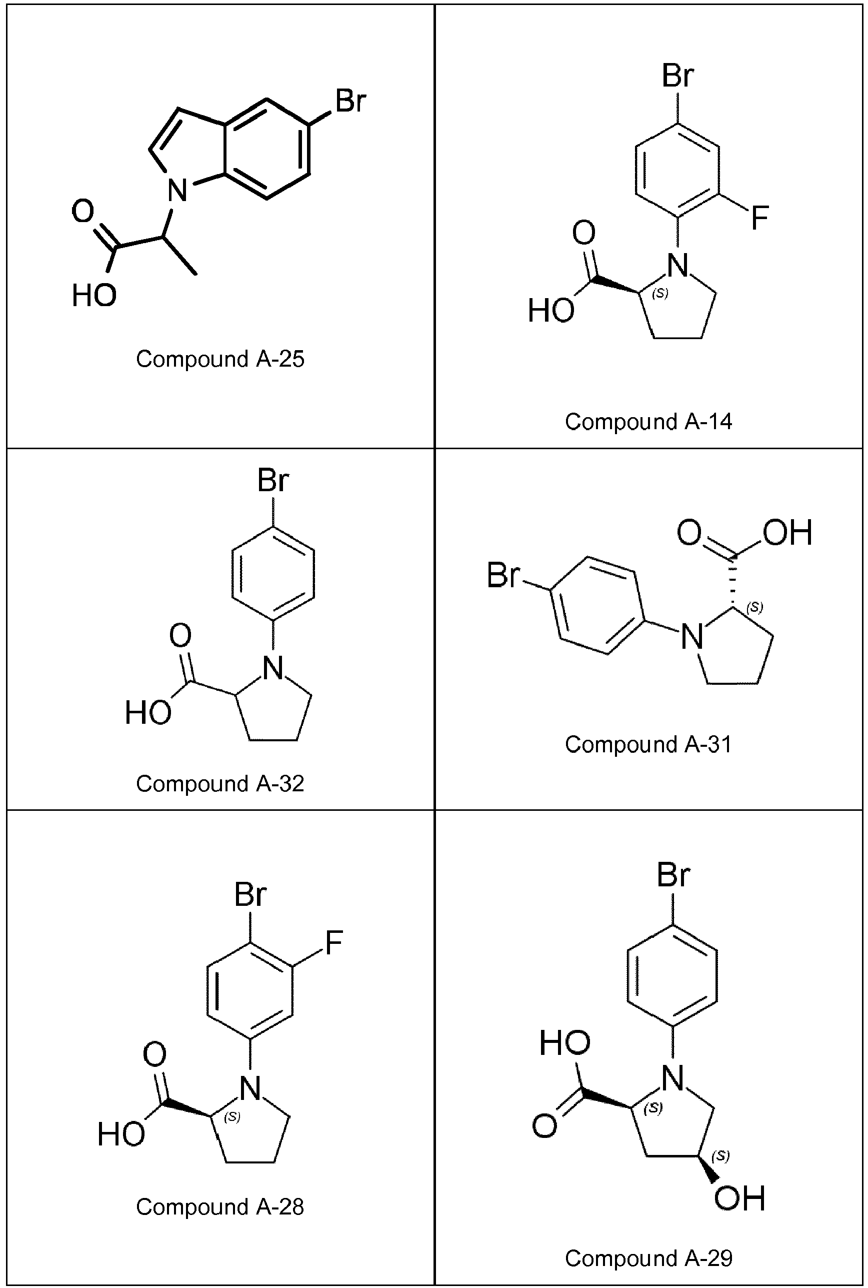 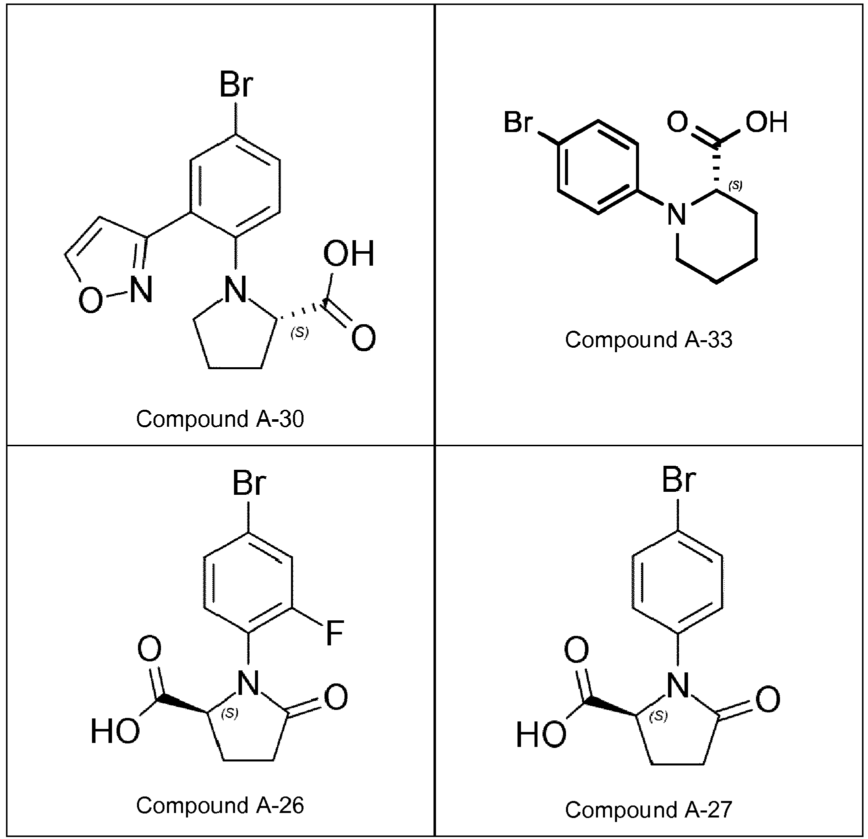
70. The compound according to any one of the preceding items, wherein the compound is selected from the group consisting of:
   6-bromo-3-methyl-3,4-dihydro-2H-1-benzopyran-2-carboxylic acid;
   6-bromo-2-methyl-3,4-dihydro-2H-1-benzopyran-2-carboxylic acid;
   6-bromo-8-(1,2-oxazol-5-yl)-3,4-dihydro-2H-1-benzopyran-2-carboxylic acid;
   6-bromo-8-[(1E)-1-(hydroxyimino)ethyl]-3,4-dihydro-2H-1-benzopyran-2-carboxylic acid;
   6,8-dibromo-3,4-dihydro-2H-1-benzopyran-2-carboxylic acid;
   (2S)-6-bromo-8-(1,2-oxazol-3-yl)-3,4-dihydro-2H-1-benzopyran-2-carboxylic acid;
   6-bromo-8-(1,2-oxazol-3-yl)-3,4-dihydro-2H-1-benzopyran-2-carboxylic acid;
   (2R)-6-bromo-8-(1,2-oxazol-3-yl)-3,4-dihydro-2H-1-benzopyran-2-carboxylic acid;
   6-bromo-3,4-dihydro-2H-1-benzopyran-2-carboxylic acid;
   6-bromo-4-oxo-3,4-dihydro-2H-1-benzopyran-2-carboxylic acid;
   6-bromo-4-oxo-4H-chromene-2-carboxylic acid;
   6-bromo-4,4-difluoro-3,4-dihydro-2H-1-benzopyran-2-carboxylic acid;
   7-bromo-2,3,4,5-tetrahydro-1-benzoxepine-2-carboxylic acid;
   5-bromo-7-(1,2-oxazol-3-yl)-2,3-dihydro-1-benzofuran-2-carboxylic acid;
   5-bromo-2,3-dihydro-1-benzofuran-2-carboxylic acid;
   5-chloro-3-(2-chlorophenyl)-1-benzofuran-2-carboxylic acid;
   2-[6-bromo-3-(1,2-oxazol-3-yl)-1H-indol-1-yl]acetic acid;
   (2S)-2-[5-bromo-3-(1 ,2-oxazol-3-yl)-1 H-indol-1-yl]propanoic acid;
   2-[5-bromo-7-(1,2-oxazol-3-yl)-1H-indol-1-yl]acetic acid;
   2-[5-chloro-3-(1,2-oxazol-3-yl)-1H-indol-1-yl]acetic acid;
   (2R)-2-(5-chloro-1H-indol-1-yl)propanoic acid;
   2-[5-bromo-3-(1,2-oxazol-3-yl)-1H-indol-1-yl]acetic acid;
   (2S)-2-(5-chloro-1 H-indol-1-yl)propanoic acid;
   (2S)-2-(5-bromo-1H-indol-1-yl)propanoic acid;
   2-(5-bromo-1H-indol-1-yl)acetic acid;
   (2R)-2-(5-bromo-1H-indol-1-yl)propanoic acid;
   2-(5-bromo-1 H-indol-1-yl)propanoic acid;
   (2S)-1-(4-bromo-2-fluorophenyl)pyrrolidine-2-carboxylic acid;
   1-(4-bromophenyl)pyrrolidine-2-carboxylic acid;
   (2S)-1-(4-bromophenyl)pyrrolidine-2-carboxylic acid;
   (2S)-1-(4-bromo-3-fluorophenyl)pyrrolidine-2-carboxylic acid;
   (2S,4S)-1-(4-bromophenyl)-4-hydroxypyrrolidine-2-carboxylic acid;
   (2R)-1-(4-bromophenyl)pyrrolidine-2-carboxylic acid;
   (2S)-1-[4-bromo-2-(1,2-oxazol-3-yl)phenyl]pyrrolidine-2-carboxylic acid;
   (2S)-1-(4-bromophenyl)-5-oxopyrrolidine-2-carboxylic acid; and
   (2S)-1-(4-bromo-2-fluorophenyl)-5-oxopyrrolidine-2-carboxylic acid.
71. The compound according to any one of the preceding items, wherein the compound is selected from the group consisting of:
   6-bromo-3-methyl-3,4-dihydro-2H-1-benzopyran-2-carboxylic acid;
   6-bromo-2-methyl-3,4-dihydro-2H-1-benzopyran-2-carboxylic acid;
   6-bromo-8-(1,2-oxazol-5-yl)-3,4-dihydro-2H-1-benzopyran-2-carboxylic acid;
   6-bromo-8-[(1E)-1-(hydroxyimino)ethyl]-3,4-dihydro-2H-1-benzopyran-2-carboxylic acid;
   6,8-dibromo-3,4-dihydro-2H-1-benzopyran-2-carboxylic acid;
   (2S)-6-bromo-8-(1,2-oxazol-3-yl)-3,4-dihydro-2H-1-benzopyran-2-carboxylic acid;
   6-bromo-8-(1,2-oxazol-3-yl)-3,4-dihydro-2H-1-benzopyran-2-carboxylic acid;
   (2R)-6-bromo-8-(1,2-oxazol-3-yl)-3,4-dihydro-2H-1-benzopyran-2-carboxylic acid;
   7-bromo-2,3,4,5-tetrahydro-1-benzoxepine-2-carboxylic acid;
   5-bromo-7-(1,2-oxazol-3-yl)-2,3-dihydro-1-benzofuran-2-carboxylic acid;
   2-[6-bromo-3-(1,2-oxazol-3-yl)-1H-indol-1-yl]acetic acid;
   (2S)-2-[5-bromo-3-(1,2-oxazol-3-yl)-1H-indol-1-yl]propanoic acid;
   2-[5-bromo-7-(1 ,2-oxazol-3-yl)-1 H-indol-1-yl]acetic acid;
   2-[5-chloro-3-(1 ,2-oxazol-3-yl)-1 H-indol-1-yl]acetic acid;
   (2R)-2-(5-chloro-1 H-indol-1-yl)propanoic acid;
   2-[5-bromo-3-(1 ,2-oxazol-3-yl)-1 H-indol-1-yl]acetic acid;
   (2S)-2-(5-chloro-1H-indol-1-yl)propanoic acid;
   (2R)-2-(5-bromo-1 H-indol-1-yl)propanoic acid;
   (2S)-1-(4-bromo-2-fluorophenyl)pyrrolidine-2-carboxylic acid;
   (2S)-1-(4-bromo-3-fluorophenyl)pyrrolidine-2-carboxylic acid;
   (2S,4S)-1-(4-bromophenyl)-4-hydroxypyrrolidine-2-carboxylic acid; and
   (2S)-1-[4-bromo-2-(1,2-oxazol-3-yl)phenyl]pyrrolidine-2-carboxylic acid.
72. The compound according to any one of the preceding items, wherein the compound is selected from the group consisting of:
   6-bromo-3-methyl-3,4-dihydro-2H-1-benzopyran-2-carboxylic acid;
   6-bromo-2-methyl-3,4-dihydro-2H-1-benzopyran-2-carboxylic acid;
   6-bromo-8-(1,2-oxazol-5-yl)-3,4-dihydro-2H-1-benzopyran-2-carboxylic acid;
   6-bromo-8-[(1E)-1-(hydroxyimino)ethyl]-3,4-dihydro-2H-1-benzopyran-2-carboxylic acid;
   6,8-dibromo-3,4-dihydro-2H-1-benzopyran-2-carboxylic acid;
   (2S)-6-bromo-8-(1,2-oxazol-3-yl)-3,4-dihydro-2H-1-benzopyran-2-carboxylic acid;
   6-bromo-8-(1,2-oxazol-3-yl)-3,4-dihydro-2H-1-benzopyran-2-carboxylic acid;
   (2R)-6-bromo-8-(1,2-oxazol-3-yl)-3,4-dihydro-2H-1-benzopyran-2-carboxylic acid;
   6-bromo-3,4-dihydro-2H-1-benzopyran-2-carboxylic acid;
   6-bromo-4-oxo-3,4-dihydro-2H-1-benzopyran-2-carboxylic acid; and
   6-bromo-4-oxo-4H-chromene-2-carboxylic acid.
73. The compound according to any one of the preceding items, wherein the compound is selected from the group consisting of:
   2-[6-bromo-3-(1 ,2-oxazol-3-yl)-1 H-indol-1-yl]acetic acid;
   (2S)-2-[5-bromo-3-(1,2-oxazol-3-yl)-1H-indol-1-yl]propanoic acid;
   2-[5-bromo-7-(1 ,2-oxazol-3-yl)-1 H-indol-1-yl]acetic acid;
   2-[5-chloro-3-(1 ,2-oxazol-3-yl)-1 H-indol-1-yl]acetic acid;
   (2R)-2-(5-chloro-1 H-indol-1-yl)propanoic acid;
   2-[5-bromo-3-(1 ,2-oxazol-3-yl)-1 H-indol-1-yl]acetic acid;
   (2S)-2-(5-chloro-1H-indol-1-yl)propanoic acid;
   (2S)-2-(5-bromo-1H-indol-1-yl)propanoic acid;
   2-(5-bromo-1H-indol-1-yl)acetic acid;
   (2R)-2-(5-bromo-1H-indol-1-yl)propanoic acid; and
   2-(5-bromo-1 H-indol-1-yl)propanoic acid.
74. The compound according to any one of the preceding items, wherein the compound is selected from the group consisting of:
   (2S)-1-(4-bromo-2-fluorophenyl)pyrrolidine-2-carboxylic acid;
   1-(4-bromophenyl)pyrrolidine-2-carboxylic acid;
   (2S)-1-(4-bromophenyl)pyrrolidine-2-carboxylic acid;
   (2S)-1-(4-bromo-3-fluorophenyl)pyrrolidine-2-carboxylic acid;
   (2S,4S)-1-(4-bromophenyl)-4-hydroxypyrrolidine-2-carboxylic acid;
   (2R)-1-(4-bromophenyl)pyrrolidine-2-carboxylic acid;
   (2S)-1-[4-bromo-2-(1,2-oxazol-3-yl)phenyl]pyrrolidine-2-carboxylic acid;
   (2S)-1-(4-bromophenyl)-5-oxopyrrolidine-2-carboxylic acid; and
   (2S)-1-(4-bromo-2-fluorophenyl)-5-oxopyrrolidine-2-carboxylic acid.
75. The compound according to any one of the preceding items, wherein the compound is selected from the group consisting of:
   6-bromo-8-(1,2-oxazol-5-yl)-3,4-dihydro-2H-1-benzopyran-2-carboxylic acid;
   (2S)-6-bromo-8-(1,2-oxazol-3-yl)-3,4-dihydro-2H-1-benzopyran-2-carboxylic acid;
   6-bromo-8-(1,2-oxazol-3-yl)-3,4-dihydro-2H-1-benzopyran-2-carboxylic acid;
   (2R)-6-bromo-8-(1,2-oxazol-3-yl)-3,4-dihydro-2H-1-benzopyran-2-carboxylic acid;
   5-bromo-7-(1,2-oxazol-3-yl)-2,3-dihydro-1-benzofuran-2-carboxylic acid;
   2-[5-bromo-7-(1,2-oxazol-3-yl)-1H-indol-1-yl]acetic acid; and
   (2S)-1-[4-bromo-2-(1,2-oxazol-3-yl)phenyl]pyrrolidine-2-carboxylic acid.
76. The compound according to any one of the preceding items, wherein the compound has activity on CIC-1 receptor.
77. The compound according to any one of the preceding items, wherein the compound is an inhibitor of the CIC-1 ion channel.
78. The compound according to item 77, wherein the EC₅₀ <50 µM, such as <40 µM, such as <30 µM, such as <20 µM, such as <15 µM, and such as <10 µM.
79. The compound according to any one of the preceding items, wherein the compound improves the recovered force in isolated rat soleus muscles after exposure to tubocurarine.
80. The compound according to any one of the preceding items, wherein the recovery of force in muscles with neuromuscular dysfunction is >5%, such as >10%, such as >15%, such as >20%, such as >25%, such as >30%, such as >35%.
81. A composition comprising the compound according to any one of the preceding items.
82. The composition according to any one of the preceding items, wherein the composition is a pharmaceutical composition.
83. The composition according to any one of the preceding items, wherein the composition further comprises a pharmaceutically acceptable carrier.
84. The composition according to any one of the preceding items, wherein the composition further comprises at least one further active agent.
85. The composition according to any one of the preceding items, wherein said further active agent is suitable for treating, preventing or ameliorating said neuromuscular disorder.
86. The composition according to any one of the preceding items, wherein said further active agent is an acetylcholine esterase inhibitor.
87. The composition according to any one of the preceding items, wherein said acetylcholine esterase inhibitor is selected from the group consisting of delta-9-tetrahydrocannabinol, carbamates, physostigmine, neostigmine, pyridostigmine, ambenonium, demecarium, rivastigmine, phenanthrene derivatives, galantamine, piperidines, donepezil, tacrine, edrophonium, huperzine, ladostigil, ungeremine and lactucopicrin.
88. The composition according to any one of the preceding items, wherein said acetylcholine esterase inhibitor is neostigmine or pyridostigmine.
89. The composition according to any one of the preceding items, wherein said further active agent is sugamadex.
90. The composition according to any one of the preceding items, wherein said further active agent is tirasemtiv or CK-2127107.
91. The composition according to any one of the preceding items, wherein said further active agent is 3,4-aminopyridine.
92. The compound or the composition according to any one of the preceding items, for use as a medicament.
93. The compound according to any one of the preceding items for use in treating, ameliorating and/or preventing a neuromuscular disorder, and/or for use in reversing and/or ameliorating a neuromuscular blockade.
94. The compound for use according to any one of the preceding items wherein the neuromuscular disorder is myasthenia gravis.
95. The compound for use according to any one of the preceding items wherein the neuromuscular disorder is autoimmune myasthenia gravis.
96. The compound for use according to any one of the preceding items wherein the neuromuscular disorder is congenital myasthenia gravis.
97. The compound for use according to any one of the preceding items wherein the neuromuscular disorder is Lambert-Eaton Syndrome.
98. The compound for use according to any one of the preceding items wherein the neuromuscular disorder is critical illness myopathy.
99. The compound for use according to any one of the preceding items wherein the neuromuscular disorder is amyotrophic lateral sclerosis (ALS).
100. The compound for use according to any one of the preceding items wherein the neuromuscular disorder is spinal muscular atrophy (SMA).
101. The compound for use according to any one of the preceding items wherein the neuromuscular disorder is critical illness myopathy (CIM).
102. The compound for use according to any one of the preceding items wherein the neuromuscular disorder is Charcot-Marie tooth disease (CMT).
103. The compound for use according to any one of the preceding items wherein the neuromuscular disorder is sarcopenia.
104. The compound for use according to any one of the preceding items wherein the neuromuscular disorder is reversal diabetic polyneuropathy.
105. The compound for use according to any one of the preceding items wherein the neuromuscular disorder is periodic paralysis.
106. The compound for use according to any one of the preceding items wherein the neuromuscular disorder is hypokalemic periodic paralysis or hyperkalemic periodic paralysis.
107. The compound for use according to any one of the preceding items wherein the neuromuscular disorder is selected from the group consisting of Guillain-Barre syndrome, poliomyelitis, post-polio syndrome, chronic fatigue syndrome, and critical illness polyneuropathy.
108. The compound for use according to any one of the preceding items, wherein the compound is for use in the treatment of symptoms of an indication selected from the group consisting of myasthenia gravis (such as autoimmune and congenital myasthenia gravis), Lambert-Eaton Syndrome, critical illness myopathy, amyotrophic lateral sclerosis (ALS), spinal muscular atrophy (SMA), critical illness myopathy (CIM), reversal diabetic polyneuropathy, Guillain-Barre syndrome, poliomyelitis, post-polio syndrome, chronic fatigue syndrome, critical illness polyneuropathy, periodic paralysis, sarcopenia, hypokalemic periodic paralysis and hyperkalemic periodic paralysis.
109. The compound for use according to any one of the preceding items wherein the neuromuscular disorder has been induced by a neuromuscular blocking agent.
110. The compound for use according to any one of the preceding items, wherein the neuromuscular blockade is neuromuscular blockade after surgery.
111. The compound for use according to any one of the preceding items, wherein the neuromuscular blockade is drug induced.
112. The compound for use according to any one of the preceding items, wherein the drug is an antibiotic.
113. The compound for use according to any one of the preceding items, wherein the drug is a non-depolarizing neuromuscular blocker.
114. The compound for use according to any one of the preceding items, wherein said compound further has been modified in order to increase its half-life when administered to a patient, in particular its plasma half-life.
115. The compound for use according to any one of the preceding items, wherein said compound further comprises a moiety conjugated to said compound, thus generating a moiety-conjugated compound.
116. The compound for use according to any one of the preceding items, wherein the moiety-conjugated compound has a plasma and/or serum half-life being longer than the plasma and/or serum half-life of the non-moiety conjugated compound.
117. The compound for use according to any one of the preceding items, wherein the moiety conjugated to the compound is one or more type(s) of moieties selected from the group consisting of albumin, fatty acids, polyethylene glycol (PEG), acylation groups, antibodies and antibody fragments.
118. A method of treating, preventing and/or ameliorating a neuromuscular disorder, said method comprising administering a therapeutically effective amount of the compound as defined in any one of the preceding items to a person in need thereof.
119. Use of a compound as defined in any one of the preceding items, for the manufacture of a medicament for the treatment, prevention and/or amelioration of a neuromuscular disorder, and/or for reversing and/or ameliorating of a neuromuscular blockade.
120. A method of reversing and/or ameliorating a neuromuscular blockade, said method comprising administering a therapeutically effective amount of the compound as defined in any one of the preceding items to a person in need thereof.
121. A method for recovery of neuromuscular transmission, said method comprising administering a therapeutically effective amount of the compound as defined in any one of the preceding items to a person in need thereof.
122. A method for recovering neuromuscular transmission, the method comprising administering a compound as defined in any one of the preceding items to an individual in need thereof.
123. A method for manufacturing the compound according to any one of the preceding items, the method comprising the steps of
   a. reacting a compound having formula (GM.II) 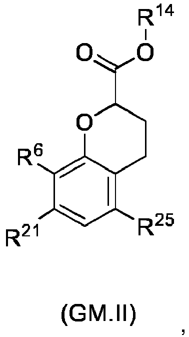 wherein R⁶, R²¹ and R2⁵ are defined herein and R¹⁴ is H with a halogenating agent, to generate a compound having formula (GM.III) 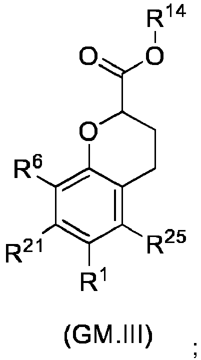 and
   b. isolating the product compound of a) thus generating a compound of Formula (D) as defined herein.
124. A method for manufacturing the compound according to any one of the preceding items, the method comprising the steps of
   a. reacting a compound having formula (GM.V) 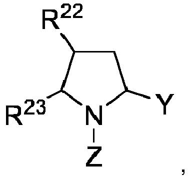 Wherein R²¹ and R²² are defined herein, Y represents a carboxylic acid and Z = H or suitable secondary amine protecting group, with a halobenzene derivative to generate a compound having formula (GM.VI) 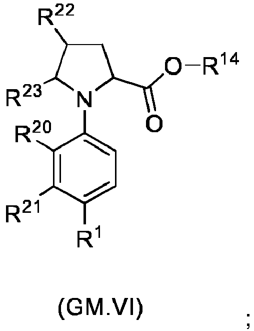 and
   b. hydrolysing the product compound of a) thus generating a compound of Formula (I) as defined herein.

### Examples

### Materials and methods

### Chemicals

Compounds for testing were obtained from different suppliers including Enamine, Vitas, and CanAm Bioresearch. For synthesis of particular compounds please see below.

### NMR Spectra

¹H-NMR spectra were recorded either on a Bruker AM-300 spectrometer and were calibrated using residual nondeuterated solvent as internal reference. Spectra were processed using Spinworks version 4.0 (developed by Dr. Kirk Marat, Department of Chemistry, University of Manitoba), or on a Bruker 400 MHZ Ultrashield plus equipped with probe BBO 400MHz S1 5mm with Z gradient probe or a Bruker 500 MHz Avance III HD spectrometer, equipped with a Bruker 5mm SmartProbeTM, calibrated using residual non-deuterated solvent as internal reference and spectra processed using topspin version 3.2.7.

### LCMS method 1

Waters Acquity UPLC, X-Select; column: Waters X-Select UPLC C18, 1.7 µm, 2.1 x 30mm. Solvent A: 0.1% formic acid in water; solvent B: 0.1% formic acid in MeCN. Gradient 5-95 % Solvent B over 3 minutes; detector: diode array.

### LCMS method 2

Waters Acquity UPLC, X-Select; column: Waters X-Select UPLC C18, 1.7 µm, 2.1 x 30mm. Solvent A: 0.1% formic acid in water; solvent B: 0.1% formic acid in MeCN. Gradient 5-95 % Solvent B over 10 minutes; detector: diode array.

### HPLC method

The product was analysed by Waters 2695 HPLC consisting of a Waters 996 photodiode array detector, Kromasil Eternity C18, 5 µm, 4.6 X 150 mm column. Flow rate: 1 mL/minute, run time 20 minutes. Solvent A: methanol; solvent B: 0.1% formic acid in water. Gradient 0-100 % Solvent B over 15 minutes with monitoring at 280 nm.

### Chiral SFC method 1

Compounds were analysed using a Waters ACQUITY ultra-performance convergence chromatography (UPC2) system equipped with a binary solvent delivery pump, an auto-sampler, a column oven (CM-30S), a back pressure regulator, and a diode array detector.

Column: Lux A1 (4.6mm x 250mm, 5µm).

Conditions: 40°C, 4 mL/min, isocratic 15:85 EtOH:CO₂ (0.1% v/v TFA), 125 BarG.

### General synthetic strategies

Compounds of formula (I) may be synthesized by the following general methods:

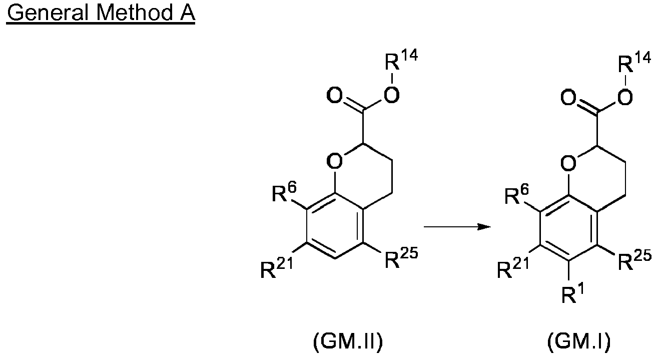

General Method A is illustrated for the convenient synthesis of a range of derivatives of chromane-2-carboxylic acid; however, it can be adapted for other examples wherein ring B in Formula B illustrated above is not a tetrahydropyran. These examples of ring B, i.e. involving 5- or 7-membered heterocycles, include the oxygen-containing heterocycles tetrahydrofuran, oxepane and related systems, optionally substituted with one or more substituents as defined in Formula B above.

General Method A involves the synthesis of compound GM.I, which is an acetic acid derivative, substituted by a fused bicyclic heterocyclic system as defined in Formula B, and -R¹, -R⁶, -R¹⁴, R²¹ and -R²⁵ are as defined in Formula B. Compound GM.II, wherein R¹⁴ = H, and -R⁶, R²¹ and -R²⁵ are as defined in Formula B, is available either commercially or synthetically. Compound GM.II can be converted into Compound GM.I wherein -R¹ is a halogen, R¹⁴ = H, and -R⁶, -R¹⁴, -R²¹ and -R²⁵ are as defined in Formula B, for example by utilising the reagent N-bromosuccinamide in a suitable solvent, such as dichloromethane or 1,2-dichloroethane.

A suitable -R⁶ substituent can be elaborated into a range of desired, alternative -R⁶ substituents in GM.I by a series of standard synthetic methods, some of which comprise more than one synthetic step. In the case of the desired -R⁶ substituent being a heterocycle, suitable methods of preparation include transition metal-catalysed coupling reactions which can proceed by two general methods. First where -R⁶ is a halogen such as bromine or iodine, a palladium-catalysed coupling reaction with a suitable substituted heterocycle containing a boronic acid or boronic ester functionality, will yield under appropriate conditions a compound of Formula GM.I, wherein -R¹, -R⁶,-R¹⁴, R²¹ and -R²⁵ are as defined in Formula B. In some cases, for such reactions to be successful, -R¹⁴ may need to be a suitable protecting group such as an ester functionality, which can be removed from the product under standard conditions to provide the desired target compound.

Alternatively, where -R⁶ is a boronic acid or boronic ester functionality, a palladium-catalyzed coupling reaction with a suitable halogen-substituted heterocycle will yield under appropriate conditions a compound of Formula GM.I, wherein -R¹, -R⁶, -R¹⁴, R²¹ and -R²⁵ are as defined in Formula B. Again, in some cases, for such reactions to be successful, -R¹⁴ may need to be a suitable protecting group such as an ester functionality, which can be removed under standard conditions to provide the desired target compound.

Alternative methods to obtain a compound of Formula GM.I, wherein -R¹, -R⁶, -R¹⁴, R²¹ and -R²⁵ are as defined in Formula B involve the reaction of e.g. a compound of Formula GM.II, wherein -R⁶ = H, with a suitable formylating reagent such as dichloro(methoxy)methane in a suitable solvent such as dichoromethane. The resultant benzaldehyde derivative can then be elaborated further into a range of heterocycles. In the case where -R⁶ in compound GM.I is an isozazole, then a multistep synthetic method such as described in Mogensen, J.P. et al., Bioorg. Med. Chem. Lett. 1998, 8, 1767, can conveniently be utilised.

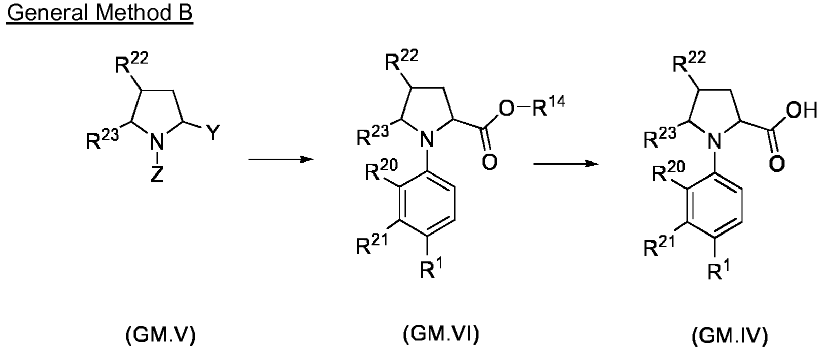

Method B involves the synthesis of compound GM.IV, an aryl-substituted proline derivative, wherein -R¹, -R²⁰, -R²¹, -R²² and -R²³ are as defined in Formula I above. Compound GM.V, wherein the groups -R²¹ and -R²² are as defined in Formula I above, where Z = H or suitable secondary amine protecting group, and Y represents a carboxylic acid, or a feasible carboxylic acid precursor, which can include an ester, an alcohol or a nitrile function, is available either commercially or synthetically. In the case of the acid precursor being a primary alcohol, it can be oxidised to a carboxylic acid under standard conditions involving potassium permanganate, Jones oxidation conditions, the Heyns oxidation, ruthenium tetroxide or TEMPO.

Compound GM.V thereby provides *via* a range of synthetic approaches, varied and facile access to a carboxylic acid GM.V wherein Y = -CO₂H, Z = -H and -R²² and -R²³ are as defined in Formula I above. Such a carboxylic acid derivative, can be chiral, which when the carboxylate function is protected with a suitable group, then reacted with a halobenzene derivative e.g. 1,4-dibromo-2-fluorobenzene in the presence of e.g. copper (I) bromide and an alkali metal carbonate in a high-boiling solvent such as DMF at temperatures in excess of 100°C, provides a proline derivative such as GM.VI, wherein -R¹, -R¹⁴, -R²⁰, -R²¹, -R²² and -R²³ are as defined in Formula I above. In the case of the specific compound illustrated by this method, -R²⁰ = -H or -F, -R¹ = Br, -R²² and -R²³ = H, and in cases where multiple compounds are obtained, these can typically be isolated by column chromatography.

In cases wherein -R¹⁴ in compound GM.VI is an ester or another suitable protecting group, saponification under basic or acidic conditions can be performed to provide the desired compound GM.IV.

### Exemplified Compounds

Table 1 below illustrates Example compounds defined by the general Formula (I) which were prepared in >95% purity.

**Table 1: Illustrative Examples of the Disclosure**

| **Cpd Number** | **IUPAC name** | **Spectroscopic data** | **Synthesis method** |
|---|---|---|---|
| A-1 | 2-[6-bromo-3-(1,2-oxazol-3-yl)-1H-indol-1-yl]acetic acid | 1H NMR (300 MHz, MeOD) δ 8.62 (s, 1H); 8.04 (d, 1H); 7.62 (s, 1H); 7.32 (d, 1H); 6.81 (s, 1H); 6.57 (s, 1H); 5.04 (s, 2H). | Example 5 |
| | | HPLC Retention Time: 10.12 min | |
| A-2 | 6-bromo-3-methyl-3,4-dihydro-2H-1-benzopyran-2-carboxylic acid | ¹H NMR (400 MHz, DMSO-d⁶) δ 7.15 (dd, *J* = 6.5, 2.6 Hz, 2H), 6.73 - 6.69 (m, 1H), 4.07 (d, *J* = 2.8 Hz, 1H), 2.85 (dd, *J* = 16.1, 5.5 Hz, 1H), 2.44 (dd, *J* = 16.1, 5.0 Hz, 1H), 2.36 (ddd, *J* = 7.0, 5.1, 2.7 Hz, 1H), 0.85 (d, *J* = 6.8 Hz, 3H). | General Method A Example 1 |
| | | LCMS method 2: m/z 269/271 (M-Na)-(ES⁻), at 3.821 min | |
| A-3 | 6-bromo-2-methyl-3,4-dihydro-2H-1-benzopyran-2-carboxylic acid | ¹H NMR (400 MHz, DMSO-d⁶) δ 7.10 (dd, *J* = 6.2, 2.6 Hz, 2H), 6.64 - 6.57 (m, 1H), 2.72 - 2.60 (m, 1H), 2.25 (ddd, *J* = 12.7, 5.8, 2.9 Hz, 1H), 1.46 (td, *J* = 12.4, 5.2 Hz, 1H), 1.36 (s, 3H) (1x CH obscured by DMSO peak). | General Method A Example 1 |
| | | LCMS method 2: m/z 269/271 (M-H)⁻ (ES⁻), at 3.855 min | |
| A-4 | 2-[5-chloro-3-(1,2-oxazol-3-yl)-1H-indol-1-yl]acetic acid | 1H NMR (300 MHz, MeOD) δ 8.62 (s, 1H), 8.13 (s, 1H), 7.85 (s, 1H), 7.31 (dd, 2H), 6.81 (s, 1H), 5.05 (s, 2H). | Example 5 |
| | | HPLC Retention Time: 9.91 min | |
| A-5 | 6-bromo-8-[(1E)-1-(hydroxyimino)ethyl]-3,4-dihydro-2H-1-benzopyran-2-carboxylic acid | ¹H NMR (400 MHz, DMSO-d⁶) δ 11.09 - 10.87 (m, 1H), 7.13 (d, *J* = 2.6 Hz, 1H), 7.05 (d, *J* = 2.6 Hz, 1H), 4.32 (q, *J* = 4.4 Hz, 1H), 2.70 - 2.55 (m, 2H), 2.13 (s, 3H), 2.09 (dd, *J* = 7.8, 5.3 Hz, 1H), 1.87 (ddt, *J* = 13.7, 10.1, 5.3 Hz, 1H) (1 x CH obscured by DMSO peak). | General Method A Example 2 |
| | | LCMS method 2: m/z 316/318 (M+H)⁺ (ES⁺); 314/316 (M-H)⁻ (ES⁻), at 3.250 min | |
| A-6 | 6-bromo-8-(1,2-oxazol-5-yl)-3,4-dihydro-2H-1-benzopyran-2-carboxylic acid | ¹H NMR (400 MHz, DMSO-d⁶) δ 8.64 (d, *J* = 1.8 Hz, 1H), 7.72(d, *J* = 2.5 Hz, 1H), 7.41 (d, *J* = 1.9 Hz, 1H), 7.33 (d, *J* = 2.5 Hz, 1H), 4.36 (dd, *J* = 7.7, 3.4 Hz, 1H), 2.75 (q, *J* = 6.2 Hz, 2H), 2.16 (ddd, *J* = 12.0, 9.4, 5.9 Hz, 1H), 1.92 (dtd, *J* = 13.5, 7.8, 5.8 Hz, 1H). | General Method A Example 2 |
| | | LCMS method 2: m/z 269/271 (M-H)⁻ (ES⁻), at 4.142 min | |
| A-7 | 7-bromo-2,3,4,5-tetrahydro-1-benzoxepine-2-carboxylic acid | ¹H NMR (400 MHz, DMSO-d⁶) δ 7.30 (m, 1H), 7.24 (m, 1H), 6.84 (m, 1H), 3.62 (m, 1H), 2.67 (m, 2H), 2.12 (m, 1H), 1.83 (m, 2H), 1.38 (m, 1H). | General Method A Example 1 |
| | | LCMS method 2: m/z 324/326 (M+H)⁺ (ES⁺); 322/324 (M-H)⁻ (ES⁻), at 3.812 min | |
| A-8 | 5-chloro-3-(2-chlorophenyl)-1-benzofuran-2-carboxylic acid | 1H NMR (300MHz, DMSO-d6) δ 13.65 (br s, 1H), 7.84 (d, 1H), 7.61 (t, 2H), 7.55-7.41 (m, 3H), 7.33 (s, 1H). | |
| | | HPLC Retention Time: 12.41 min | |
| A-9 | (2S)-2-[5-bromo-3-(1,2-oxazol-3-yl)-1H-indol-1-yl]propanoic acid | 1H NMR (300 MHz, MeOD) δ 8.63 (m, 1H), 8.31 (m, 1H), 8.03 (s, 1H), 7.45-7.32 (m, 2H), 5.35 (q, 1H), 1.89 (d, 3H). | Example 5 |
| | | HPLC Retention Time: 10.85 min LC/MS: 335.15 [M-1] | |
| A-10 | 6,8-dibromo-3,4-dihydro-2H-1-benzopyran-2-carboxylic acid | ¹H NMR (400 MHz, DMSO-d⁶) δ 7.47 (d, *J* = 2.4 Hz, 1H), 7.18 (d, *J* = 2.3 Hz, 1H), 4.48 (t, *J* = 4.2 Hz, 1H), 2.64 (dt, *J* = 10.1, 5.3 Hz, 2H), 2.18 - 2.08 (m, 1H), 1.93 - 1.80 (m, 1H). | General Method A Example 1 |
| | | LCMS method 2: m/z 333/335/337 (M-H)⁻ (ES⁻), at 4.781 min | |
| A-11 | 2-[5-bromo-7-(1,2-oxazol-3-yl)-1H-indol-1-yl]acetic acid | 1H NMR (300 MHz, MeOD) δ 8.77 (s, 1H); 7.85 (s, 1H); 7.25 (s, 1H); 6.66 (s, 1H); 6.57 (s, 1H); 4.93 (s, 2H). HPLC Retention Time: 10.28 min | Example 5 |
| A-12 | 2-[5-bromo-3-(1,2-oxazol-3-yl)-1H-indol-1-yl]acetic acid | 1H NMR (300 MHz, MeOD) δ 8.62 (s, 1H), 8.29 (s, 1H), 7.85 (s, 1H), 7.36 (m, 2H), 6.81 (s, 1H), 5.05 (s, 2H). | Example 6 |
| | | HPLC Retention Time: 12.51 min | |
| A-13 | (2S)-6-bromo-8-(1,2-oxazol-3-yl)-3,4-dihydro-2H-1-benzopyran-2-carboxylic acid | See A-16 Chiral SFC method: 4.64 mins | Example 3 |
| A-14 | (2S)-1-(4-bromo-2-fluorophenyl)pyrrolidine-2-carboxylic acid | ¹H NMR (400 MHz, DMSO-d⁶) δ 7.28 (dd, *J* = 13.8, 2.3 Hz, 1H), 7.17 (dd, *J* = 8.8, 2.4 Hz, 1H), 6.64 (t, *J* = 9.3 Hz, 1H), 4.45 - 4.34 (m, 1H), 3.47 - 3.33 (m, 2H), 2.30 - 2.20 (m, 1H), 2.02 - 1.83 (m, 3H). | General Method B Example 4 |
| | | LCMS method 2: m/z 286/288 (M-H)⁻ (ES⁻), at 4.187 in | |
| A-15 | 6-bromo-8-(1,2-oxazol-3-yl)-3,4-dihydro-2H-1-benzopyran-2-carboxylic acid | ¹H NMR (400 MHz, DMSO-d⁶) δ 13.33 (s, 1H), 9.02 (d, *J* = 1.7 Hz, 1H), 7.77 (d, *J* = 2.6 Hz, 1H), 7.45 (d, *J* = 2.6 Hz, 1H), 7.19 (d, *J* = 1.7 Hz, 1H), 4.97 (dd, *J* = 7.3, 3.8 Hz, 1H), 2.91 (dt, *J* = 17.0, 6.5 Hz, 1H), 2.74 (dt, *J* = 16.9, 6.3 Hz, 1H), 2.25 (dddd, *J* = 13.3, 7.2, 5.6, 3.7 Hz, 1H), 2.13 - 2.01 (m, 1H). | Example 2 |
| | | LCMS method 2: m/z 322/324 (M-H)⁻ (ES⁻), at 4.122 min | |
| A-16 | 5-bromo-7-(1,2-oxazol-3-yl)-2,3-dihydro-1-benzofuran-2-carboxylic acid | ¹H NMR (400 MHz, DMSO-d⁶) δ 9.02 (d, 1H), 7.69 (d, 1H), 7.43 (q, 1H), 7.09 (d, 1H), 4.89 (dd, 1H), 3.41 (dd, 1H), 3.20 (dd, 1H). | General Method A Example 2 |
| | | LCMS method 2: m/z 308/310 (M-H)⁻ (ES⁻), at 3.708 min | |
| A-17 | 6-bromo-3,4-dihydro-2H-1-benzopyran-2-carboxylic acid | ¹H NMR (400 MHz, DMSO-d⁶) δ 7.13 (d, *J* = 7.6 Hz, 2H), 6.68 - 6.62 (m, 1H), 4.19 (dd, *J* = 5.8, 3.9 Hz, 1H), 2.66 - 2.56 (m, 2H), 2.04 - 1.84 (m, 2H). | General Method A Example 1 |
| | | LCMS method 2: m/z 255/257 (M-H)⁻ (ES⁻), at 3.461 min | |
| A-18 | 5-bromo-2,3-dihydro-1-benzofuran-2-carboxylic acid | ¹H NMR (400 MHz, DMSO-d⁶) δ 7.28 (dt, 1H), 7.18 (dd, 1H), 6.67 (d, 1H), 4.77 (dd, 1H), 3.31 (m, 1H), 3.14 (dd, 1H) | General Method A Example 1 |
| | | LCMS method 2: m/z 241/243 (M-H)⁻ (ES⁻), at 2.976 min | |
| A-19 | 6-bromo-4-oxo-3,4-dihydro-2H-1-benzopyran-2-carboxylic acid | 1H NMR (300MHz, CD3OD) δ 7.88 (d, 1H), 7.66 (dd, 1H), 7.06 (d, 1H), 5.19-5.06 (br, 1H), 3.17-2.95 (m, 2H). | |
| | | HPLC method: 9.37 min | |
| A-20 | 6-bromo-4,4-difluoro-3,4-dihydro-2H-1-benzopyran-2-carboxylic acid | 1H NMR (300MHz, CDCl3) δ 7.71 (d, 1H), 7.48 (dd, 1H), 7.41 (Br, 1H), 6.91 (d, 1H), 4.95 (d, 1H), 2.93-2.56 (m, 2H). | |
| | | HPLC method: 10.97 min | |
| A-21 | 6-bromo-4-oxo-4H-chromene-2-carboxylic acid | 1H NMR (300MHz, DMSO-d6) δ 8.10 (d, 1H), 8.01 (dd, 1H), 7.73 (d, 1H), 3.90-2.90 (br s, 1H). | |
| | | HPLC retention time: 7.71 min LC/MS: 268.5 [M-1] | |
| A-22 | (2S)-2-(5-chloro-1H-indol-1-yl)propanoic acid | 1H NMR (300 MHz, CDCl3) δ 11.71-11.27 (br, 1H); 7.62 (s, 1H); 7.32-7.11 (m, 3H); 6.54 (d, 1H); 5.12 (q, 1H); 1.86 (d, 3H). | Example 7 |
| | | HPLC Retention Time: 13.52 min | |
| A-23 | (2S)-2-(5-bromo-1H-indol-1-yl)propanoic acid | 1H NMR (300 MHz, CDCl3) δ 9.78-9.13 (br, 1H); 7.75 (s, 1H); 7.30 (s, 1H); 7.20 (d, 1H); 7.13 (d, 1H); 6.51 (d, 1H); 5.06 (q, 1H); 1.82 (d, 3H). | Example 7 |
| | | HPLC Retention Time: 13.89 min | |
| A-24 | 2-(5-bromo-1H-indol-1-yl)acetic acid | 1H NMR (300 MHz, CDCl₃) δ 10.58-9.29 (br s, 1H), 7.77 (s, 1H), 7.77 (s, 1H), 7.33 (d, 1H), 7.11 (d, 1H), 7.07 (d, 1H), 6.52 (d, 1H), 4.87 (s, 2H). | Example 7 |
| | | HPLC Retention Time: 13.26 min | |
| A-25 | 2-(5-bromo-1H-indol-1-yl)propanoic acid | 1H NMR (300 MHz, CDCl3) δ 10.99-10.17 (br s, 1H), 7.77 (m, 1H), 6.53 (d, 1H), 5.13 (q, 1H), 1.87 (d, 3H). | Example 7 |
| | | HPLC Retention Time: 13.98 min | |
| A-26 | (2S)-1-(4-bromo-2-fluorophenyl)-5-oxopyrrolidine-2-carboxylic acid | ¹H NMR (400 MHz, DMSO-d⁶) δ 7.53 (dd, *J* = 10.3, 2.2 Hz, 1H), 7.44 (t, *J* = 8.3 Hz, 1H), 7.36 (dd, *J* = 9.1, 2.0 Hz, 1H), 4.13 (dd, *J* = 8.2, 3.9 Hz, 1H), 2.41 (tdd, *J* = 13.5, 6.9, 2.9 Hz, 1H), 2.32 - 2.19 (m, 2H), 1.98 (dddd, *J* = 13.0, 7.5, 5.4, 2.9 Hz, 1H). | General Method B Example 4 |
| | | LCMS method 2: m/z 302/304 (M+H)+ (ES+); 300/302 (M-H)- (ES-), at 2.195 min | |
| A-27 | (2S)-1-(4-bromophenyl)-5-oxopyrrolidine-2-carboxylic acid | ¹H NMR (400 MHz, DMSO-d⁶) δ 7.60 - 7.54 (m, 2H), 7.50 - 7.42 (m, 2H), 4.19 (dd, *J* = 8.8, 2.4 Hz, 1H), 2.60 - 2.52 (m, 1H), 2.31 (ddd, *J* = 16.3, 9.4, 2.8 Hz, 1H), 2.18 (dq, *J* = 12.2, 9.4 Hz, 1H), 1.94 (ddt, *J* = 12.1, 9.5, 2.6 Hz, 1H). | General Method B Example 4 |
| | | LCMS method 2: m/z 284/286 (M+H)⁺ (ES⁺); 282/284 (M-H)⁻ (ES⁻), at 2.288 min | |
| A-28 | (2S)-1-(4-bromo-3-fluorophenyl)pyrrolidin e-2-carboxylic acid | ¹H NMR (400 MHz, DMSO-d⁶) δ 7.24 (t, *J* = 8.7 Hz, 1H), 6.34 (dd, *J* = 12.9, 2.7 Hz, 1H), 6.23 (dd, *J* = 8.9, 2.7 Hz, 1H), 3.64 (dd, *J* = 7.4, 3.2 Hz, 1H), 3.27 (dd, *J* = 8.5, 4.5 Hz, 1H), 3.19 (q, *J* = 8.1, 7.4 Hz, 1H), 2.09 - 1.91 (m, 3H), 1.87 - 1.79 (m, 1H). | General Method B Example 4 |
| | | LCMS method 2: m/z 288/290 (M+H)+ (ES+); 286/288 (M-H)- (ES-), at 4.255 min | |
| A-29 | (2S,4S)-1-(4-bromophenyl)-4-hydroxypyrrolidine-2-carboxylic acid | ¹H NMR (400 MHz, DMSO-d⁶) δ 7.21 (d, *J* = 8.9 Hz, 2H), 6.55 (d, *J* = 8.9 Hz, 2H), 4.19 (d, *J* = 4.3 Hz, 1H), 3.64 (d, *J* = 9.2 Hz, 1H), 3.33 - 3.23 (m, 2H), 2.12 (ddd, *J* = 13.6, 9.4, 4.6 Hz, 1H), 1.86 (d, *J* = 13.0 Hz, 1H). Cannot distinguish methylene peaks at 3.33-3.23 from water peak. Alcohol OH not observed. | General Method B Example 4 |
| | | LCMS method 2: m/z 286/288 (M+H)+ (ES+); 284/286 (M-H)- (ES-), at 3.078 min | |
| A-30 | (2S)-1-[4-bromo-2-(1,2-oxazol-3-yl)phenyl]pyrrolidine-2-carboxylic acid | 1H NMR (300MHz, CDCl3) δ 10.94 (br s, 1H), 8.48 (s, 1H), 7.45 (d, 1H), 6.72 (s, 1 H), 4.34 (m, 1 H), 3.30 (m, 1 H), 2.82 (m, 1H), 2.47-2.31 (m, 1H), 1.9 -1.82 (m, 2H). | General Method B Example 4 |
| | | HPLC Retention time: 10.48 min | |
| A-31 | (2S)-1-(4-bromophenyl)pyrrolidine-2-carboxylic acid | 1H NMR (300 MHz, MeOD) δ 7.19 (d, 2H), 6.44 (d, 2H), 3.90 (m, 1H), 3.56 (m, 1H), 2.35-1.86 (m, 4H). | General Method B Example 4 |
| | | HPLC Retention Time: 14.51 min | |
| A-32 | 1-(4-bromophenyl)pyrrolidine-2-carboxylic acid | 1H NMR (300 MHz, MeOD) δ 7.26 (d, 2H), 6.46 (d, 2H), 4.18 (m, 1H), 3.50 (m, 1H), 2.42-1.99 (m, 4H). | General Method B Example 4 |
| | | HPLC Retention Time: 14.42 min | |
| A-33 | (2S)-1-(4-bromophenyl)piperidine-2-carboxylic acid | ¹H NMR (500 MHz, Chloroform-d) δ 7.38 - 7.28 (m, 2H), 6.82 - 6.70 (m, 2H), 4.47 (dd, *J* = 5.9, 3.1 Hz, 1H), 3.41 (dt, *J* = 12.2, 4.1 Hz, 1H), 3.25 (td, *J* = 11.9, 3.4 Hz, 1H), 2.28 - 2.16 (m, 1H), 1.99 - 1.86 (m, 1H), 1.86 - 1.76 (m, 1H), 1.76 - 1.66 (m, 1H), 1.66 - 1.55 (m, 1H), 1.53 - 1.34 (m, 1H). (OH not observed). | General Method B Example 4 |
| | | LCMS method 2: m/z 284.114, 286.115 (M+H)⁺ (ES⁺); at 3.163 min | |

### Example 1: 6-bromo-3,4-dihydro-2H-1-benzopyran-2-carboxylic acid

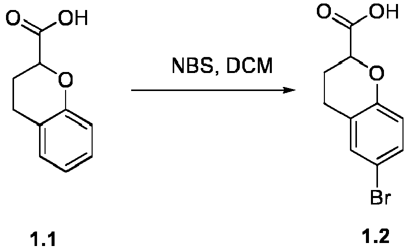

N-bromosuccinimide (NBS) (1.135 g, 6.38 mmol) was added to a stirred solution of chroman-2-carboxylic acid **1.1** (1.033 g, 5.80 mmol) in dichloromethane (10 ml, 155 mmol) at room temperature. After 1 hour, further NBS (1.135 g, 6.38 mmol) was added and the reaction was stirred for 1 hour further. Sodium metabisulfite solution (20 ml) was added and the mixture stirred for 30 minutes then insoluble material was filtered, washed with water (3 x 5 ml) and dried *in vacuo* at 50°C overnight. The filtrate was acidified with 1M hydrochloric acid to pH 4-5, phases were separated, organics dried (MgSO₄) and evaporated *in vacuo* to give 6-bromo-3,4-dihydro-2H-1-benzopyran-2-carboxylic acid **1.2** as a white solid (1.275 g, 4.56 mmol, 79 % yield).
LCMS method 2: m/z 255/257 (M-H)⁻ (ES⁻), at 3.461 min.
¹H NMR (400 MHz, DMSO-d⁶) δ 7.13 (d, *J* = 7.6 Hz, 2H), 6.68 - 6.62 (m, 1H), 4.19 (dd, *J* = 5.8, 3.9 Hz, 1H), 2.66 - 2.56 (m, 2H), 2.04 - 1.84 (m, 2H).

### Example 2: 6-bromo-8-(1,2-oxazol-3-yl)-3,4-dihydro-2H-1-benzopyran-2-carboxylic acid

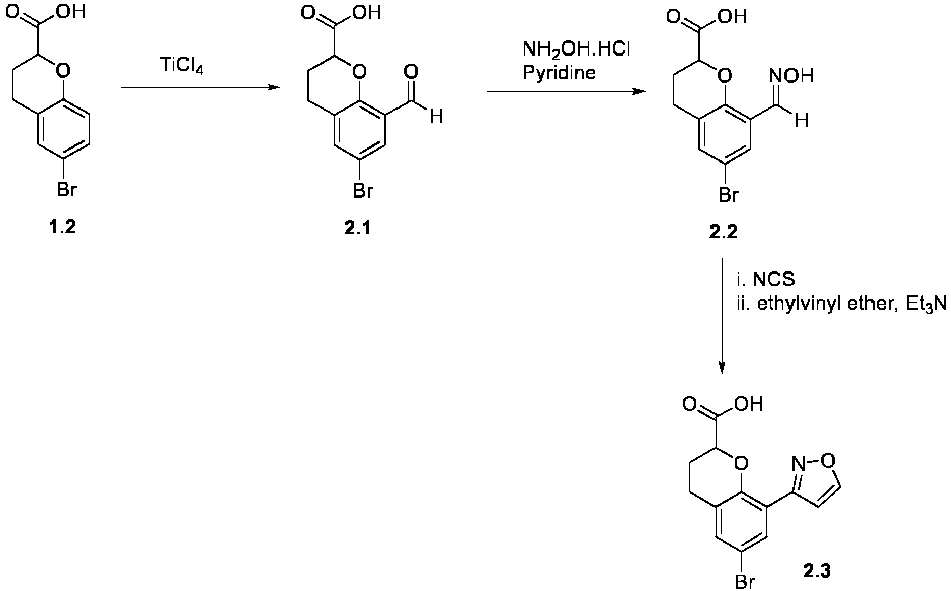

TiCl₄ (1M in dichloromethane) (16.37 mL, 16.37 mmol) was added dropwise over 5 minutes to a stirred solution of 6-bromo-3,4-dihydro-2H-1-benzopyran-2-carboxylic acid **1.2** (1.271 g, 4.55 mmol) and dichloro(methoxy)methane (1.358 mL, 15.01 mmol) in anhydrous DCM (16 mL, 249 mmol) at 0°C under nitrogen. After 2 hours water (32 ml) was added and the mixture stirred for 2 hours. The phases were separated and the aqueous phase extracted with dichloromethane (30 ml). Combined organic extracts were dried (MgSO₄) and evaporated *in vacuo* to give 6-bromo-8-formyl-3,4-dihydro-2H-1-benzopyran-2-carboxylic acid **2.1** as an oil (1.297g, 4.55 mmol, 100 % yield).

Hydroxylamine hydrochloride (0.632 g, 9.10 mmol) and pyridine (0.736 mL, 9.10 mmol) were added to a stirred suspension of 6-bromo-8-formyl-3,4-dihydro-2H-1-benzopyran-2-carboxylic acid **2.1** (1.297 g, 4.55 mmol) in ethanol (13 mL, 223 mmol) at room temperature and stirred for 2 h. The mixture was then heated at 60°C for 2 hours. The mixture was cooled to room temp. After 20 hours 1M sodium hydroxide (16 ml) was added. After 30 minutes the mixture was diluted with water (80 ml) and extracted with ethyl acetate (80 ml). The aqueous phase was acidified to pH 2-3 with conc. hydrochloric acid and extracted with ethyl acetate (80 ml). Organic extracts were dried (MgSO₄) and evaporated *in vacuo* to give 6-bromo-8-[(hydroxyimino)methyl]-3,4-dihydro-2H-1-benzopyran-2-carboxylic acid **2.2** as a solid (1.25 g, 3.35 mmol, 73.6 % yield).
LCMS method 1: m/z 300/302 (M+H)⁺ (ES⁺); 298/300 (M-H)⁻ (ES⁻).
¹H NMR (400 MHz, DMSO-d⁶) δ 12.43 (s, br, 1H), 11.43 (s, 1H), 8.23 (s, 1H), 7.57 (d, *J* = 2.5 Hz, 1H), 7.31 (d, *J* = 2.5 Hz, 1H), 4.92 (dd, *J* = 6.2, 4.1 Hz, 1H), 2.82 (dt, *J* = 16.6, 5.9 Hz, 1H), 2.70 - 2.60 (m, 1H), 2.18 (ddt, *J* = 13.8, 9.3, 5.2 Hz, 1H), 2.08 (dt, *J* = 13.8, 6.3 Hz, 1H).

Hydrochloric acid (0.167 mL, 0.167 mmol) followed by NCS (0.447 g, 3.35 mmol) were added to a stirred solution of 6-bromo-8-[(hydroxyimino)methyl]-3,4-dihydro-2H-1-benzopyran-2-carboxylic acid **2.2** (1.25 g, 3.35 mmol) in DMF (12.5 mL) at room temperature. After stirring for 15 min, ethyl vinyl ether (0.386 mL, 4.02 mmol) followed by triethylamine (0.560 mL, 4.02 mmol) were added and the mixture stirred for a further 16 hours. Further ethyl vinyl ether (0.386 mL, 4.02 mmol) and triethylamine (0.560 mL, 4.02 mmol) were added and the reaction was stirred for 30 min. The mixture was diluted with water (75 ml), acidified with 1M hydrochloric acid to pH 4-5 and extracted with ethyl acetate (50 ml). Organics were dried (MgSO₄) and evaporated *in vacuo.* The residue was dissolved in formic acid (3.85 mL, 100 mmol) and heated at 90 °C for 30 minutes. The mixture was evaporated *in vacuo.* The residual oil co-evaporated with toluene (5 ml) then purified by column chromatography (40g FlashPure silica cartridge, gradient 20-100% of 99/1 ethyl acetate/acetic acid in pentane) to afford a solid. The solid was dissolved in ethyl acetate (20 ml) at reflux, isohexane (10 ml) added and the solution allowed to stand at room temp. for 3 days. Solvent was decanted from the resulting solid which was washed with isohexane (2 x 5 ml) and dried *in vacuo* at 50 °C for 5 hours to afford 6-bromo-8-(1,2-oxazol-3-yl)-3,4-dihydro-2H-1-benzopyran-2-carboxylic acid **2.3** (167 mg, 0.489 mmol, 14.62 % yield)
¹H NMR (400 MHz, DMSO-d⁶) δ 13.33 (s, 1H), 9.02 (d, *J* = 1.7 Hz, 1H), 7.77 (d, *J* = 2.6 Hz, 1H), 7.45 (d, *J* = 2.6 Hz, 1H), 7.19 (d, *J* = 1.7 Hz, 1H), 4.97 (dd, *J* = 7.3, 3.8 Hz, 1H), 2.91 (dt, *J* = 17.0, 6.5 Hz, 1H), 2.74 (dt, *J* = 16.9, 6.3 Hz, 1H), 2.25 (dddd, *J* = 13.3, 7.2, 5.6, 3.7 Hz, 1H), 2.13 - 2.01 (m, 1H).
LCMS method 2: m/z 322/324 (M-H)- (ES-), at 4.122 min

### Example 3: Chiral separation of 6-bromo-8-(1,2-oxazol-3-yl)-3,4-dihydro-2H-1-benzopyran-2-carboxylic acid

6-Bromo-8-(1,2-oxazol-3-yl)-3,4-dihydro-2H-1-benzopyran-2-carboxylic acid (144 mg) was dissolved to 20 mg/mL in methanol/dichloromethane (4:1 ratio) and was then purified by Supercritical Fluid Chromatography (SFC).

Column: Lux A1 (21.2mm x 250mm, 5µm). 125 BarG.

Conditions: 40°C, 50 mL/min; mobile phase A: CO₂; mobile phase B: EtOH (0.1% v/v TFA). Gradient 20-28% mobile phase B over 4 minutes; then 50% B over 0.1 min; then 50% B for 1 min; then equilibrate at 20% B for 1 min. Sequential injections of 500 µL (10 mg).

Combined fractions of the first eluting enantiomer were diluted 10-fold in water and freeze dried to give the (*R*)-enantiomer as a white solid (16 mg) in 96% e.e. Chiral SFC method: 3.79 mins
Combined fractions of the second eluting enantiomer were diluted 10-fold in water and freeze dried to give the (*S*)-enantiomer as a white solid (21 mg) in 96% e.e. Chiral SFC method: 4.64 mins

### Example 4: (2S)-1-(4-bromo-2-fluorophenyl)pyrrolidine-2-carboxylic acid and (2S)-1-(4-bromo-3-fluorophenyl)pyrrolidine-2-carboxylic acid

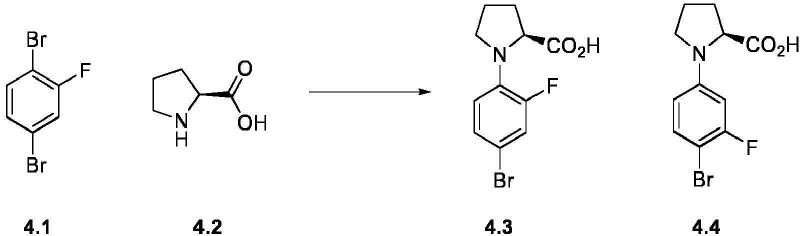

A suspension of 1,4-dibromo-2-fluorobenzene **4.1** (1 g, 3.94 mmol), (S)-pyrrolidine-2-carboxylic acid **4.2** (0.499 g, 4.33 mmol), copper(I) bromide (0.085 g, 0.591 mmol) and potassium carbonate (1.633 g, 11.82 mmol) in anhydrous DMF (10 ml) was stirred at 140 °C for 2 hours. The mixture was cooled to room temp., diluted with water (100 ml) and extracted with ethyl acetate (50 ml). The aqueous phase was acidified with 1M hydrochloric acid to pH 5-6 and extracted with ethyl acetate (100 ml). Organics were dried (MgSO₄) and evaporated *in vacuo.* The residue was subjected to column chromatography (40g FlashPure silica cartridge, gradient 10-100% ethyl acetate/acetic acid (99/1) in isohexane). The residue was triturated with isohexane/ethyl acetate (9/1, 10 ml) and liquors decanted off the resulting solid that was dried *in vacuo* to give (S)-1-(4-bromo-3-fluorophenyl)pyrrolidine-2-carboxylic acid (43mg). Liquors were evaporated *in vacuo* to an impure residue (223mg). All material was re-combined and purified by preparative HPLC to give (S)-1-(4-bromo-2-fluorophenyl)pyrrolidine-2-carboxylic acid **4.3** (32mg, 0.111 mmol, 2.82 % yield) and (S)-1-(4-bromo-3-fluorophenyl)pyrrolidine-2-carboxylic acid **4.4** (175mg, 0.607 mmol, 15.42 % yield) as brown gums. (S)-1-(4-bromo-2-fluorophenyl)pyrrolidine-2-carboxylic acid **4.3:**
¹H NMR (400 MHz, DMSO-d⁶) δ 7.28 (dd, *J* = 13.8, 2.3 Hz, 1H), 7.17 (dd, *J* = 8.8, 2.4 Hz, 1H), 6.64 (t, *J* = 9.3 Hz, 1H), 4.45 - 4.34 (m, 1H), 3.47 - 3.33 (m, 2H), 2.30 - 2.20 (m, 1H), 2.02 - 1.83 (m, 3H).
(S)-1-(4-bromo-3-fluorophenyl)pyrrolidine-2-carboxylic acid **4.4:**
¹H NMR (400 MHz, DMSO-d⁶) δ 12.85 (s, 1H), 7.39 (t, *J* = 8.6 Hz, 1H), 6.44 (dd, *J* = 12.4, 2.8 Hz, 1H), 6.27 (dd, *J* = 8.9, 2.8 Hz, 1H), 4.20 (dd, *J* = 8.8, 2.4 Hz, 1H), 3.39 (ddd, *J* = 9.1, 7.1, 4.5 Hz, 1H), 3.32 - 3.24 (m, 1H), 2.30 - 2.19 (m, 1H), 2.12 - 2.03 (m, 1H), 1.98 (ddt, *J* = 15.4, 7.9, 4.2 Hz, 2H).

1M sodium hydroxide volumetric solution (104 µl, 0.104 mmol) was added to a suspension of (S)-1-(4-bromo-2-fluorophenyl)pyrrolidine-2-carboxylic acid **4.3** (30 mg, 0.104 mmol) in water (1 ml) and the mixture ultrasonicated to give a cloudy solution. The solution was passed through a syringe filter then evaporated *in vacuo* (50 psi, 45°C) then the residue dried *in vacuo* at 50 °C overnight to give sodium (S)-1-(4-bromo-2-fluorophenyl)pyrrolidine-2-carboxylate (28 mg, 0.092 mmol, 89 % yield). LCMS method 2: m/z 288/290 (M+H)⁺ (ES⁺); 286/288 (M-H)⁻ (ES⁻), at 4.187 min.
¹H NMR (400 MHz, DMSO-d⁶) δ 7.12 (dd, *J* = 13.8, 2.4 Hz, 1H), 7.02 (dd, *J* = 8.8, 2.4 Hz, 1H), 6.56 - 6.48 (m, 1H), 3.85 (dd, *J* = 7.4, 3.5 Hz, 1H), 3.50 (t, *J* = 8.5 Hz, 1H), 3.38 (tt, *J* = 9.5, 4.7 Hz, 1H), 2.04 - 1.83 (m, 3H), 1.80 - 1.70 (m, 1H).

### Example 5: 2-[5-chloro-3-(1,2-oxazol-3-yl)-1H-indol-1-yl]acetic acid

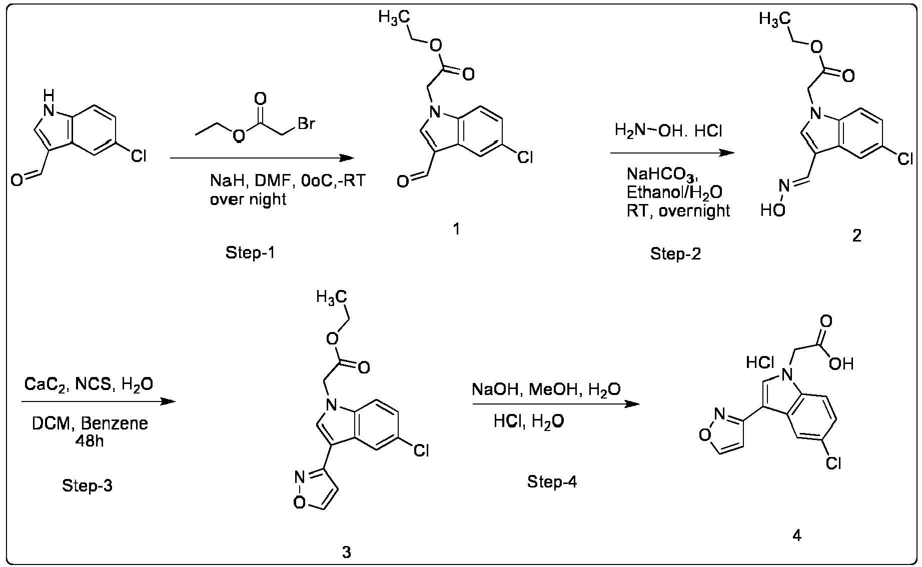

### Step 1: Ethyl 2-(5-chloro-3-formyl-1H-indol-1-yl)acetate

To a solution of 5-chloro-1H-indole-3-carbaldehyde (1.0g, 5.56 mmol, 1.0 equiv.) and ethyl 2-bromoacetate (1.02g, 6.12 mmol, 1.1 equiv.) in DMF (10 mL) at 0°C was added NaH (0.245g, 6.12 mmol. 1.1 equiv.). Let it stir overnight at room temperature. After the completion of reaction, DMF was evaporated on high vacuum pump. Dissolved the material in DCM (10 mL) and washed with water (10 mL), sat.NaHCO₃ (10 mL) and brine (10 mL). Organic layer was dried over Na₂SO₄, filtered and evaporated to get crude product. The solvent was removed under reduced pressure, the residue was purified by chromatography on silica gel (0-20% EtOAc/hexane) to afford product, ethyl 2-(5-chloro-3-formyl-1H-indol-1-yl)acetate (61 % yield).
1H NMR (300 MHz, CDCl₃) δ 9.95 (s, 1H), 8.29 (s, 1H), 7.73 (s, 1H), 7.28 (d, 1H), 7.27 (d, 1H), 4.87 (s, 2H), 4.25 (q, 2H), 1.28 (t, 3H).

### Step 2: Ethyl 2-(5-chloro-3-((hydroxyimino)methyl)-1H-indol-1-yl)acetate

To a solution of ethyl 2-(5-chloro-3-formyl-1H-indol-1-yl)acetate (0.893g, 3.36 mmol, 1.0 equiv.) and hydroxylamine hydrochloric acid (0.350g, 5.04 mmol, 1.5 equiv.) in ethanol (15 mL) and water (10 mL) at room temperature was added solid NaHCO₃ (0.565g, 6.72 mmol, 2.0 equiv.) slowly. The reaction mixture was stirred for 1h at the same temperature (monitored by TLC or 1H NMR). The reaction was not completed then let it stir overnight. The reaction mixture was concentrated to dryness. The crude product was dissolved in DCM (15 mL) and filtered to remove solids. Filtrate was concentrated to provide the product, which was used for next step without further purification.
1H NMR (300 MHz, CDCl₃) δ 8.29 (s, 1H), 8.14 (s, 1H), 7.34-7.12 (m, 3H), 7.06 (s, 1H), 4.82 (s, 2H), 4.24 (q, 2H), 1.27 (t, 3H).

### Step 3: Ethyl 2-(5-chloro-3-(isoxazol-3-yl)-1H-indol-1-yl)acetate

The reaction mixture of Aldoxime (0.430g, 1.53 mmol, 1.0 equiv.), CaC₂ (0.686g, 10.69 mmol, 6.66 equiv.) and NCS (0.285g, 2.13 mmol, 1.33 equiv.) in DCM (5 mL) and Benzene (5 mL) in a sealed tube was stirred at room temperature for 30 minutes to get dissolve all the reagents (still some CaC₂ was there as solid). Opened the cap and water (0.385µL, 21.39 mmol, 13.33 equiv.) was added and immediately sealed the tube. The reaction mixture was stirred for 48h at room temperature (monitored by TLC). Reaction mixture was filtered and solid residue was washed with DCM/Benzene (30 mL). Combined all the organic layers and evaporated to get crude product. Purified product ethyl 2-(5-chloro-3-(isoxazol-3-yl)-1H-indol-1-yl)acetate (49 % Yield) was obtained after purification using 100 % DCM.
1H NMR (300 MHz, CDCl₃) δ 8.33 (s, 1H), 8.22 (s, 1H), 7.42 (s, 1H), 7.19 (d, 1H), 7.11 (d, 1H), 6.47 (s, 1H), 4.76 (s, 2H), 4.19 (q, 2H), 1.23 (t, 3H).

### Step 4: 2-(5-chloro-3-(isoxazol-3-yl)-1H-indol-1-yl)acetic acid

To a solution of ethyl 2-(5-chloro-3-(isoxazol-3-yl)-1H-indol-1-yl)acetate (0.227g, 0.744 mmol, 1.0 equiv.) in MeOH (10 mL) and H₂O (1 mL) at room temperature solid NaOH (0.038g, 0.951 mmol, 1.5 equiv.) was added and stirred resulting mixture at room temperature for overnight. After completion of the reaction remove volatiles and dilute the crude with water and wash aqueous layer with DCM (2X10 ml) (to remove unreacted ester and other impurities). The aqueous was acidified with aq. HCI (1 M) and extracted with EtOAc (2X10 ml). The combined organics were dried over Na₂SO₄. The solvents were removed and dried under vacuum to afford, 2-(5-chloro-3-(isoxazol-3-yl)-1H-indol-1-yl)acetic acid hydrochloride (72 % yield).
1H NMR (300 MHz, MeOD) δ 8.62 (s, 1H), 8.13 (s, 1H), 7.85 (s, 1H), 7.31 (dd, 2H), 6.81 (s, 1H), 5.05 (s, 2H): HPLC Retention Time: 9.907

### Example 6: 2-[5-Bromo-3-(1,2-oxazol-3-yl)-1H-indol-1-yl]acetic acid

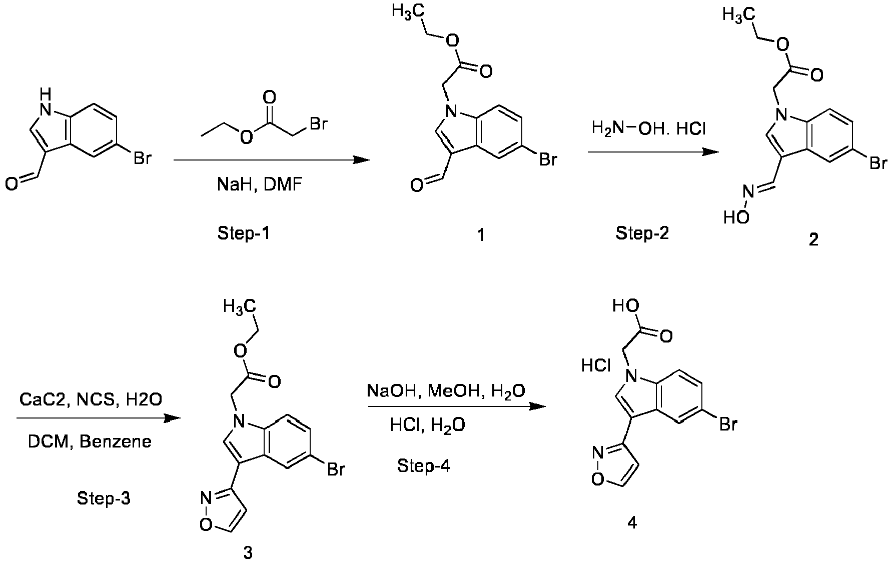

### Ethyl 2-(5-bromo-3-formyl-1H-indol-1-yl)acetate

Same procedure as in Example 5 step 1.
1H NMR (300 MHz, CDCl₃) δ 10.0 (s, 1H), 8.49 (s, 1H), 7.74 (s, 1H), 7.44 (d, 1H), 7.15 (d, 1H), 4.88 (s, 2H), 4.26 (q, 2H), 1.29 (t, 3H).

### Ethyl 2-(5-bromo-3-((hydroxyimino)methyl)-1H-indol-1-yl)acetate

Same procedure as in Example 5 step 2.
1H NMR (300 MHz, DMSO) δ 10.73 (s, 1H), 8.25 (s, 1H), 8.17 (s, 1H), 7.66 (s, 1H), 7.45 (d, 1H), 7.34 (d, 1H), 5.17 (s, 2H), 4.14 (q, 2H), 1.20 (t, 3H).

### Ethyl 2-(5-bromo-3-(isoxazol-3-yl)-1H-indol-1-yl)acetate

Same procedure as in Example 5 step 3.
1H NMR (300 MHz, CDCl₃) δ 8.37 (m, 2H), 7.43 (s, 1H), 7.34 (d, 1H), 7.09 (d, 1H), 6.51 (s, 1H), 4.79 (s, 2H), 4.20 (q, 2H), 1.25 (t, 3H).

### 2-(5-bromo-3-(isoxazol-3-yl)-1H-indol-1-yl)acetic acid

Same procedure as in Example 5 step 4.
1H NMR (300 MHz, MeOD) δ 8.62 (s, 1H), 8.29 (s, 1H), 7.85 (s, 1H), 7.36 (m, 2H), 6.81 (s, 1H), 5.05 (s, 2H): HPLC Retention Time: 12.510.

### Example 7: 2-(5-bromo-1H-indol-1-yl)acetic acid

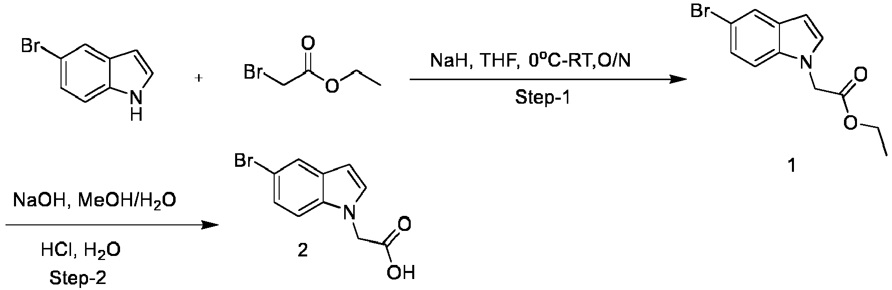

### Step 1: Ethyl 2-(5-bromo-1H-indol-1-yl)acetate

To a stirred suspension of NaH (0.155g, 6.12mmol, 1.2 equiv.) in THF (10 mL) was added solution of 5-bromo-1H-indole (1.0g, 5.10 mmol, 1.0 equiv.) at 0°C. After the stirring for 15 minutes at the same temperature ethyl 2-bromoacetate (1.27g, 7.65 mmol, 1.5 equiv.) was added. The reaction mixture was stirred at room temperature for 1h (monitored by TLC). Water (10 mL) was added and extracted with EtOAc (2X10 ml). Combined all the organic layers and washed with brine (10 mL) and dried over Na₂SO₄. The solvents were removed to get crude product. Purified product ethyl 2-(5-bromo-1H-indol-1-yl)acetate (42 % yield) was obtained by chromatography by using 0-5% Ethyl acetate in hexane.
1H NMR (300 MHz, CDCl₃) δ 7.77 (m, 1H), 7.35-7.28 (m, 1H), 7.16-7.08 (m, 2H), 6.51 (d, 1H), 4.83 (s, 2H), 4.22 (q, 2H), 1.27 (t, 3H):

### Step 2: 2-(5-bromo-1H-indol-1-yl)acetic acid

Same procedure as in Example 5 step 4.
1H NMR (300 MHz, CDCl₃) δ 10.58-9.29 (br s, 1H), 7.77 (s, 1H), 7.77 (s, 1H), 7.33 (d, 1H), 7.11 (d, 1H), 7.07 (d, 1H), 6.52 (d, 1H), 4.87 (s, 2H): HPLC Retention Time: 13.256

### Example 8: Measurement of force in an in vitro model

The current disclosure relates to compounds that inhibit CIC-1 ion channels and increase muscle excitability and thereby improve muscle function in clinical conditions where muscle activation is failing. Such conditions result in loss of contractile function of skeletal muscle, weakness and excessive fatigue. In this series of experiments the compounds were tested for their ability to restore contractile function of isolated rat muscle when the neuromuscular transmission had been compromised akin to neuromuscular disorders.

Experimentally, soleus muscles from 4-5 wk old rats were isolated with the motor nerve remaining attached. The nerve-muscle preparations were mounted in experimental setups that enabled electrical stimulation of the motor nerve. Stimulation of the motor nerve led to activation of the muscle fibres and ensuing force production that was recorded. The nerve-muscle preparations were also in these experiments incubated in the standard Krebs Ringer (see example 5) and the solution was heated to 30°C and continuously equilibrated with a mixture of 95% O₂ and 5% CO₂, pH ∼7.4.

After mounting the nerve-muscle preparation in the experimental setup, the contractile function of the muscle was initially assessed under the control conditions (Figure 1A). Sub-maximal concentration of tubocurarine (115 nM), an acetylcholine receptors antagonist, was then added to the experimental bath to impose partial inhibition of the ability of the motor nerve to activate the muscle fibres. The experimental condition mimics the failing neuromuscular transmission in a range of neuromuscular disorders. After addition of tubocurarine the contractile force declined over the next 90 mins to 10-50 % of the control force. 50 µM of the test compound was then added and the contractile force recovered despite the continued presence of tubocurarine. To quantify the ability of the compound to restore force the percentage of the initial force that was restored was determined after 40 mins of compound exposure (Figure 1B) and the point increase is reported in Table 3.

**Table 3: Percentage increase of initial force that was restored**

| **Compound investigated** | **Point increase (%)** |
|---|---|
| A-4 | 12% |
| A-12 | 13% |
| A-13 | 24% |
| A-14 | 26% |
| A-24 | 10% |

In conclusion, this example demonstrates that the compounds of the present disclosure are able to increase muscle excitability and thereby improve muscle function in clinical conditions.

### Example 9: Screening of compounds on the human isoform of CIC-1 expressed in CHO cells using automated patch-clamp

The investigatory goal of these experiments was to evaluate how compounds affect the open probability and current amplitude of human CIC-1 channels expressed in CHO cells. Experiments were performed using an automated patch clamp system that allowed high throughput testing of whole cell patches together with both intracellular and extracellular addition of compound.

### Automated voltage clamp measurements

Automated whole cell patch clamp experiments were performed with the Qpatch 16 system (Sophion Bioscience, Ballerup, Denmark) at room temperature. Data acquisition and analysis were performed in the Qassay software (ver. 5.6, Odense).

### Voltage protocol and analysis of whole cell CIC-1 currents

To evoke CIC-1 currents in whole cell patches, the membrane potential was initially stepped from a holding potential of -30 mV to +60 mV for 100 ms and then to various test voltages (sweeps) ranging from +120 mV to -140 mV in steps of 20 mV for 300 ms. To obtain tail currents, the membrane potential was stepped to -100 mV after each test voltage for 300 ms and then relaxed to -30 mV for 2 sec between sweeps (Figure 2). I/V relationships for whole cell instant and steady state current amplitudes were obtained by plotting average current densities at the beginning and at the end of the 300 ms step against the membrane potential (Figure 3).

In order to determine the relative overall open probability (P₀), the instantaneous tail currents were normalized to the maximal tail current obtained following the most positive voltage step and plotted against the test voltage. Plots of normalized tail currents from each whole cell patch were then fitted to a Boltzmann function allowing determination of half activation voltages (V_{1/2}, Figure 4).

### Solutions

For automated patch clamp experiments extracellular solutions contained: 2 mM CaCl₂, 1 mM MgCl₂, 10 mM HEPES, 4 mM KCl, 145 mM NaCl, 10 mM Glucose, pH adjusted to 7.4 with NaOH (2 M). Osmolality adjusted to -320 using sucrose.

Intracellular solutions contained: 80 mM CsF, 60 mM CsCl, 5/1 mM KOH/EGTA, 10 mM HEPES, 10 mM NaCl, pH adjusted to 7.2 with NaOH (2 M). Osmolality adjusted to ∼320 mOsm using sucrose.

### Cell line information:

Cells used in patch clamp experiments were Chinese hamster ovary cells (CHO) constitutively expressing human CIC-1 channels. The amino acid sequence encoded by the cDNA used to create this cell line was identical to the translated sequence for GenBank accession number NM_000083.2. Cells were produced by Charles River (Catalogue CT6175, Cleveland OH, USA) in a cryopreserved format. Experiments were performed on the cells directly after thawing (3 x 10⁶ cells used in each experiment).

### Test protocol

To evaluate the compound effect on CIC-1, when applied directly to the intracellular side of the cell membrane, the half activation voltage, V_{1/2}, was determined from whole cell patches with compound added to the intracellular solution and then compared to V_{1/2} determined from control cell patches with only vehicle added to the intracellular solution. Additionally, the effect of extracellular added compound was evaluated by determine V_{1/2} and steady state current amplitudes before and after exchanging the extracellular solution to contain compound.

The difference in half activation voltage of CIC-1 channels, ΔV_{1/2}, was determined as the difference between the cell patches treated intracellularly with compound and control cells patches and is reported in Table 4 below. A positive shift in ΔV_{1/2} is reflecting CIC-1 channel inhibition by the tested compound. P-values of <0.05 is considered significant.

**Table 4: Percentage increase of initial force that was restored**

| **Compound investigated** | **ΔV1/2 (mV)** | **P-value** |
|---|---|---|
| Compound A-14 | 8.4 | 0.02 |
| Compound A-24 | 27.9 | <0.01 |
| Compound A-25 | 5.5 | <0.01 |

## Claims

1. A compound of Formula A: 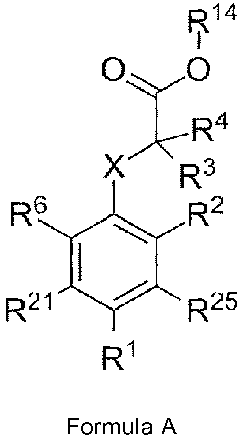 wherein
X is O, NR⁵, or CHR⁵;
R¹ is Cl, Br F, I or H;
R² is H, a bond, -O-, C₁₋₃ alkyl or C₂₋₃ alkenyl, wherein said alkyl or alkenyl is optionally substituted with one or more F, =O, -OH, or phenyl optionally substituted with halogen;
R³ is a bond, H, C₁₋₃ alkyl or C₂₋₃ alkenyl, wherein said alkyl or alkenyl is optionally substituted with one or more F, =O, -OH, or phenyl optionally substituted with halogen;
R⁴ is a bond, H, C₁₋₃ alkyl or C₂₋₃ alkenyl;
R⁵ is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
R⁶ is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F;
R¹⁶ is independently selected from the group consisting of deuterium, methoxy, nitro, cyano, Cl, Br, I and F;
R²¹ is H, F or Br; and
R²⁵ is H, F or Br;
and wherein at least one ring is formed by linking together:
a. R² and R³ to form a ring;
b. R² and R⁵ to form a ring;
c. R² and R³/R⁴ to form a ring, wherein R³ and R⁴ together form a double bond linked to R²; or
d. R³ and R⁵ to form a ring;
or a pharmaceutically acceptable salt, hydrate, polymorph, tautomer, or solvate thereof
for use in treating, ameliorating and/or preventing a neuromuscular disorder,
and/or for use in reversing and/or ameliorating a neuromuscular blockade.

2. The compound for use according to claim 1, wherein the compound is of Formula B: 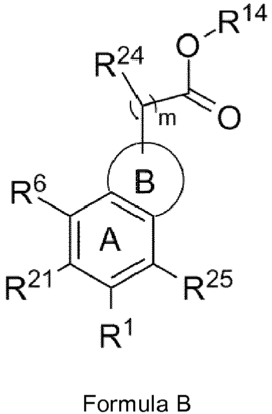 wherein
B is a 5-, 6-, or 7-membered heterocycle, optionally substituted with one or more substituents individually selected from the group consisting of C₁₋₃ alkyl, a 5-membered aromatic heterocycle optionally substituted with C₁₋₃ alkyl, =O, F, -OH and phenyl optionally substituted with halogen;
m is 0 or 1;
R¹ is Cl, Br F or I;
R⁶ is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F; and
R¹⁶ is independently selected from the group consisting of deuterium, methoxy, nitro, cyano, Cl, Br, I and F;
R²⁴ is H or C₁₋₃ alkyl;
R²¹ is H or F; and
R²⁵ is H or F;
or a pharmaceutically acceptable salt, hydrate, polymorph, tautomer, or solvate thereof.

3. The compound for use according to claim 2, wherein rings A and B together constitutes a bicyclic structure selected from the group consisting of formulas AB1 to AB7:

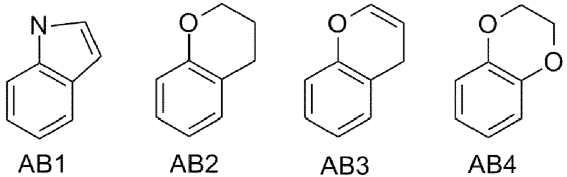

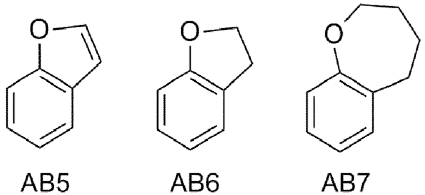

4. The compound for use according to any one of the preceding claims, wherein R⁶ is isoxazol-3-yl or isoxazol-5-yl.

5. The compound for use according to any one of the preceding claims, wherein the compound is of Formula D: 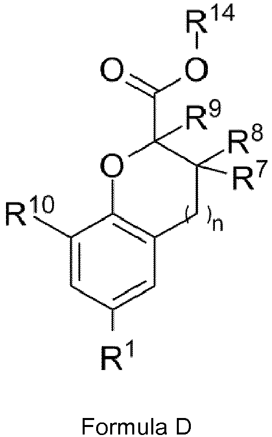 wherein
R¹ is Cl, Br, F or I;
R⁷ is H, F or C₁₋₅ alkyl optionally substituted with one or more, identical or different, substituents R¹⁵;
R⁸ is H or F;
R⁹ is H or methyl;
R¹⁰ is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F;
R¹⁶ is independently selected from the group consisting of deuterium, methoxy, nitro, cyano, Cl, Br, I and F; and
n is 0, 1 or 2;
or a pharmaceutically acceptable salt, hydrate, polymorph, tautomer, or solvate thereof.

6. The compound for use according to any one of the preceding claims, wherein n is 1.

7. The compound for use according to any one of the preceding claims, wherein the compound is of Formula H: 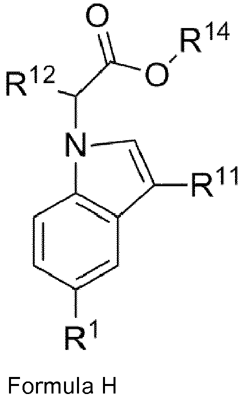 wherein
R¹ is Cl, Br, F or I;
R¹¹ is H or a 5-membered aromatic heterocycle, such as isoxazole, optionally substituted with C₁₋₃ alkyl;
R¹² is H, C₁₋₅ alkyl, or C₃₋₆ cycloalkyl;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F; and
R¹⁶ is independently selected from the group consisting of deuterium, methoxy, nitro, cyano, Cl, Br, I and F;
or a pharmaceutically acceptable salt, hydrate, polymorph, tautomer, or solvate thereof.

8. The compound for use according to any one of the preceding claims, wherein the compound is of Formula I: 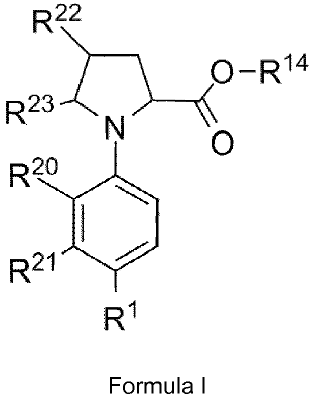 wherein
R¹ is Br, Cl, F or I;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F;
R¹⁶ is independently selected from the group consisting of deuterium, methoxy, nitro, cyano, Cl, Br, I and F;
R²⁰ is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
R²¹ is H or F;
R²² is H or -OH;
R²³ is H or =O;
or a pharmaceutically acceptable salt, hydrate, polymorph, tautomer, or solvate thereof.

9. The compound for use according to any one of the preceding claims, wherein the compound is selected from the group consisting of:
6-bromo-3-methyl-3,4-dihydro-2H-1-benzopyran-2-carboxylic acid;
6-bromo-2-methyl-3,4-dihydro-2H-1-benzopyran-2-carboxylic acid;
6-bromo-8-(1,2-oxazol-5-yl)-3,4-dihydro-2H-1-benzopyran-2-carboxylic acid;
6-bromo-8-[(1E)-1-(hydroxyimino)ethyl]-3,4-dihydro-2H-1-benzopyran-2-carboxylic acid;
6,8-dibromo-3,4-dihydro-2H-1-benzopyran-2-carboxylic acid;
(2S)-6-bromo-8-(1,2-oxazol-3-yl)-3,4-dihydro-2H-1-benzopyran-2-carboxylic acid;
6-bromo-8-(1,2-oxazol-3-yl)-3,4-dihydro-2H-1-benzopyran-2-carboxylic acid;
(2R)-6-bromo-8-(1,2-oxazol-3-yl)-3,4-dihydro-2H-1-benzopyran-2-carboxylic acid;
6-bromo-3,4-dihydro-2H-1-benzopyran-2-carboxylic acid;
6-bromo-4-oxo-3,4-dihydro-2H-1-benzopyran-2-carboxylic acid;
6-bromo-4-oxo-4H-chromene-2-carboxylic acid;
6-bromo-4,4-difluoro-3,4-dihydro-2H-1-benzopyran-2-carboxylic acid;
7-bromo-2,3,4,5-tetrahydro-1-benzoxepine-2-carboxylic acid;
5-bromo-7-(1,2-oxazol-3-yl)-2,3-dihydro-1-benzofuran-2-carboxylic acid;
5-bromo-2,3-dihydro-1-benzofuran-2-carboxylic acid;
5-chloro-3-(2-chlorophenyl)-1-benzofuran-2-carboxylic acid;
2-[6-bromo-3-(1,2-oxazol-3-yl)-1H-indol-1-yl]acetic acid;
(2S)-2-[5-bromo-3-(1,2-oxazol-3-yl)-1H-indol-1-yl]propanoic acid;
2-[5-bromo-7-(1,2-oxazol-3-yl)-1H-indol-1-yl]acetic acid;
2-[5-chloro-3-(1,2-oxazol-3-yl)-1H-indol-1-yl]acetic acid;
(2R)-2-(5-chloro-1H-indol-1-yl)propanoic acid;
2-[5-bromo-3-(1,2-oxazol-3-yl)-1H-indol-1-yl]acetic acid;
(2S)-2-(5-chloro-1H-indol-1-yl)propanoic acid;
(2S)-2-(5-bromo-1H-indol-1-yl)propanoic acid;
2-(5-bromo-1H-indol-1-yl)acetic acid;
(2R)-2-(5-bromo-1H-indol-1-yl)propanoic acid;
2-(5-bromo-1H-indol-1-yl)propanoic acid;
(2S)-1-(4-bromo-2-fluorophenyl)pyrrolidine-2-carboxylic acid;
1-(4-bromophenyl)pyrrolidine-2-carboxylic acid;
(2S)-1-(4-bromophenyl)pyrrolidine-2-carboxylic acid;
(2S)-1-(4-bromo-3-fluorophenyl)pyrrolidine-2-carboxylic acid;
(2S,4S)-1-(4-bromophenyl)-4-hydroxypyrrolidine-2-carboxylic acid;
(2R)-1-(4-bromophenyl)pyrrolidine-2-carboxylic acid;
(2S)-1-[4-bromo-2-(1,2-oxazol-3-yl)phenyl]pyrrolidine-2-carboxylic acid;
(2S)-1-(4-bromophenyl)-5-oxopyrrolidine-2-carboxylic acid; and
(2S)-1-(4-bromo-2-fluorophenyl)-5-oxopyrrolidine-2-carboxylic acid.

10. The compound for use according to any one of claims 1 to 0, wherein the neuromuscular disorder is selected from the group consisting of myasthenia gravis, amyotrophic lateral sclerosis (ALS), spinal muscular atrophy (SMA), critical illness myopathy (CIM), Charcot-Marie tooth disease (CMT) and sarcopenia.

11. The compound for use according to any one of claims 1 to 10, wherein the neuromuscular disorder has been induced by a neuromuscular blocking agent.

12. A compound of Formula A: 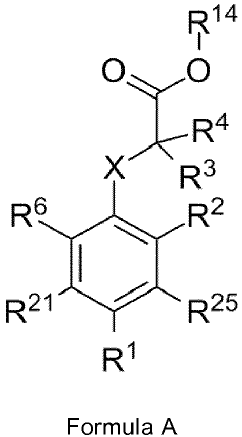 wherein
X is O, NR⁵, or CHR⁵;
R¹ is Cl, Br F, I or H;
R² is H, a bond, -O-, C₁₋₃ alkyl or C₂₋₃ alkenyl, wherein said alkyl or alkenyl is optionally substituted with one or more F, =O, -OH, or phenyl optionally substituted with halogen;
R³ is a bond, H, C₁₋₃ alkyl or C₂₋₃ alkenyl, wherein said alkyl or alkenyl is optionally substituted with one or more F, =O, -OH, or phenyl optionally substituted with halogen;
R⁴ is a bond, H, C₁₋₃ alkyl or C₂₋₃ alkenyl;
R⁵ is a bond, C₁₋₃ alkyl optionally substituted with -OH, C₂₋₃ alkenyl optionally substituted with -OH, or a 5-membered aromatic heterocycle, such as isoxazole, wherein said 5-membered aromatic heterocycle is optionally substituted with C₁₋₃ alkyl;
R⁶ is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵, and C₂₋₅ alkenyl optionally substituted with R¹⁵;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F;
R¹⁶ is independently selected from the group consisting of deuterium, methoxy, nitro, cyano, Cl, Br, I and F;
R²¹ is H, F or Br; and
R²⁵ is H, F or Br;
and wherein at least one ring is formed by linking together:
a. R² and R³ to form a ring;
b. R² and R⁵ to form a ring;
c. R² and R³/R⁴ to form a ring, wherein R³ and R⁴ together form a double bond linked to R²; or
d. R³ and R⁵ to form a ring;
with the proviso that when X is CHR⁵ then R² is -O-; and
with the proviso that when R¹ is H, then X is NR⁵, R² is a bond, R⁵ is C₂ alkenyl optionally substituted with isoxazole, and R² and R⁵ are linked together to form a ring;
or a pharmaceutically acceptable salt, hydrate, polymorph, tautomer, or solvate thereof.

13. The compound according to claim 12, wherein R⁶ is isoxazol-3-yl or isoxazol-5-yl optionally substituted with C₁₋₃ alkyl.

14. The compound according to claim 12, wherein the compound is of Formula D: 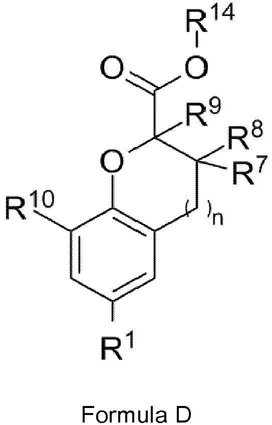 wherein
R¹ is Cl, Br, F or I;
R⁷ is H, F or C₁₋₅ alkyl optionally substituted with one or more, identical or different, substituents R¹⁵;
R⁸ is H or F;
R⁹ is H or methyl;
R¹⁰ is selected from the group consisting of H, Br, Cl, F, a 5-membered aromatic heterocycle optionally substituted with C₁₋₃ alkyl, C₂₋₅ alkyl optionally substituted with R¹⁵ or oxime, C₃₋₅ cycloalkyl optionally substituted with R¹⁵ and C₂₋₅ alkenyl optionally substituted with R¹⁵;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F;
R¹⁶ is independently selected from the group consisting of deuterium, methoxy, nitro, cyano, Cl, Br, I and F; and
n is 0, 1 or 2;
or a pharmaceutically acceptable salt, hydrate, polymorph, tautomer, or solvate thereof.

15. The compound according to claim 12, wherein the compound is of Formula H: 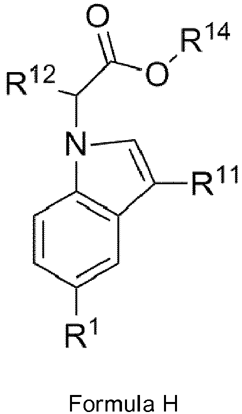 wherein
R¹ is Cl, Br, F or I;
R¹¹ is H or a 5-membered aromatic heterocycle, such as isoxazole, optionally substituted with C₁₋₃ alkyl;
R¹² is H, C₁₋₅ alkyl, or C₃₋₆ cycloalkyl;
R¹⁴ is selected from the group consisting of H, C₁₋₅ alkyl, C₂₋₅ alkenyl, C₂₋₅ alkynyl, C₃₋₆ cycloalkyl wherein the C₁₋₅ alkyl, C₂₋₅ alkenyl, C₂₋₅ alkynyl, C₃₋₆ cycloalkyl group may be optionally substituted with one or more, identical or different, substituents R¹⁵, phenyl optionally substituted with one or more, identical or different, substituents R¹⁶ and benzyl optionally substituted with one or more, identical or different, substituents R¹⁶;
R¹⁵ is independently selected from the group consisting of deuterium and F; and
R¹⁶ is independently selected from the group consisting of deuterium, methoxy, nitro, cyano, Cl, Br, I and F;
or a pharmaceutically acceptable salt, hydrate, polymorph, tautomer, or solvate thereof.

16. The compound according to claim 15, wherein R¹¹ is isoxazole, such as isoxazol-3-yl, isoxazol-4-yl, or isoxazol-5-yl optionally substituted with C₁₋₃ alkyl.

17. The compound according to any one of the claims 12 to 16, wherein the compound is selected from the group consisting of
6-bromo-3-methyl-3,4-dihydro-2H-1-benzopyran-2-carboxylic acid;
6-bromo-2-methyl-3,4-dihydro-2H-1-benzopyran-2-carboxylic acid;
6-bromo-8-(1,2-oxazol-5-yl)-3,4-dihydro-2H-1-benzopyran-2-carboxylic acid;
6-bromo-8-[(1E)-1-(hydroxyimino)ethyl]-3,4-dihydro-2H-1-benzopyran-2-carboxylic acid;
6,8-dibromo-3,4-dihydro-2H-1-benzopyran-2-carboxylic acid;
(2S)-6-bromo-8-(1,2-oxazol-3-yl)-3,4-dihydro-2H-1-benzopyran-2-carboxylic acid;
6-bromo-8-(1,2-oxazol-3-yl)-3,4-dihydro-2H-1-benzopyran-2-carboxylic acid;
(2R)-6-bromo-8-(1,2-oxazol-3-yl)-3,4-dihydro-2H-1-benzopyran-2-carboxylic acid;
7-bromo-2,3,4,5-tetrahydro-1-benzoxepine-2-carboxylic acid;
5-bromo-7-(1,2-oxazol-3-yl)-2,3-dihydro-1-benzofuran-2-carboxylic acid;
2-[6-bromo-3-(1,2-oxazol-3-yl)-1H-indol-1-yl]acetic acid;
(2S)-2-[5-bromo-3-(1,2-oxazol-3-yl)-1H-indol-1-yl]propanoic acid;
2-[5-bromo-7-(1,2-oxazol-3-yl)-1H-indol-1-yl]acetic acid;
2-[5-chloro-3-(1,2-oxazol-3-yl)-1H-indol-1-yl]acetic acid;
(2R)-2-(5-chloro-1 H-indol-1-yl)propanoic acid;
2-[5-bromo-3-(1,2-oxazol-3-yl)-1H-indol-1-yl]acetic acid;
(2S)-2-(5-chloro-1H-indol-1-yl)propanoic acid;
(2R)-2-(5-bromo-1 H-indol-1-yl)propanoic acid;
(2S)-1-(4-bromo-2-fluorophenyl)pyrrolidine-2-carboxylic acid;
(2S)-1-(4-bromo-3-fluorophenyl)pyrrolidine-2-carboxylic acid;
(2S,4S)-1-(4-bromophenyl)-4-hydroxypyrrolidine-2-carboxylic acid; and
(2S)-1-[4-bromo-2-(1,2-oxazol-3-yl)phenyl]pyrrolidine-2-carboxylic acid.
